# EUROPÄISCHE PATENTSCHRIFT

(11) **EP 1 280 805 B1**
(45) Veröffentlichungstag und Bekanntmachung des Hinweises auf die Patenterteilung: **09.02.2005**
(21) Anmeldenummer: 01911663.1
(22) Anmeldetag: 20.02.2001
(51) Int. Cl.: C07D 487/04, A61K 31/53, A61P 15/10

(54) **NEUE IMIDAZOTRIAZINONE UND IHRE VERWENDUNG**
NOVEL IMIDAZOTRIAZINONES AND THE USE THEREOF
NOUVELLES IMIDAZOTRIAZINONES ET LEUR UTILISATION

(30) Priorität: 02.03.2000 DE 10010067
(43) Veröffentlichungstag der Anmeldung: 05.02.2003
(73) Patentinhaber: Bayer HealthCare AG, 51368 Leverkusen (DE)
(72) Erfinder: NIEWÖHNER, Ulrich, 42929 Wermelskirchen (DE); ES-SAYED, Mazen, 40764 Langenfeld (DE); LAMPE, Thomas, 42105 Wuppertal (DE); HANING, Helmut, 42115 Wuppertal (DE); SCHMIDT, Gunter, 42115 Wuppertal (DE); SCHLEMMER, Karl-Heinz, 42113 Wuppertal (DE); BISCHOFF, Erwin, 42115 Wuppertal (DE); DEMBOWSKY, Klaus, Boston, MA 02116 (US); PERZBORN, Elisabeth, 42327 Wuppertal (DE)
(86) Internationale Anmeldenummer: PCT/EP2001/001871
(87) Internationale Veröffentlichungsnummer: WO 2001/064677

(56) Entgegenhaltungen:
- EP-A- 0 526 004
- WO-A-94/28902
- WO-A-99/24433
- DUMAITRE B ET AL: "SYNTHESIS AND CYCLIC GMP PHOSPHODIESTERASE INHIBITORY ACTIVITY OF A SERIES OF 6-PHENYLPYRAZOLOU3,4-DPYRIMIDONES" JOURNAL OF MEDICINAL CHEMISTRY,AMERICAN CHEMICAL SOCIETY. WASHINGTON,US, Bd. 39, Nr. 8, 1996, Seiten 1635-1644, XP000651134 ISSN: 0022-2623

## Beschreibung

Die vorliegende Erfindung betrifft neue Imidazotriazinone, Verfahren zu ihrer Herstellung und ihre Verwendung als Arzneimittel, insbesondere als Inhibitoren cGMP-metabolisierender Phosphodiesterasen.

In der Offenlegungsschrift DE-OS 2811780 sind Imidazotriazine als Bronchodilatoren mit spasmolytischer Aktivität und Hemmaktivität gegen cyclisches Adenosinmonophosphat metabolisierende Phosphodiesterasen (cAMP-PDE's, gemäß der Nomenklatur nach Beavo auch als PDE III und PDE IV bezeichnet) beschrieben. Eine Hemmwirkung gegen cyclisches Guanosinmonophosphat metabolisierende Phosphodiesterasen [cGMP-PDE's, gemäß der Nomenklatur nach Beavo und Reifsnyder (Trends in Pharmacol. Sci. 11, 150-155, 1990) auch als PDE I, PDE II und PDE V bezeichnet] ist nicht beschrieben. Weiterhin werden Imidazotriazinone in der FR-22 13 058, der CH-59 46 71, der DE-22 55 172, der DE-23 64 076 und der EP-000 9384 beschrieben, die in der 2-Position keinen substituierten Arylrest besitzen, und ebenfalls als Bronchodilatatoreh mit cAMP-PDE inhibitorischer Wirkung beschrieben werden.

In der WO-A-99/24433 werden ebenfalls Imidazotriazinone als cGMP-metabolisierende Phosphodiesterase-Inhibitoren beschrieben, die jedoch in para-Position zur Alkoxygruppe im Phenylring zwingend eine Sulfonamidgruppe umfassen.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten führen. Es kann die Kurz- oder Langzeitmodulation der vaskulären und kardialen Inotropie, den Herzrhythmus und die kardiale Erregungsleitung beeinflussen (J. C. Stoclet, T. Keravis, N. Komas and C. Kugnier, Exp. Opin. Invest. Drugs (1995), 4 (11), 1081-1100). Die Inhibition der cGMP-PDE's kann auch eine Verstärkung der Erektion bewirken. Daher sind solche Verbindungen zur Behandlung zur erektilen Dysfunktion geeignet.

Die vorliegende Erfindung betrifft somit neue Imidazotriazinone der allgemeinen Formel (I) 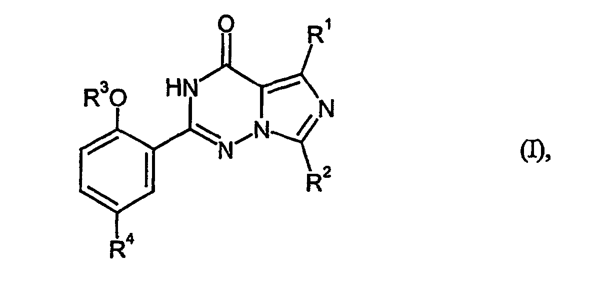 in welcher
- R¹: für (C₁-C₆)-Alkyl steht,
- R²: für (C₃-C₈)-Cycloalkyl oder (C₁-C₁₂)-Alkyl steht,
- R³: für (C₁-C₆)-Alkyl steht,
- R⁴: für einen Rest der Formeln 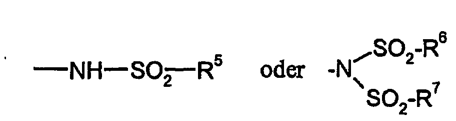 steht,
worin
R⁵, R⁶ und R⁷ gleich oder verschieden sind und Vinyl oder (C₁-C₆)-Alkyl bedeuten, das gegebenenfalls bis zu 3-fach, gleich oder verschieden, durch Trifluormethyl, Halogen, (C₁-C₆)-Alkoxy oder durch Reste der Formeln
- R⁴: für einen Rest der Formel -NH-CO-NR¹⁷R¹⁸ steht,
worin
R¹⁷ und R¹⁸ gleich oder verschieden sind und Wasserstoff oder (C₁-C₆)-Alkyl bedeuten, das gegebenenfalls durch Hydroxy oder durch einen Rest der Formeln 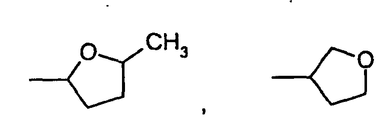 oder -NR¹⁹R²⁰ substituiert ist,
worin
R¹⁹ und R²⁰ gleich oder verschiedene sind und Wasserstoff, Phenyl oder (C₁-C₆)-Alkyl bedeuten oder
R¹⁷ und R¹⁸ gemeinsam mit dem Stickstoffatom, an das sie gebunden sind, einen heterocyclischen Ring der Formeln 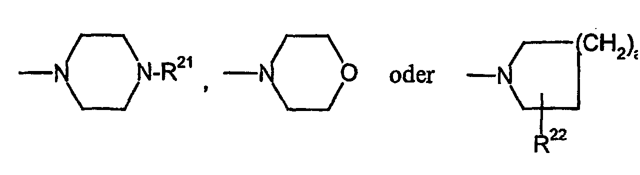 bilden,
worin
R²¹ Wasserstoff oder (C₁-C₆)-Alkyl bedeutet,
a entweder 1 oder 2 bedeutet,
R²² Hydroxy oder (C₁-C₆)-Alkyl bedeutet, das gegebenenfalls durch Hydroxy substituiert ist, oder
R¹⁷ und/oder R¹⁸ (C₆-C₁₂)-Aryl bedeuten, das gegebenenfalls durch Halogen, Trifluorethyl oder durch -SCF₃ substituiert ist oder
R¹⁷ Wasserstoff bedeutet und
R¹⁸ einen Rest der Formel -SO₂-R²³ bedeutet,
worin
R²³ (C₁-C₆)-Alkyl oder (C₆-C₁₂)-Aryl bedeutet, das gegebenenfalls durch Halogen substituiert ist, oder für einen Rest der Formeln 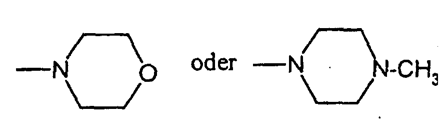 steht,
und ihre Tautomeren sowie deren pharmazeutisch verträgliche Salze, Hydrate und Prodrugs.

Die erfindungsgemäßen Verbindungen der allgemeinen Formel (I) können in Abhängigkeit von dem Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen der allgemeinen Formel (I) in tautomeren Formen vorliegen. Dies ist dem Fachmann bekannt, und derartige Verbindungen sind ebenfalls vom Umfang der Erfindung umfasst.

Physiologisch unbedenkliche, d. h. pharmazeutisch verträgliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Als pharmazeutisch verträgliche Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin oder Methylpiperidin.

Als "Hydrate" werden erfindungsgemäß solche Formen der Verbindungen der obigen allgemeinen Formel (I) bezeichnet, welche in festem oder flüssigem Zustand durch Hydratation mit Wasser eine Molekül-Verbindung (Solvat) bilden. In den Hydraten sind die Wassermoleküle nebenvalent durch zwischenmolekulare Kräfte, insbesondere Wasserstoff-Brückenbindungen angelagert. Feste Hydrate enthalten Wasser als sogenanntes Kristall-Wasser in stöchiometrischen Verhältnissen, wobei die Wassermoleküle hinsichtlich ihres Bindungszustands nicht gleichwertig sein müssen. Beispiele für Hydrate sind Sesquihydrate, Monohydrate, Dihydrate oder Trihydrate. Gleichermaßen kommen auch die Hydrate von Salzen der erfindungsgemäßen Verbindungen in Betracht.

Als "Prodrugs" werden erfindungsgemäß solche Formen der Verbindungen der obigen allgemeinen Formel (I) bezeichnet, welche selbst biologisch aktiv oder inaktiv sein können, jedoch in die entsprechende biologisch aktive Form überführt werden können (beispielsweise metabolisch, solvolytisch oder auf andere Weise).

(C₁-C₁₂)-Alkyl steht für einen geradkettigen oder verzweigten Alkylrest mit 1 bis 12 Kohlenstoffatomen. Beispielsweise seien genannt: Methyl, Ethyl, n-Propyl, Isopropyl, n-Butyl, Isobutyl, tert.-Butyl, n-Pentyl und n-Hexyl. Aus dieser Definition leiten sich analog die entsprechenden Alkylgruppen mit weniger Kohlenstoffatomen wie z.B. (C₁-C₆)-Alkyl und (C₁-C₄)-Alkyl ab. Im allgemeinen gilt, dass (C₁-C₄)-Alkyl bevorzugt ist.

(C₃-C₈)-Cycloalkyl steht für einen cyclischen Alkylrest mit 3 bis 8 Kohlenstoffatomen. Beispielsweise seien genannt: Cyclopropyl, Cyclobutyl, Cyclopentyl, Cyclohexyl, Cycloheptyl oder Cyclooctyl. Aus dieser Definition leiten sich analog die entsprechenden Cycloalkylgruppen mit weniger Kohlenstoffatomen wie z.B. (C₃-C₅)-Cycloalkyl ab. Bevorzugt sind Cyclopropyl, Cyclopentyl und Cyclohexyl.

(C₁-C₆)-Alkoxy steht für einen geradkettigen oder verzweigten Alkoxyrest mit 1 bis 6 Kohlenstoffatomen. Beispielsweise seien genannt: Methoxy, Ethoxy, n-Propoxy, Isopropoxy, n-Butoxy, Isobutoxy, tert.-Butoxy, n-Pentoxy und n-Hexoxy. Aus dieser Definition leiten sich analog die entsprechenden Alkoxygruppen mit weniger Kohlenstoffatomen wie z.B. (C₁-C₆)-Alkoxy und (C₁-C₄)-Alkoxy ab. Im allgemeinen gilt, dass (C₁-C₄)-Alkoxy bevorzugt ist.

Aus dieser Definition leitet sich auch die Bedeutung des entsprechenden Bestandteils anderer komplexerer Substituenten ab wie z. B. Alkoxycarbonyl.

(C₆-C₁₂)-Aryl steht für einen aromatischen Rest mit 6 bis 12 Kohlenstoffatomen. Beispielsweise seien genannt: Phenyl und Naphthyl.

5- bis 6-gliedriger, aromatischer oder gesättigter Heterocyclus mit bis zu 3 Heteroatomen aus der Reihe S, N und/oder O steht entweder für einen Heteroaromaten, der über ein Ringkohlenstoffatom des Heteroaromaten, gegebenenfalls auch über ein Ringstickstoffatom des Heteroaromaten, verknüpft ist; beispielsweise seien genannt: Pyridyl, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, wobei Pyridyl, Pyrimidyl, Pyridazinyl, Furyl und Thienyl bevorzugt sind, oder für einen gesättigten Heterocyclus, der über ein Ringkohlenstoffatom oder ein Ringstickstoffatom verknüpft ist, oder für einen (C₅-C₆)-Cycloalkylrest, wie oben definiert; beispielsweise seien genannt: Tetrahydrofuryl, Pyrrolidinyl, Piperidinyl, Piperazinyl, Morpholinyl, Thiomorpholinyl, Cyclopentyl und Cyclohexyl wobei Piperidinyl, Morpholinyl und Pyrrolidinyl bevorzugt sind.

Bevorzugt sind erfindungsgemäßeVerbindungen der allgemeinen Formel (I),
in welcher
- R¹: für (C₁-C₄)-Alkyl steht,
- R²: für Cyclopentyl, Cycloheptyl oder (C₁-C₁₀)-Alkyl steht,
- R³: für (C₁-C₄)-Alkyl steht,
- R⁴: für einen Rest der Formel -NH-CO-NR¹⁷R¹⁸ steht,
worin
R¹⁷ und R¹⁸ gleich oder verschieden sind und Wasserstoff oder (C₁-C₄)-Alkyl bedeuten, das gegebenenfalls durch Hydroxy oder durch einen Rest der Formeln 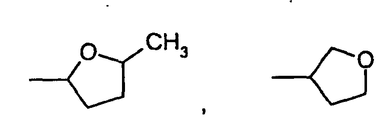 oder -NR¹⁹R²⁰ substituiert ist,
worin
R¹⁹ und R²⁰ gleich oder verschiedene sind und Wasserstoff, Phenyl oder (C₁-C₄)-Alkyl bedeuten oder
R¹⁷ und R¹⁸ gemeinsam mit dem Stickstoffatom, an das sie gebunden sind, einen heterocyclischen Ring der Formeln 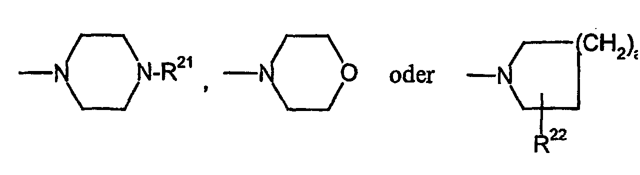 bilden,
worin
R²¹ Wasserstoff oder (C₁-C₄)-Alkyl bedeutet,
a entweder 1 oder 2 bedeutet,
R²² Hydroxy oder (C₁-C₄)-Alkyl bedeutet, das gegebenenfalls durch Hydroxy substituiert ist, oder
R¹⁷ und/oder R¹⁸ Phenyl bedeuten, das gegebenenfalls durch Chlor,Trifluorethyl oder durch -SCF₃ substituiert ist oder
R¹⁷ Wasserstoff bedeutet und
R¹⁸ einen Rest der Formel -SO₂-R²³ bedeutet,
worin
R²³ (C₁-C₄)-Alkyl oder Phenyl bedeutet, das gegebenenfalls durch Halogen substituiert ist, oder für einen Rest der Formeln 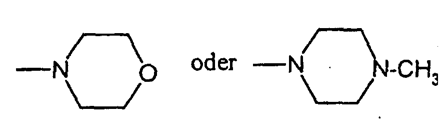 steht,
und ihre Tautomeren sowie deren pharmazeutisch verträgliche Salze, Hydrate und Prodrugs.

Besonders bevorzugt sind erfindungsgemäße Verbindungen der allgemeinen Formel (I), in welcher
- R¹: für (C₁-C₄)-Alkyl steht,
- R²: für Cyclopentyl, Cyclohexyl, Cycloheptyl oder (C₁-C₁₀)-Alkyl steht,
- R³: für (C₁-C₄)-Alkyl steht,
- R⁴: für einen Rest der Formel -NH-CO-NR¹⁷R¹⁸ steht,
worin
R¹⁷ und R¹⁸ gleich oder verschieden sind und Wasserstoff oder (C₁-C₄)-Alkyl bedeuten, das gegebenenfalls durch Hydroxy substituiert ist,
oder
R¹⁷ und R¹⁸ gemeinsam mit dem Stickstoffatom, an das sie gebunden sind, einen heterocyclischen Ring der Formeln 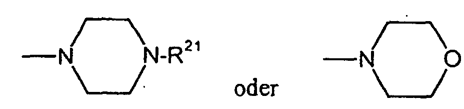 bilden,
worin
R²¹ Wasserstoff oder (C₁-C₄)-Alkyl bedeutet, oder
R¹⁷ und/oder R¹⁸ Phenyl bedeuten, das gegebenenfalls durch Chlor, Trifluorethyl oder durch -SCF₃ substituiert ist oder
R¹⁷ Wasserstoff bedeutet und
R¹⁸ einen Rest der Formel -SO₂-R²³ bedeutet,
worin
R²³ (C₁-C₄)-Alkyl oder Phenyl bedeutet, das gegebenenfalls durch Halogen substituiert ist, oder für einen Rest der Formeln 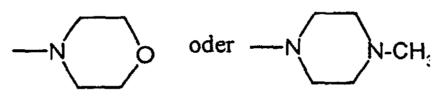 steht,
und ihre Tautomeren sowie deren pharmazeutisch verträgliche Salze, Hydrate und Prodrugs.

Ganz besonders bevorzugt sind die erfindungsgemäßen Verbindungen mit den folgenden Strukturen:

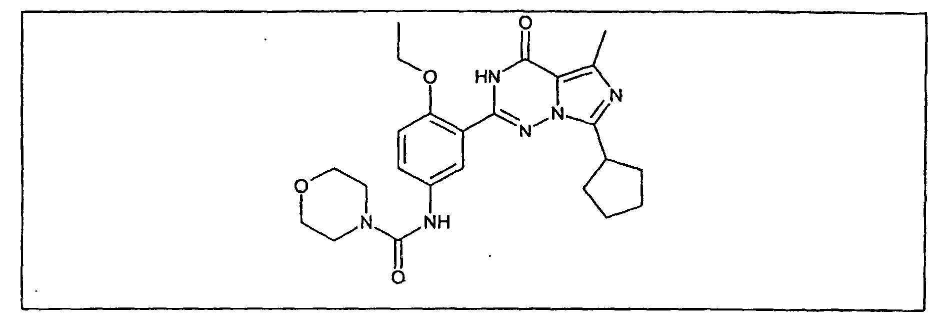

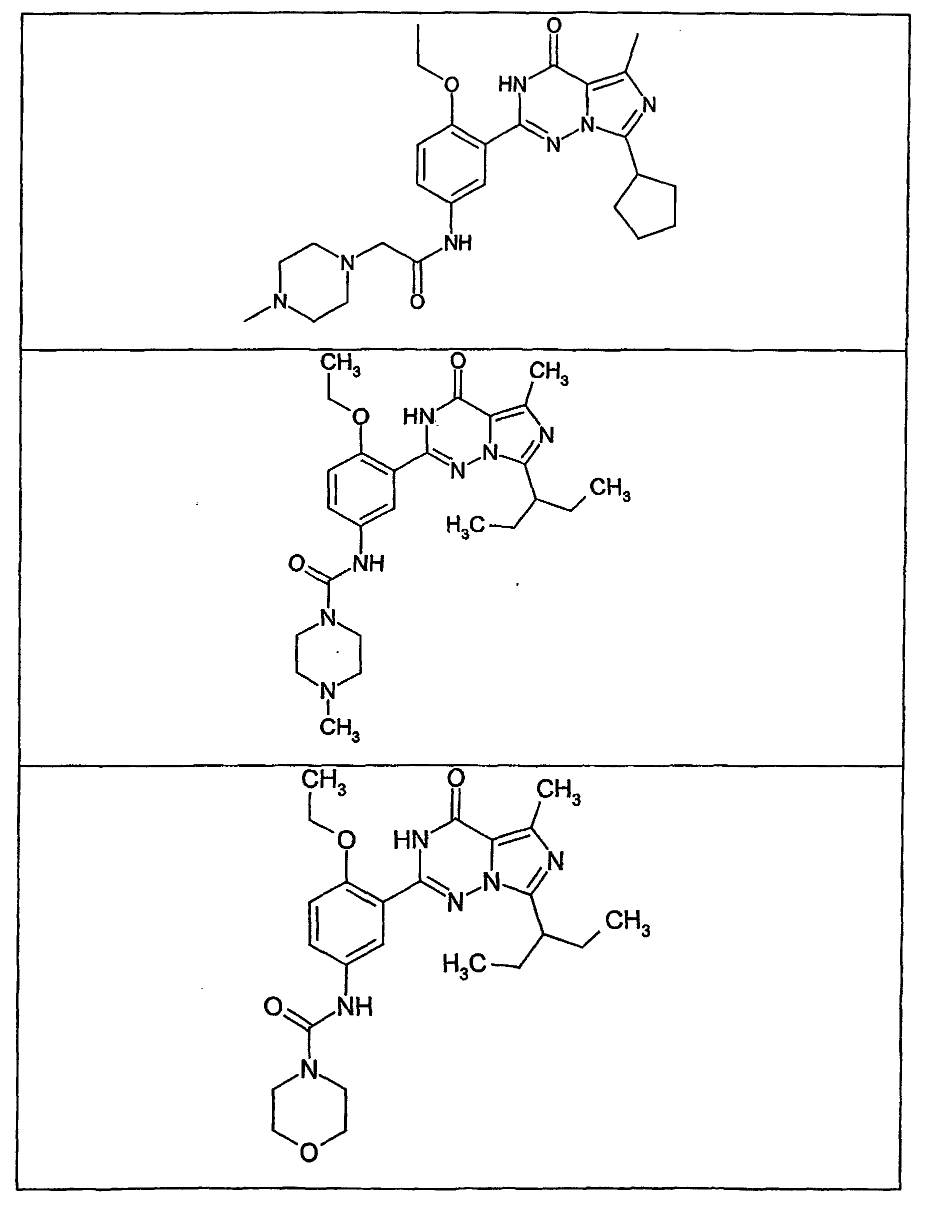

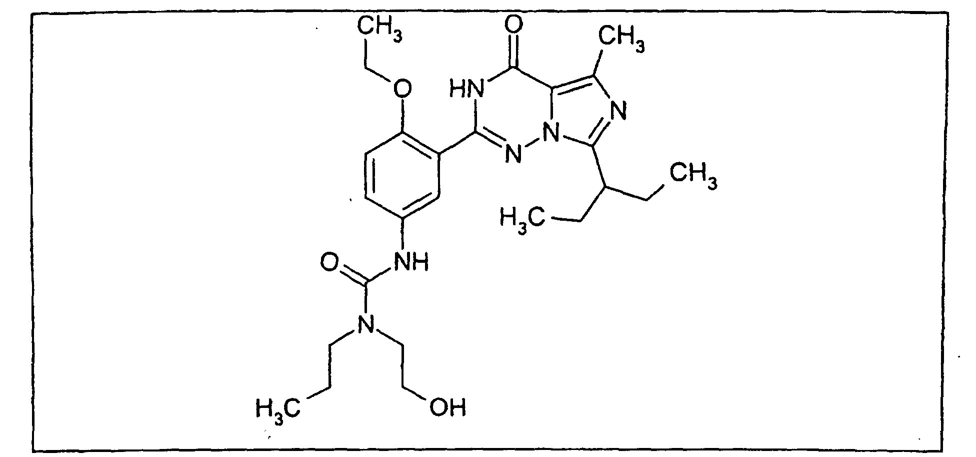

und ihre Tautomeren sowie deren pharmazeutisch verträgliche Salze, Hydrate und Prodrugs.

Die erfindungsgemäßen Verbindungen der allgemeinen Formel (I) können hergestellt werden, indem man
[A] im Fall, dass R⁴ für einen wie zuvor definierten, über ein Stickstoffatom gebundenen Rest steht, Verbindungen der allgemeinen Formel (II) 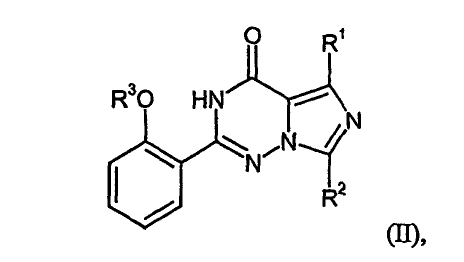 in welcher
R¹, R² und R³ die oben angegebene Bedeutung haben,
zunächst durch Umsetzung mit HNO₃/CF₃CO₂H in die Verbindungen der allgemeinen Formel (III) 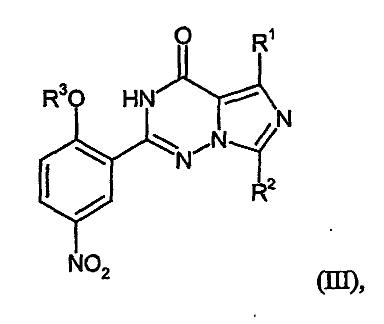 in welcher
R¹, R² und R³ die oben angegebene Bedeutung haben,
überführt,
in einem nächsten Schritt mit H₂/Pd-C zu den Aminen der allgemeinen Formel (IV) 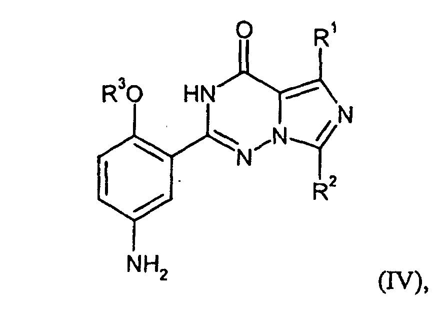 in welcher
R¹, R² und R³ die oben angegebene Bedeutung haben,
reduziert
und
abschließend mit Verbindungen der allgemeinen Formel (V)

A - D (V),

worin
- A: für die oben unter R⁴ aufgeführten Reste R¹⁷ oder R¹⁸ steht,
- D: für die Reste -NH-CO-Cl, -N=C=O oder -SO₂-N=C=O steht,
in inerten Lösemitteln, gegebenenfalls in Anwesenheit einer Base und/oder eines Hilfsmittels umsetzt,
R¹, R² und R³ die oben angegebene Bedeutung haben,

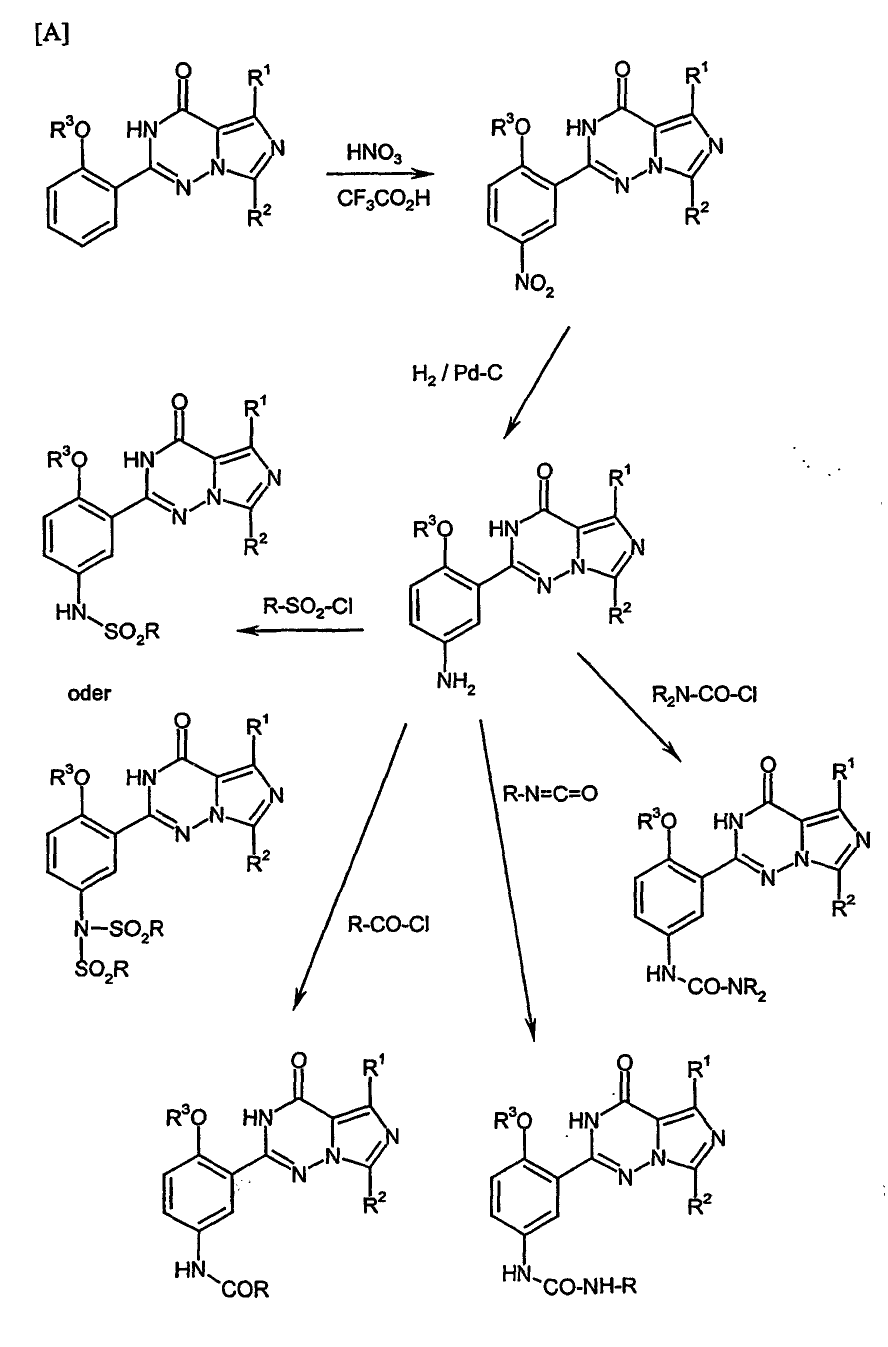

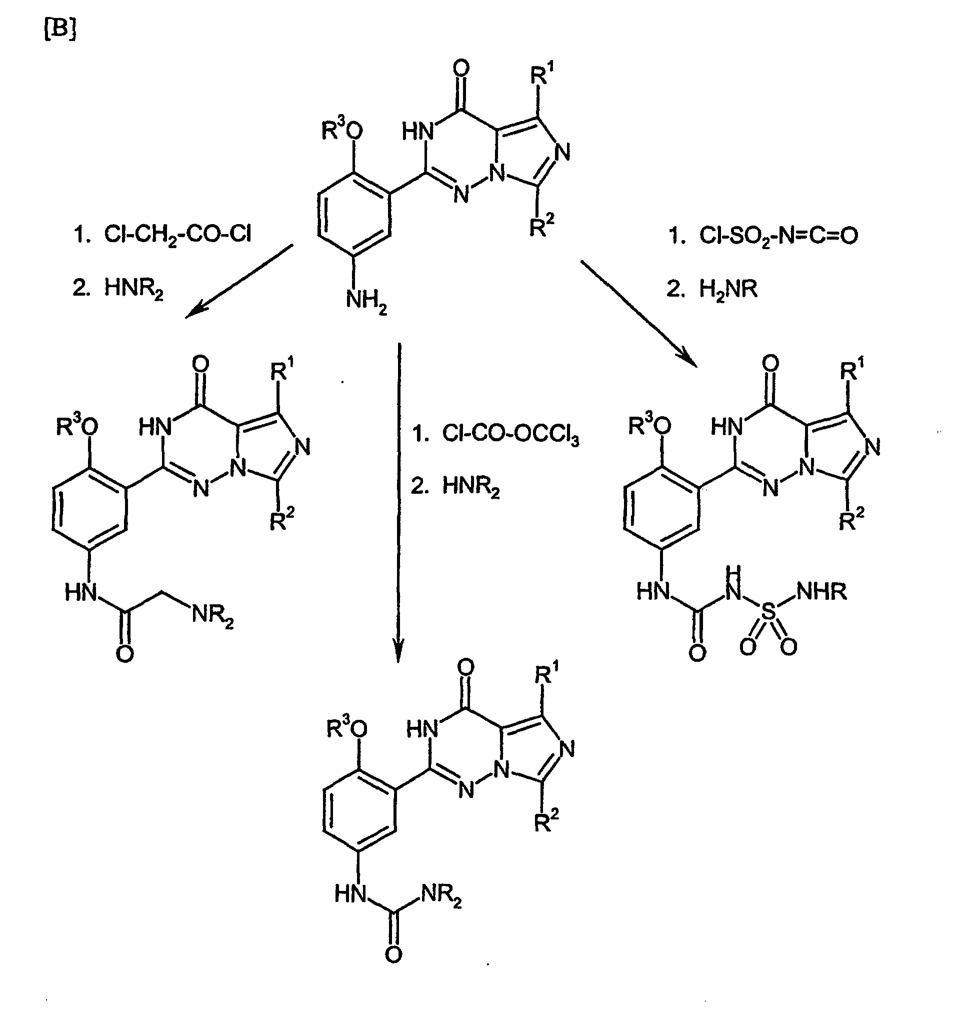

Als Lösemittel für die einzelnen Schritte eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden.

Die Reaktionstemperaturen können im allgemeinen in einem größeren Bereich variieren. Im allgemeinen arbeitet man in einem Bereich von -20°C bis 200°C, bevorzugt von 0°C bis 70°C.

Die erfindungsgemäßen Verfahrensschritte werden im allgemeinen bei Normaldruck durchgeführt. Es ist aber auch möglich, bei Überdruck oder bei Unterdruck durchzuführen (z. B. in einem Bereich von 0,5 bis 5 bar).

Die Umsetzungen können beispielsweise in einem Temperaturbereich von 0°C bis Raumtemperatur und bei Normaldruck erfolgen.

Die Verbindungen der allgemeinen Formel (II) können hergestellt werden, indem man Verbindungen der allgemeinen Formel (XII) 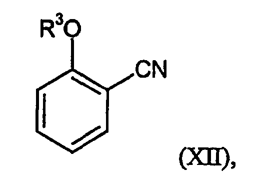 in welcher
- R³: die oben angegebene Bedeutung hat,
durch Umsetzung der Nitrilgruppe in die entsprechenden Amidine überführt, diese zunächst mit Hydrazin und dann mit Verbindungen der allgemeinen Formel (XIII) 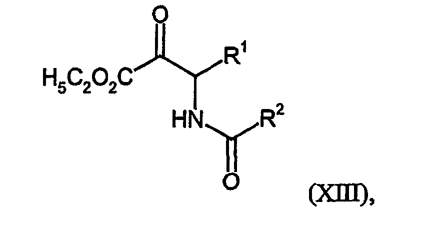 in welcher
R¹ und R² die oben angegebene Bedeutung haben,
zu Verbindungen der allgemeinen Formel (XIV) 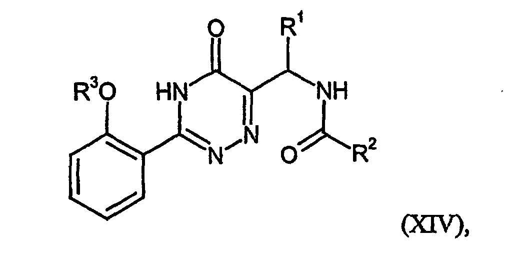 in welcher
R¹, R² und R³ die oben angegebene Bedeutung haben,
umsetzt und diese anschließend durch Einwirkung von POCl₃ zu Verbindungen der allgemeinen Formel (II) cyclisiert. Für Einzelheiten dieses Verfahrens kann verwiesen werden auf die WO-A-99/24433, deren voller Inhalt hiermit durch Bezug eingeschlossen ist.

Die Verbindungen der allgemeinen Formeln (III), (IV), (VII), (VIII) und (X) sind größtenteils neu und können wie oben beschrieben hergestellt werden.

Die Verbindungen der allgemeinen Formeln (V), (VI), (XI), (XII), (XIII) und (XIV) sind an sich bekannt oder nach üblichen Methoden herstellbar.

Die Verbindungen der allgemeinen Formel (IX) sind teilweise neu und können hergestellt werden, indem man Verbindungen der allgemeinen Formel (XV) 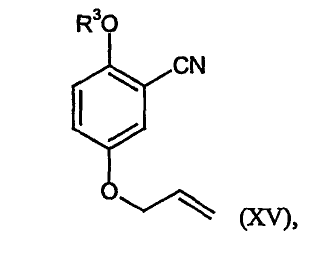 in welcher
- R³: die oben angegebene Bedeutung hat,
durch Umsetzung der Nitrilgruppe in die entsprechenden Amidine überführt, diese mit Hydrazin und anschließend mit Verbindungen der allgemeinen Formel (XII) umsetzt und abschließend mit POCl₃ zu den entsprechenden Verbindungen der allgemeinen Formel (IX) cyclisiert.

Die Verbindungen der allgemeinen Formel (XV) sind an sich bekannt oder nach üblichen Methoden herstellbar.

Die erfindungsgemäßen Verbindungen der allgemeinen Formel (I) zeigen ein nicht vorhersehbares, wertvolles pharmakologisches Wirkspektrum.

Sie inhibieren entweder eine oder mehrere der cGMP-metabolisierenden Phosphodiesterasen (PDE I, PDE II und PDE V). Dies führt zu einem Anstieg von cGMP. Die differenzierte Expression der Phosphodiesterasen in verschiedenen Zellen, Geweben und Organen ebenso wie die differenzierte subzelluläre Lokalisation dieser Enzyme, ermöglichen in Verbindung mit den erfindungsgemäßen selektiven Inhibitoren eine selektive Adressierung der verschiedenen von cGMP regulierten Vorgänge.

Außerdem verstärken die erfindungsgemäßen Verbindungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Daher sind die erfindungsgemäßen Verbindungen der allgemeine Formel (I) geeignet zur Prophylaxe und/oder Behandlung von Erkrankungen, bei denen ein Anstieg der cGMP-Konzentration heilsam ist, d.h. Erkrankungen, die im Zusammenhang mit cGMP-regulierten Vorgängen stehen (im Englischen meist einfach als 'cGMP-related diseases' bezeichnet). Hierzu zählen kardiovaskuläre Erkrankungen, Erkrankungen des Urogenitalsystems sowie cerebrovaskuläre Erkrankungen.

Unter dem Begriff "kardiovaskulären Erkrankungen" im Sinne der vorliegenden Erfindung fallen Erkrankungen wie beispielsweise Bluthochdruck, neuronale Hypertonie, stabile und instabile Angina, periphere und kardiale Gefäßerkrankungen, Arrhythmien, thromboembolische Erkrankungen und Ischämien wie Myokardinfarkt, Hirnschlag, transistorische und ischämische Attacken, Angina pectoris, periphere Durchblutungsstörungen, Verhinderung von Restenosen nach Thrombolysetherapie, percutaner transluminaler Angioplastie (PTA), percutan transluminaler Koronarangioplastien (PTCA) und Bypass.

Weiterhin können die erfindungsgemäßen Verbindungen der allgemeine Formel (I) auch Bedeutung für cerebrovaskuläre Erkrankungen, cerebrale Ischämie, Hirnschlag, Reperfusionsschäden, Hirntrauma, Ödeme oder cerebrale Thrombose haben.

Daneben sind die vorliegenden Verbindungen auch geeignet zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern-und Gedächtnisproblemen, Alzheimersche Krankheit, Vaskuläre Demenz, Demenz mit Lewy-Körperchen, Demenz mit Degeneration der Frontallappen einschliesslich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die relaxierende Wirkung auf glatte Muskulatur macht sie geeignet für die Behandlung von Erkrankungen des Urogenitalsystems wie Prostatahypertrophie, Inkontinenz sowie insbesondere zur Behandlung der erektilen Dysfunktion und der weiblichen sexuellen Dysfunktion.

### Aktivität der Phosphordiesterasen (PDE's)

Die cGMP-stimulierbare PDE II, die cGMP-hemmbare PDE III und die cAMP-spezifische PDE IV wurden entweder aus Schweine- oder Rinderherzmyokard isoliert. Die Ca²⁺-Calmodulin stimulierbare PDE I wurde aus Schweineaorta, Schweinehirn oder bevorzugt aus Rinderaorta isoliert. Die cGMP spezifische PDE V wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990) und C. Lugman et al. Biochemical Pharmacology Vol. 35 1743-1751 (1986).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, dass während der Inkubationszeit von 30 min ca. 50% des Substrates umgesetzt werden. Um die cGMP stimulierbare PDE II zu testen, wird als Substrat ³HcAMP verwendet und dem Ansatz 10⁻⁶ mol/l nicht markiertes cGMP zugesetzt. Um die Ca²⁺-Calmodulinabhängige PDE I zu testen, werden dem Reaktionsansatz noch 1 µM CaCl₂ und 0,1 µM Calmodulin zugesetzt. Die Reaktion wird durch Zugabe von 100 µl Acetonitril, das 1 mM cAMP und 1 mM AMP enthält, gestoppt. 100 µl des Reaktionsansatzes werden mittels HPLC getrennt und die Spaltprodukte "Online" mit einem Durchflussscintillationszähler quantitativ bestimmt. Es wird die Substanzkonzentration gemessen, bei der die Reaktionsgeschwindigkeit um 50 % vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt. Für die Aktivitätsbestimmung der PDE II wurde der [³H] cAMP SPA assay verwendet, wobei dem Reaktionsansatz 10⁻⁶ M cGMP zur Aktivierung des Enzyms zugegeben wurde. Für die Messung der PDE I wurden 10⁻⁷ M Calmodulin und 1 µM CaCl₂ zum Reaktionsansatz zugegeben. Die PDE V wurde mit dem [³H] cGMP SPA assay gemessen.

Grundsätzlich führt die Inhibition einer oder mehrerer Phosphodiesterasen dieses Typs zu einer Erhöhung der cGMP-Konzentration. Dadurch sind die Verbindungen interessant für alle Therapien, in denen eine Erhöhung der cGMP-Konzentration als heilsam angenommen werden kann.

Die Untersuchung der kardiovaskulären Wirkungen wurden an normotonen und an SH-Ratten und an Hunden durchgeführt. Die Substanzen wurden intravenös oder oral appliziert.

Die Untersuchung auf erektionsauslösende Wirkung wurde am wachen Kaninchen durchgeführt [H. Naganuma, T. Egashira, J. Fuji, Clinical and Experimental Pharmacology and Physiology 20, 177-183 (1993)]. Die Substanzen wurden oral oder parenteral appliziert.

Die neuen Wirkstoffe sowie ihre physiologisch unbedenklichen Salze (z. B. Hydrochloride, Maleinate oder Lactate) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, z. B. perlingual , buccal, intravenös, nasal, rektal oder inhalativ.

Für die Anwendung beim Menschen werden bei oraler Administration im allgemeinen Dosierungen von 0,001 bis 50 mg/kg vorzugsweise 0,01 mg/kg - 20 mg/kg verabreicht. Bei parenteraler Administration, wie z. B. über Schleimhäute nasal, buccal, inhalativ, ist eine Dosierung von 0,001 mg/kg - 0,5 mg/kg sinnvoll.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der oben genannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die erfindungsgemäßen Verbindungen sind auch zur Anwendung in der Tiermedizin geeignet. Für Anwendungen in der Tiermedizin können die Verbindungen oder ihre nicht toxischen Salze in einer geeigneten Formulierung in Übereinstimmung mit den allgemeinen tiermedizinischen Praxen verabreicht werden. Der Tierarzt kann die Art der Anwendung und die Dosierung nach Art des zu behandelnden Tieres festlegen.

Die vorliegende Erfindung wird durch die folgenden Beispiele veranschaulicht, die die Erfindung jedoch keineswegs beschränken sollen.

Bei den im folgenden aufgeführten Shukrurformeln, in denen der Rest 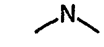 vorhanden ist, ist stets 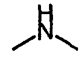 gemeint.

### Herstellung der Vorstufen

### Beispiel 1A

### 2-Butyrylaminopropionsäure

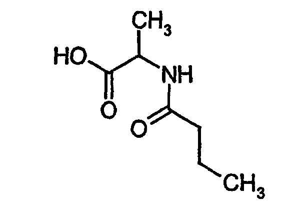

22.27g (250 mmol) D,L-Alanin und 55.66 g (550 mmol) Triethylamin werden in 250 ml Dichlormethan gelöst und die Lösung auf 0°C abgekühlt. 59,75 g (550 mmol) Trimethylsilylchlorid werden zugetroft und die Lösung 1 Stunde bei Raumtemperatur und eine Stunde bei 40°C gerührt. Nach dem Abkühlen auf -10°C werden 26.64 g (250 mmol) Buttersäurechlorid zugetropft und die resultierende Mischung 2 Stunden bei -10°C und eine Stunde bei Raumtemperatur gerührt. Unter Eiskühlung werden 125 ml Wasser zugetropft und die Reaktionsmischung 15 Minuten bei Raumtemperatur gerührt. Die wässrige Phase wird bis zur Trockene eingedampft, der Rückstand mit Aceton verrieben und die Mutterlauge abgesaugt. Nach dem Entfernen des Lösungsmittels wird der Rückstand chromatographiert. Das erhaltene Produkt wird in 3N Natronlauge gelöst und die resultierende Lösung bis zur Trockene eingedampft. Es wird mit konz. HCl aufgenommen und wieder bis zur Trockene eingedampft. Es wird mit Aceton verrührt, vom ausgefallenen Feststoff abgesaugt und das Lösungsmittel im Vakuum entfernt. Man erhält 28.2 g (71 %) eines zähen Öls, das nach einiger Zeit kristallisiert.
200 MHz ¹H-NMR (DMSO-d6): 0.84 (t, 3H); 1.22 (d, 3H); 1.50 (hex, 2H); 2.07 (t, 2H); 4.20 (quin., 1H); 8.09 (d, 1H).

### Beispiel 2A

### 2-Butyrylamino-buttersäure

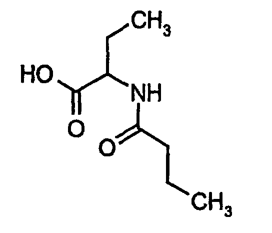

25.78 g 2-Aminobuttersäure (250 mmol) und 55.66 g (550mmol) Triethylamin werden in 250 ml Dichlormethan gelöst und die Lösung auf 0°C abgekühlt. 59.75 g (550 mmol) Trimethylsilylchlorid werden zugetroft und die Lösung 1 Stunde bei Raumtemperatur und eine Stunde bei 40°C gerührt. Nach dem Abkühlen auf -10°C werden 26.64 g (250 mmol) Buttersäurechlorid zugetropft und die resultierende Mischung 2 Stunden bei -10°C und eine Stunde bei Raumtemperatur gerührt. Unter Eiskühlung werden 125 ml Wasser zugetropft und die Reaktionsmischung 15 Minuten bei Raumtemperatur gerührt. Die organische Phase wird mit Natronlauge versetzt und das organische Lösungsmittel im Vakuum entfernt. Nach dem Ansäuern wird der ausgefallene Feststoff 1 mal mit Wasser und 2 mal mit Petrolether verrührt und im Vakuum bei 45°C getrocknet. 29.1 g (67 %) farbloser Feststoff.
200 MHz ¹H-NMR (DMSO-d6):0.88 (2t, 6H); 1.51 (quart., 2H); 1.65 (m, 2H); 2.09 (t, 2H); 4.10 (m, 1H); 8.01 (d, 1H); 12.25 (s,m 1H).

### Beispiel 3A

### 2-(2-Ethyl)-butanoylaminopropionsäure

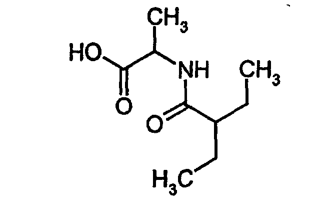

Man legt 24,5 g (0,275 mol) D,L-Alanin in 250 ml Dichlormethan vor und setzt 61,2 g (0,605 mol) Triethylamin hinzu. Man kühlt auf 0°C und tropft 65,7 g (0,605 mol) Trimethylsilylchlorid hinzu. Man rührt eine Stunde bei Raumtemperatur und eine Stunde bei 40°C. Man kühlt auf -10°C und tropft langsam 37 g (0,275 mol) 2-Ethylbuttersäurechlorid hinzu. Man lässt zwei Stunden bei -10°C und über Nacht bei Raumtemperatur rühren. Man kühlt im Eisbad und tropft 150 ml Wasser hinzu. Man setzt 50 g (1,25 mol) NaOH in 100 ml Wasser gelöst hinzu, trennt die wässrige Phase ab und engt sie ein. Der Rückstand wird erneut in Wasser aufgenommen, mit konzentrierter Salzsäure angesäuert, die wässrige Lösung wird mehrfach mit Dichlormethan extrahiert, die organische Phase über Na₂SO₄ getrocknet und eingeengt.
Ausbeute: 43,55 g (84,6 % der Theorie)
200 MHz ¹H-NMR (CDCl₃): 0,91 (t, 6H); 1,5 (d, 3H); 1,52-1,73 (m, 4H); 1,99 (m, 1H); 4,61 (quin, 1H); 6,25 (d, 1H); 6,76 (bs, 1H).

### Beispiel 4A

### 2-(2,2-Dimethyl)-pentanoylamino-propionsäure

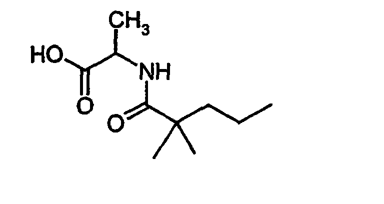

48,04 g (344,2 mmol) D,L-Alaninmethylester-hydrochlorid und 76,67 g (757,2 mmol) Triethylamin werden in 600 ml Dichlormethan gelöst und bei 0°C werden 56 g (344,2 mmol) 2,2-Dimethylpentansäurechlorid in 50 ml Dichlormethan zugetropft. Es wird 2 h bei RT gerührt, abfiltriert, mit 10%iger HCl-Lösung, gesättigter Natriumhydrogencarbonatlösung und gesättigter Natriumchloridlösung gewaschen. Nach Trocknen über Natriumsulfat wird eingedampft. Der Rückstand wird in Methanol aufgenommen und mit einer Lösung von 55 g (1377 mmol) Natriumhydroxid in 300 l Wasser versetzt. Man lässt 2 Stunden bei RT rühren, filtriert ab und dampft das Methanol im Vakuum ab. Die wässrige Phase wird mit konzentrierter Salzsäurelösung sauer gestellt und mit Essigsäureethylester (2 x) ausgeschüttelt. Die vereinigten Essigsäureethylesterphasen werden über Natriumsulfat getrocknet und eingedampft. Der Rückstand wird aus Ether kristallisiert.
Ausbeute: 30 g (40,5%)
Fp.: 168°C

### Beispiel 5A

### 2-Heptanoylamino-propionsäure

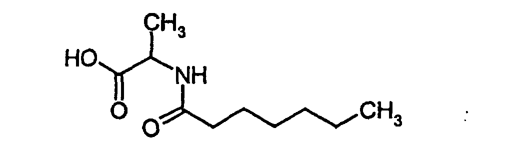

30 g (291 mmol) D,L-Alaninmethylester-hydrochlorid und 64,77 g (640 mmol) Triethylamin weden in 300 ml trockenem Methylenchlorid bei 0°C vorgelegt. Dazu werden 43,24 g (291 mmol) Heptansäurechlorid in 50 ml Methylenchlorid zugetropft. Man lässt auf RT kommen und rührt 2 h bei dieser Temperatur nach. Der Niederschlag wird abfiltriert, die Methylenchloridphase mit gesättigter Natriumhydrogencarbonatlösung und mit gesättigter Natriumchloridlösung ausgeschüttelt und über Natriumsulfat getrocknet. Das Lösemittel wird im Vakuum entfernt und der Rückstand in 300 ml Methanol gelöst. Zu dieser Lösung werden 300 ml Wasser, in denen 46,55 g (1164 mmol) Natriumhydroxid gelöst sind, zugegeben und 2 h bei RT gerührt. Es wird filtriert, das Methanol wird abrotiert und die zurückbleibende Wasserphase wird mit konz. Hcl auf pH 1-2 angesäuert. Das ausgefallene Produkt wird abfiltriert und getrocknet. Durch Extrahieren der Wasserphase mit Essigsäureethylester wird eine zweite Fraktion des Produktes gewonnen.
Ausbeute: 50 g (85,4%)
¹H-NMR (CD₃OD): 0,9 (t, 3H); 1,2 - 1,4 (m, 9H); 1,6 (quin., 2H); 2,2 (t, 2H); 4,38 (quar., 1H).

### Beispiel 6A

### 2-Octanoylamino-propionsäure

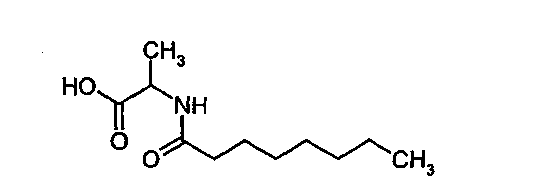

Die Herstellung erfolgt analog der Vorschrift des Beispiels 1A aus 16,5 g (0,185 mol) D,L-Alanin, 41,23 g (0,407 mol) Triethylamin, 44,27 g (0,407 mol) Trimethylsilylchlorid und 30,12 g (0,185 mol) Octansäurechlorid. Das Produkt kristallisiert aus Toluol/n-Hexan aus.
Ausbeute: 34,3 g (86%)
¹H-NMR (CD₃OD): 0,9 (t, 3H); 1,2 - 1,4 (m, 11H); 1,6 (quin. 2H); 2,2 (t, 2H); 4,35 (quar 1H).

### Beispiel 7A

### 2-Decanoylamino-propionsäure

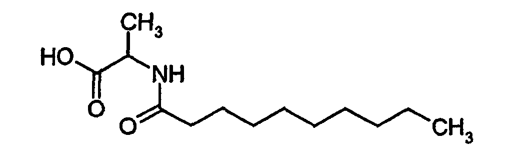

Die Herstellung erfolgt analog der Vorschrift des Beispiels 4A aus 19,0 g (184 mmol) D,L-Alaninmeihylester-hydrochlorid und 35,14 g (184 mmol) Decansäurechlorid.
Ausbeute: 37,3 g (83,2 %)
¹H-NMR (CD₃OD): 0,9 (t, 3H); 1,2 - 1,4 (m, 15H); 1,6 (m, 2H); 2,2 (t, 2H); 4,35 (quar., 1H).

### Beispiel 8A

### 2-(2-Ethyl)-octanoylamino-propionsäure

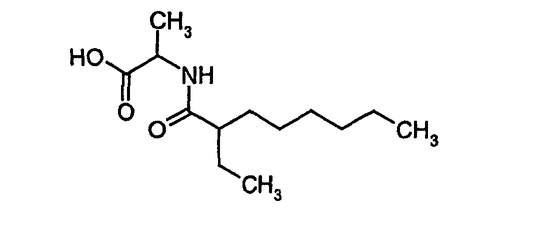

18,6 g (0,211 mol) D,L-Alanin und 46,6 g (0,41 mol) Triethylamin werden in 300 ml Dichlormethan vorgelegt. Bei 0°C werden 50,09 g (0,461 mol) Trimethylsilylchlorid zugetropft und 1 h bei Raumtemperatur, dann 1 h bei 40°C gerührt. Die Lösung wird auf -10°C gekühlt und 40 g (0,21 mol) 2-Ethyloctansäurechlorid in 50 ml Dichlormethan zugetropft. Nach Rühren über Nacht bei Raumtemperatur werden unter Eiskühlung 100 ml Wasser zugetropft und 10 Minuten nachgerührt. Die Phasen werden getrennt, die wässrige Phase mit noch 2 mal mit je 100 ml Dichlormethan ausgeschüttelt, die vereinigten organischen Phasen werden über Natriumsulfat getrocknet und im Vakuum eingedampft. Der Rückstand wird aus Toluol durch Zugabe von n-Hexan kristallisiert und bei 60°C getrocknet.
Ausbeute: 3,9 g (78,2%)
¹H-NMR (CDCl₃): 0,9 (m, 6h); 1,25 (pseudo s, 8H); 1,45 (d, 3H); 1,4 - 1,7 (m, 4H); 2,0 (m, 1H); 4,6 (quin. 1H); 6,1 (d, 1H).

### Beispiel 9A

### 2-Cyclopentanoylamino-propionsäure

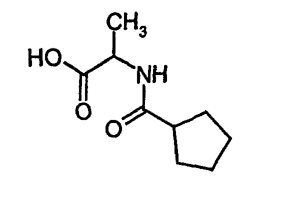

16,8 g (0,189 Mol)-D, L-Alanin und 41,98 g (0,415 Mol) Triethylamin werden in 200 ml Dichlormethan vorgelegt. Bei 0°C werden 45,07 g (0,415 mol) Trimethylsilylchlorid zugetropft und 1 h bei Raumtemperatur, dann 1 h bei 40°C gerührt. Die Lösung wird auf -10°C abgekühlt und 25 g (0,189 mol) Cyclopentancarbonsäurechlorid zugetropft. Es wird 2 h bei -10°C und 1 h bei Raumtemperatur gerührt. Unter Eiskühlung werden 100 ml Wasser zugetropft, 10 Min. nachgerührt und der ausgefallene Niederschlag abgesaugt. Der Niederschlag wird mit 300 ml Wasser, danach mit 300 ml Diethylether nachgewaschen und anschließend bei 60°C getrocknet.
Ausbeute: 25,8 g (73,9% d.Th.)
¹H-NMR (CD₃OD): 1,35 (d, 3H); 1,5 - 1,9 (m, 8H); 2,7 (quin, 1H); 4,5 (quar., 1H):

### Beispiel 10A

### 2-Cyclopentanoylamino-buttersäure

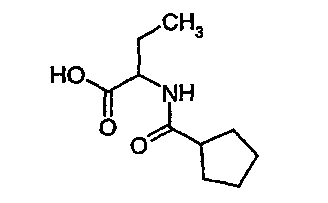

10.31 g 2-Aminobuttersäure (100 mmol) und 22.26 g (220 mmol) Triethylamin werden in 100 ml Dichlormethan gelöst und die Lösung auf 0°C abgekühlt. 23.90 g (220 mmol) Trimethylsilylchlorid werden zugetropft und die Lösung 1 Stunde bei Raumtemperatur und eine Stunde bei 40°C gerührt. Nach dem Abkühlen auf -10°C werden 13.26g (100mmol) Cyclopentancarbonsäurechlorid zugetropft und die resultierende Mischung 2 Stunden bei -10°C und eine Stunde bei Raumtemperatur gerührt.
Unter Eiskühlung werden 50 ml Wasser zugetropft und die Reaktionsmischung 15 Minuten bei Raumtemperatur gerührt. Es wird mit Wasser und Dichlormethan verdünnt und der ausgefallene Niederschlag abgesaugt: 11.1 g (55 %) farbloser Feststoff. Die Dichlormethanphase wird über Natriumsulfat getrocknet und das Lösungsmittel im Vakuum entfernt. Der Rückstand wird mit Toluol verrührt und der Niederschlag abgesaugt: 5.75 g (28 %) farbloser Feststoff.
200 MHz ¹H-NMR (DMSO-d₆): 0.88 (t, 3H); 1.61 (m, 10H); 2.66 (m, 1H); 4.09 (hex., 1H); 7.97 (d, 1H); 12.44 (s, 1H).

### Beispiel 11A

### 2-Cycloheptanoylamino-propionsäure

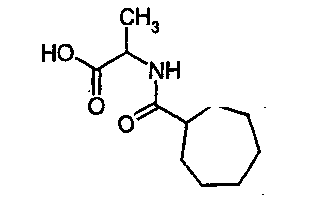

Die Herstellung erfolgt analog der Vorschrift des Beispiels 4A aus 20 g (143 mmol) D,L-Alaninmethylester-hydrochlorid und 23,02 g (143 mmol) Cycloheptansäurechlorid.
Ausbeute: 16 g (52,4 %)
¹H-NMR (CD₃OD): 1,35 (d, 3H); 1,45 - 1,65 (m, 8H); 1,7 - 1,95 (m, 4H); 2,35 (m, 1H); 4,25 (quar., 1H).

### Beispiel 12A

### 2-Ethoxy-benzonitril

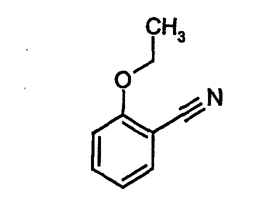

25 g (210 mmol) 2-Hydroxybenzonitril werden mit 87g Kaliumcarbonat und 34.3 g (314.8 mmol) Ethylbromid in 500 ml Aceton über Nacht refluxiert. Es wird vom Feststoff abfiltriert, das Lösungsmittel im Vakuum entfernt und der Rückstand im Vakuum destilliert. Man erhält 30.0 g (97 %) einer farblosen Flüssigkeit.
200 MHz ¹H-NMR (DMSD-d₆): 1.48 (t, 3H); 4.15 (quart., 2H); 6.99 (dt, 2H); 7.51 (dt, 2H).

### Beispiel 13A

### 2-Ethoxy-benzamidinhydrochlorid

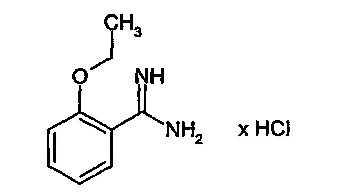

21.4 g (400 mmol) Ammoniumchlorid werden in 375 ml Toluol suspendiert und die Suspension auf 0°C abgekühlt. 200 ml einer 2M Lösung von Trimethylaluminium in Hexan werden zugetropft und die Mischung bis zur beendeten Gasentwicklung bei Raumtemperatur gerührt. Nach Zugabe von 29.44 g (200 mmol) 2-Ethoxybenzonitril wird die Reaktionsmischung über Nacht bei 80°C (Bad) gerührt. Die abgekühlte Reaktionsmischung wird unter Eiskühlung zu einer Suspension aus 100g Kieselgel und 950 ml Chloroform gegeben und die Mischung 30 Minuten bei Raumtemperatur gerührt. Es wird abgesaugt und mit der gleichen Menge Methanol nachgewaschen. Die Mutterlauge wird eingedampft, der erhaltene Rückstand mit einer Mischung aus Dichlormethan und Methanol (9:1) verrührt, der Feststoff abgesaugt und die Mutterlauge eingedampft. Man erhält 30.4 g (76 %) farblosen Feststoff.
200 MHz ¹H-NMR (DMSO-d₆): 1.36 (t, 3H); 4.12 (quart., 2H); 7.10 (t, 1H); 7.21 (d, 1H); 7.52 (m, 2H); 9.30 (s, breit, 4H).

### Beispiel 14A

### 2-Propoxybenzonitril

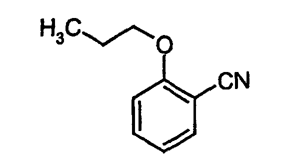

75 g (630 mmol) 2-Hydroxybenzonitril werden mit 174 g (1,26 Mol) Kaliumcarbonat und 232,3 g (1,89 Mol) n-Propylbromid in 1 1 Aceton über Nacht refluxiert. Es wird vom Feststoff abfiltriert, das Lösemittel im Vakuum entfernt und der Rückstand im Vakuum destilliert.
Kp.: 89°C (0,7 mbar)
Ausbeute: 95,1g (93,7 % d.Th.)

### Beispiel 15A

### 2-Propoxybenzamidin-hydrochlorid

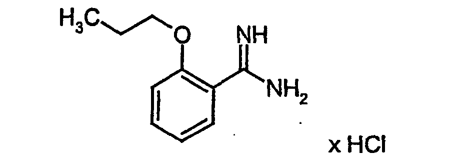

21,41 g (400 ml) Ammoniumchlorid werden in 400 ml Toluol suspendiert und auf 0-5°C gekühlt. 200 ml einer 2 M Lösung von Triethylaluminium in Hexan werden zugetropft und die Mischung bis zur beendeten Gasentwicklung bei Raumtemperatur gerührt. Nach Zugabe von 32,2 g (200 mmol) 2-Propoxybenzonitril wird die Reaktionsmischung über Nacht bei 80°C (Bad) gerührt. Die abgekühlte Reaktionsmischung wird unter Eiskühlung zu einer Suspension von 300 g Kieselgel und 2,85 ml eisgekühltem Chloroform gegeben und 30 Minuten gerührt. Es wird abgesaugt und mit der gleichen Menge Methanol nachgewachen. Das Lösemittel wird im Vakuum abdestilliert, der Rückstand in 500 ml einer Mischung aus Dichlormethan und Methanol (9:1) verrührt, der Feststoff abfiltriert und die Mutterlauge eingedampft. Der Rückstand wird mit Petrolether verrührt und abgesaugt. Man erhält 22,3 g (52%) Produkt.
200 MHz ¹H-NMR (CD₃OD): 1,05 (t, 3H); 1,85 (sex, 2H); 4,1 (t, 2H); 7,0 - 7,2 (m, 2H); 7,5 - 7,65 (m, 2H).

### Beispiel 16A

### 2-(2-Ethoxyphenyl)-5-methyl-7-propyl-3H-imidazo[5,1-f][1,2,4]-triazin-4-on

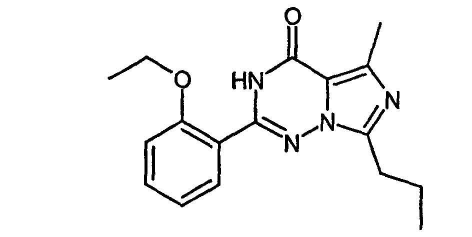

7.16 g (45 mmol) 2-Butanoylamino-propionsäure (Beispiel 1A) werden mit 10.7 g Pyridin in 45 ml Tetrahydrofuran gelöst und nach Zugabe einer Spatelspitze 4-Dimethylaminopyridin zum Rückfluss erhitzt. 12.29 g (90 mmol) Oxalsäuremonoethylesterchlorid werden langsam zugetropft und die Reaktionsmischung wird 3 Stunden refluxiert. Es wird auf Eiswasser gegossen, 3 mal mit Essigsäureethylester extrahiert, über Natriumsulfat getrocknet und eingedampft. Der Rückstand wird in 15 ml Ethanol aufgenommen und mit 2.15 g Natriumhydrogencarbonat 2.5 Stunden refluxiert. Die abgekühlte Lösung wird filtriert.
Zu einer Lösung von 9.03 g (45 mol) 2-Ethoxy-benzamidin-hydrochlorid (Beispiel 13A) in 45 ml Ethanol werden unter Eiskühlung 2.25 g (45 mmol) Hydrazinmonohydrat zugetropft und noch 10 Minuten bei Raumtemperatur gerührt. Zu dieser Suspension wird die oben beschriebene ethanolische Lösung getropft und 4 Stunden bei 70°C gerührt. Nach Filtration wird die Lösung eingedampft, der Rückstand zwischen Dichlormethan und Wasser verteilt und die organische Phase nach dem Trocknen über Natriumsulfat eingedampft.
Der Rückstand wird in 60 ml 1,2-Dichlorethan aufgenommen und es werden 7.5 ml Phosphoroxytrichlorid zugetropft. Nach 2 Stunden Rühren unter Rückfluss wird abge-kühlt, mit Dichlormethan verdünnt und 2 mal mit gesättigter Natriumhydrogencarbonat-Lösung ausgeschüttelt. Die organische Phase wird getrocknet und das Lösungsmittel im Vakuum entfernt. Chromatographie mit Essigsäureethylester und Kristallisation ergeben 4.00 g (28.0 % d. Th.) weißer Feststoff.
¹H-NMR (200MHz, CDCl₃): 1.02 (t, 3H), 1.56 (t, 3H), 1.89 (hex, 2H), 2.67 (s, 3H), 3.00 (t, 2H), 4.26 (quar, 2H), 7.05 (m, 2H), 7.50 (dt, 1H), 8.17 (dd, 1H), 10.00 (s, 1H);
DC: R_{f}=0.42 (Dichlormethan:Methanol = 95:5).

### Beispiel 17A

### 2-(2-Ethoxyphenyl)-5-ethyl-7-propyl-3H-imidazo[5,1-f][1,2,4]triazin-4-on

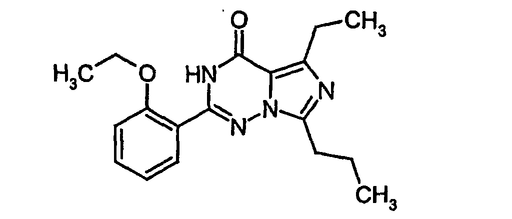

Die Herstellung erfolgt in Analogie zur Vorshrift des Beispiels 16A aus 29,06 g (167,8 mmol) 2-Butanoylaminobuttersäure (Beispiel 2A) und 33,6 (167,8 mmol) 2-Ethoxy-benzamidinhydrochlorid (Beispiel 13A). Die Reinigung erfolgt durch Chromatographie über Kieselgel (Eluens: CH₂Cl₂ / CH₃OH 50:1).
Ausbeute: 7,4 g (12,4%)
R_{f} = 0,46 (CH₂Cl₂ / CH₃OH = 20:1)
¹H-NMR (200 MHz, CDCl₃): 1,32 (t, 3H); 1,57 (t, 32H); 1,94 (m, 8H); 3,03 (quart, 2H); 3,64 (quin, 1H); 4,27 (quart, 2H); 7,06 (d, 1H); 7,12 (t, 1H); 7,50 (dt, 1H); 8,16 (dd, 1H); 9,91 (s, 1H).

### Beispiel 18A

### 2-(2-Ethoxyphenyl)-5-methyl-7-(1-ethylpropyl)-3H-imidazo[5,1-f][1,2,4]-triazin-4-on

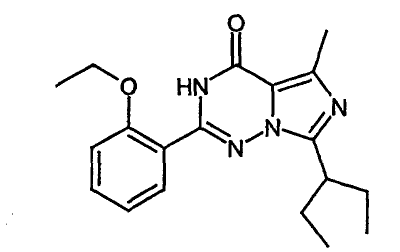

Die Herstellung erfolgt in Analogie zur Vorschrift des Beispiels 16A aus 21.45 g (0.1 mol) 2-(2-Ethyl)-butyrylamino-propionsäure (Beispiel 3A) und 20.6 g (0.1 mol) 2-Ethoxybenz-amidin-hydrochlorid (Beispiel 13A). Die Reinigung erfolgt durch Chromatographie über Kieselgel mit Dichlormethan/Methanol 60: als Eluens.
Ausbeute: 7.22 g (21.3 % d. Th.).
¹H-NMR (200MHz, CDCl₃): 0.87 (t, 6H), 1.57 (t, 3H), 1.88 (m, 4H), 2.67 (s, 3H), 3.28 (m, 1H), 4.28 (quar, 2H), 7.05 (d, 1H), 7.13 (dt, 1H), 8.15 (dd, 1H).

### Beispiel 19A

### 2-(2-Ethoxyphenyl)-5-methyl-7-(1,1-dimethylbutyl)-3H-imidazo[5,1-f][1,2,4]triazin-4-on

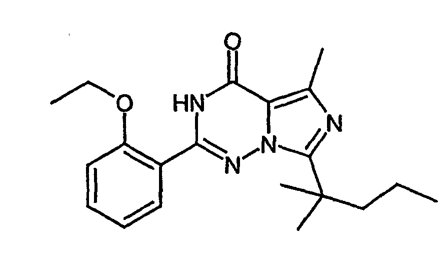

Die Herstellung erfolgt in Analogie zur Vorschrift des Beispiels 16A aus 22,85 g (0,1 mol) 2-(2,2-Dimethyl)pentanoylamino-propionsäure (Beispiel 4A) und 20,6 g (0,1 mol) 2-Ethoxybenzamidinhydrochlorid (Beispiel 13A). Die Reinigung erfolgt durch Chromatographie über Kieselgel (Eluens: CH₁Cl₂ / CH₃OH = 50:1).
Ausbeute: 6,56 g (18,5%)
¹H-NMR (200 MHz, CD₃OD): 0,82 (t, 3H); 1,1 (m, 2H); 1,45 (t, 3H); 1,5 (s, 6H); 1,95 (m, 2H); 2,57 (s, 3h); 4,2 (quar., 2H); 7,1 (t, 1H); 7,18 (d, 1H); 7,52 (dt, 1H); 7,72 (dd, 1H).

### Beispiel 20A

### 2-(2-Ethoxyphenyl)-5-methyl-7-hexyl-3H-imidazo]5,1-f][1,2,4]-triazin-4-on

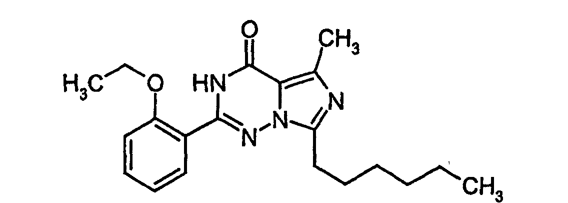

Die Herstellung erfolgt in Analogie zur Vorschrift des Beispiels 16A aus 14,1 g (70 mmol) 2-Heptanoylamino-propionsäure (Beispiel 5A) und 14,05 g (70 mmol) 2-Ethoxybenzamidin-hydrochlorid (Beispiel 13A). Die Reinigung des Produktes erfolgt durch Chromatographie über Kieselgel mit Petrolether / Essigsäureethylester 1:1 als Eluens.
Ausbeute: 3,5 g (14,1%)
¹H-NMR (CD₃OD): 0,9 (t, 3H); 1,3 - 1,45 (m, 6H); 1,4 (t, 3H); 1,7 - 1,9 (m, 2H); 2,15 (s, 3H); 3,1 (t, 2H); 4,2 (quar., 2H); 7,1 (t, 1H); 7,15 (d, 1H); 7,05 (td, 1H); 7,7 (dd, 1H).

### Beispiel 21A

### 2-(2-Ethoxyphenyl)-5-methyl-7-heptyl-3H-imdazo-[5,1-f][1,2,4]triazin-4-on

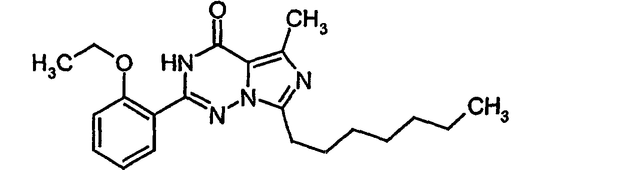

Die Herstellung erfolgt in Analogie zur Vorschrift des Beispiels 16A aus 14,7 g (68,1 mmol) 2-Octanoylamino-propionsäure (Beispiel 6A) und 13,66 g (68,1 mmol) 2-Ethoxybenzamidin-hdyrochlorid (Beispiel 13A). Die Reinigung des Produktes erfolgt durch Chromatographie über Kieselgel mit Dichlormethan / Methanol 50:1 als Eluens.
Ausbeute: 4,65 g (18,5 %), Öl
¹H-NMR (CD₃OD): 0,85 (t, 3H); 1,2 - 1,4 (m, 8H); 1,45 (t, 3H); 2,8 (quin, 2H); 2,6 (s, 3H); 3,0 (t, 2H); 4,2 (quar, 2H); 7,1 (t, 1H); 7,2 (d, 1H); 7,55 (td, 1H), 7,7 (dd, 1H).

### Beispiel 22A

### 2-(2-Ethoxyphenyl)-5-methyl-7-nonyl-3H-imidazo[5,1-f]-[1,2,4-]-triazin-4-on

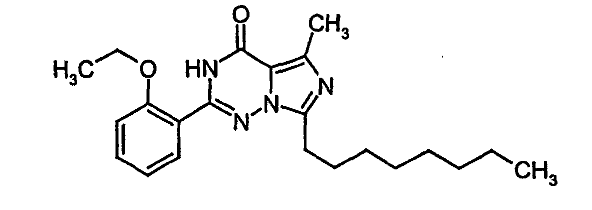

Die Herstellung efolgt in Analogie zur Vorschrift des Beispiels 16A aus 17,0 g (70 mmol) 2-Decanoylamino-propionsäure (Beispiel 7A) und 14,05 g (70 mmol) 2-Ethoxybenzamidin-hydrochlorid (Beispiel 13A). Die Reinigung des Produktes erfolgt durch Chromatographie über Kieselgel mit Petrolether / Essigsäureethylester 1:1 als Eluens.
Ausbeute: 3,5 g (14,1 %)
¹H-NMP, (CD₃OD): 0,9 (t, 3H); 1,3 - 1,45 (m, 6H); 1,4 (t, 3H); 1,7 - 1,9 (m, 2H); 2,15 (s, 3H); 3,1 (t, 2H); 4,2 (quar., 2H); 7,1 (t, 1H); 7,15 (d, 1H); 7,05 (td, 1H), 7,7 (dd, 1H).

### Beispiel 23A

### 2-(2-Ethoxyphenyl)-5-methyl-7-(2-ethylheptyl)-3H-imidazo[5,1-f][1,2,4]-triazin-4-on

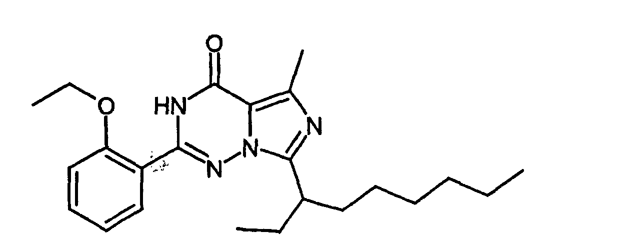

Die Herstellung erfolgt in Analogie zur Vorschrift des Beispiels 16A aus 10.95 g (45 mmol) 2-(2-Ethyl)-octanoylamino-propionsäure (Beispiel 8A) und 9.03 g (45 mmol) 2-Ethoxybenzamidin-hydrochlorid (Beispiel 13A). Die Reinigung erfolgt durch Chromatographie über Kieselgel mit Cyclohexan/Essigsäureethylester.
Ausbeute: 7.22 g (21.3 % d. Th.).
¹H-NMR (200MHz, CDCl₃): 0.75-0.90 (m, 6H), 1.10-1.40 (m, 8H), 1.50 (t, 3H), 1.80-2.05 (m, 4H), 2.70 (s, 3H), 3.40 (quin., 1H), 4.30 (t, 2H), 7.05-7.20 (pseudo quar, 2H), 7.50 (td, 1H), 8.20 (dd, 1H), 10.40 (s, 1H).

### Beispiel 24A

### 2-(2-Propoxyphenyl)-5-methyl-7-(2-ethylheptyl)-3H-imidazo[5,1-f][1,2,4]triazin-4-on

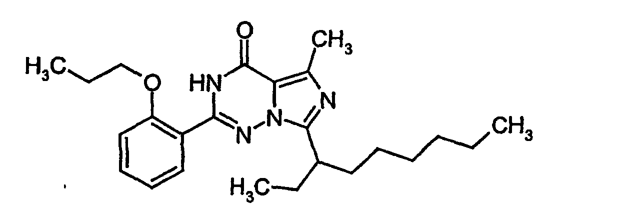

Die Herstellung erfolgt in Analogie zur Vorschrift des Beispiels 16A aus 10,95 g (45 mmol 2-(2-Ethyl)-octanoylamino-propionsäure (Beispiel 8A) und 9,66 g (45 mmol) 2-Propoxybenzamidin-hydrochlorid (Beispiel 13A). Die Reinigung des Produktes erfolgt durch Chromatographie über Kieselgel mit Dichlormethan / Methanol 60:1 als Eluens.
Ausbeute: 3,7 g (20 %), gelbes Öl
¹H-NMR (CDCl₃): 0,75 - 0,9 (m, 6H); 1,15 (t, 3h); 1,1 - 1,35 (m, 8H); 1,75 - 2,1 (m, 6h); 2,7 (s, 3H); 3,4 (quin, 1H); 4,2 (t, 2H); 7,05 - 7,2 (pseudo quar, 2H); 7,5 (td, 1H), 8,2 (dd, 1H); 10,2 (breit, 1H).

### Beispiel 25A

### 2-(2-Ethoxyphenyl)-5-methyl-7-cyclopentyl-3H-imidazo[5,1-f][1,2,4]-triazin-4-on

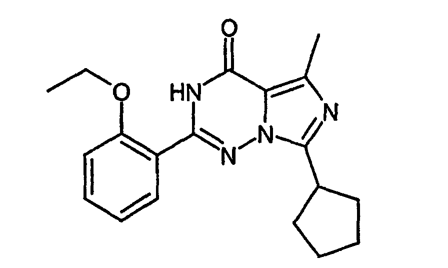

Die Herstellung erfolgt in Analogie zur Vorschrift des Beispiels 16A aus 19,9 g (100 mmol) 2-Cyclopentanoylamino-propionsäure (Beispiel 9A) und 20 g (100 mmol) Ethoxybenzamidin-hydrochlorid (Beispiel 13A). Die Reinigung erfolgt durch Chromatographie über Kieselgel mit Methylenchlorid/Methanol 50:1.
Ausbeute: 7,1 g (20,9 %)
¹H-NMR (200 MHz, CD₃OD): ,45 (t, 3H); 1,65 - 1,80 (m, 2H); 1,80-2,00 (m, 4H); 2,05 - 2,20 (m, 2H); 2,60 (s, 3H); 3,65 (quin., 1H); 4,20 (quar., 2H); 7,10 (t, 1H); 7,15 (d, 1H); 7,50 (t, 1H); 7,70 (d, 1H).

### Beispiel 26A

### 2-(2-Propoxyphenyl)-5-methyl-7-cyclopentyl-3H-imidazo[5,1-f][1,2,4]-triazin-4-on

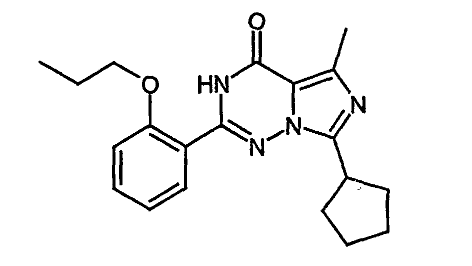

Die Herstellung erfolgt in Analogie zur Vorschrift des Beispiels 16A aus 8.33 g (45.0 mmol) 2-Cyclopentanoylamino-propionsäure (Beispiel 9A) und 9.65 g (45.0 mmol) 2-Propoxybenzamidin-hydrochlorid (Beispiel 15A). Die Reinigung erfolgt durch Chromatographie über Kieselgel mit Dichlormethan/Methanol 50:1 als Eluens. Das Produkt kann aus Essig-säureethylester/Petrolether kristallisiert werden.
Ausbeute: 1.82 g (11.5 % d. Th.) weißer Feststoff.
¹H-NMR (200MHz, CDCl₃): 1.15 (t, 3H), 1.70 (m, 2H), 1.95 (m, 4H), 2.15 (m, 2H), 2.65 (s, 3H), 3.65 (quin., 1H), 4.15 (t, 2H), 7.05 (d, 1H), 7.10 (t, 1H), 7.50 (td, 1H), 8.20 (dd, 1H).

### Beispiel 27A

### 2-(2-Ethoxyphenyl)-5-ethyl-7-cyclopentyl-3H-imidazo[5,1-f][1,2,4]triazin-4-on

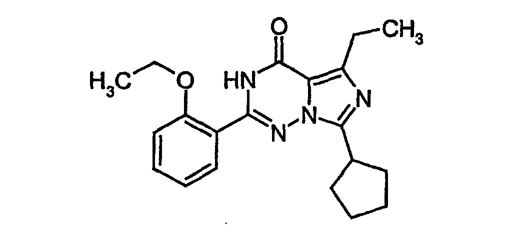

Die Herstellung erfolgt in Analogie zur Vorschrift des Beispiels 16A aus 8,77 g (44 mmol) 2-Cyclopentanoylamino-buttersäure (Beispiel 10A) und 8,83 g (44 mmol) 2-Ethoxybenzamidin-hydrochlorid (Beispiel 13A). Die Reinigung des Produktes erfolgt durch Chromatographie über Kieselgel mit Cyclohexan / Essigsäureethylester (6:4) als Eluens.
Ausbeute: 0,355 g (6,7 %), weißer Feststoff
¹H-NMR (CDCl₃): 1,32 (t, 3H); 1,57 (t, 3H); 1,94 (m, 8H); 3,03 (quar, 2H); 3,64 (quin, 1H); 4,27 (quar, 2H), 7,06 8d, 1H); 7,12 (t, 1H); 7,50 (t, 1H); 8,16 (dd, 1H); 9,91 (s, 1H).

### Beispiel 28A

### 2-(2-Ethoxyphenyl)-5-methyl-7-cycloheptyl-3H-imidazo[5,1-f][1,2,4]-triazin-4-on

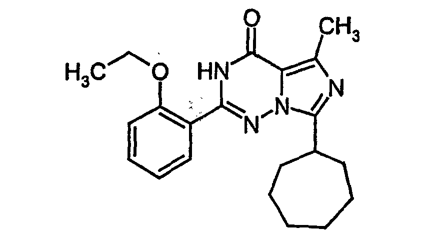

Die Herstellung erfolgt in Analogie zur Vorschrift des Beispiels 16A aus 14,9 g (70 mmol) 2-Cycloheptanoylamino-propionsäure (Beispiel 11A) und 14 g (70 mmol) 2-Ethoxybenzamidin-hydrochlorid (Beispiel 13). Die Reinigung des Produktes erfolgt durch Chromatographie über Kieselgel mit Methylenchlorid / Methanol 10:1, anschließend 50:1 als Eluens.
Ausbeute: 5.35 g (20,9%)
¹H-NMR (CD₃OD): 1,45 (t, 3H); 1,6 - 2,0 (m, 10H); 2,1 - 2,2 (m, 2H); 2,7 (s, 3H); 3,65 (quin., 1H); 4,2 (quar., 2H); 7,1 (t, 1H); 7,2 (d, 1H); 7,6 (td, 1H); 7,75 (dd, 1H).

### Beispiel 29A

### 2-(2-Ethoxyphenyl)-5-ethyl-7-cycloheptyl-3H-imidazo[5,1-f][1,2,4]-triazin-4-on

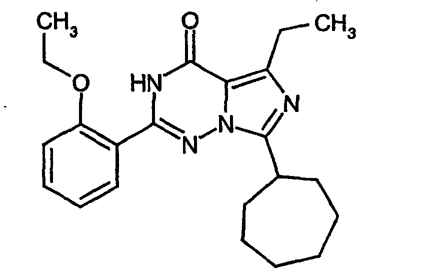

Die Herstellung erfolgt in Analogie zur Vorschrift des Beispiels 16A aus 1,02 g (4,5 mmol) 2-Cycloheptanoylamino-buttersäur (Beispiel 12A) und 0,98 g (4,9 mmol) 2-Ethoxybenzamidin-hydrochlorid (Beispiel 13A). Die Reinigung erfolgt durch Chromatographie über Kieselgel mit Essigsäureethylester / Cyclohexan 1:1.
Ausbeute: 0,391 mg (14 %)
¹H-NMR (200 MHz, D₆-DMSO): δ = 1.21 (t, 3H, CH₃), 1.30 (t, 3H, CH₃), 1.40-2.01 (m, 12H, CH₂), 2.86 (g, 2H, CH₂), 3.32 (m, 1H, CH), 4.10 (g, 2H, CH₂), 7.05 (t, 1H), 7.15 (d, 1H), 7.51 (m, 2H), 11.50 (bs, 1H, NH).

### Beispiel 30A

### 4-Benzyloxy-2-bromphenol

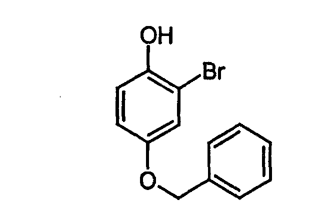

183 g 4-Benzyloxyphenol (914 mmol) werden gem. Literatur (J.C.S Perkin 1, 1981, 2123) bromiert. Nach Umkristallisation aus Petrolether (mit 5 % Ether) wird das Produkt als farbloser Feststoff erhalten.
Ausbeute: 189 g (74.1 % der Theorie)
MS (DCI, NH₃): m/z (%) = 296/298 (M+18) (100)
¹H-NMR (200 MHz, CDCl₃): δ = 4.96 (s, 2 H); 5.19 (s, 1H); 6.70-6.95 (m, 2 H); 7.10 (d, 1 H); 7.39-7.45 (m, 5 H).

### Beispiel 31A

### 5-Benzyloxy-2-ethoxybrombenzol

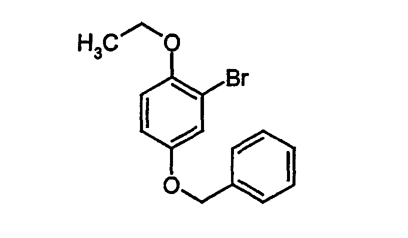

186.18 g 4-Benzyloxy-2-bromphenol (667 mmol) (Beipsiel 30A) wird mit Kaliumcarbonat (276.56 g, 2 mol) in 2 1 Aceton vorgelegt. Man troft 74.7 ml Bromethan (1 mol) hinzu und rührt 24 h unter Rückfluss nach. Es wird abfiltriert und eingedampft. Der erhaltene ölige Rückstand wird in 1200 ml Ethanol gelöst. Unter kräftigem Rühren wird durch langsame Zugabe von 900 ml Wasser das Produkt auskristallisiert. Man saugt ab und trocknet die hellbeigen Kristalle im Hochvakuum.
Ausbeute 178.9 g (95.1 % der Theorie)
MS (DCI, NH₃): m/z (%) = 326/328 (M+18) (100)
¹H-NMR (200 MHz, CDCl₃): δ = 1.45 (t, 3 H); 4.05 (q, 2 H); 4.98 (s, 2 H), 6.79-6.90 (m, 2 H); 7.18-7.46 (m, 6 H).

### Beispiel 32A

### 5-Benzyloxy-2-ethoxybenzonitril

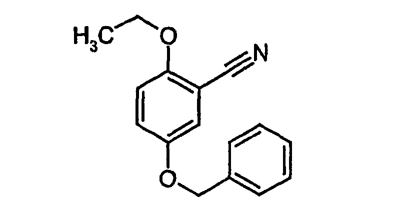

Zu 57.14 g Kupfercyanid (638 mmol) werden 178.17 g 5-Benzyloxy-2-ethoxybrombenzol (580 mmol) (Beispiel 31A) gegeben und durch Umschütteln vermischt. Nach Addition von 65 ml trockenem Pyridin wird die Mischung auf 160°C erwärmt. Das Gemisch schmilzt und bildet eine homogene Lösung. Es wird 6 h bei 160°C nachgerührt. Nach Abkühlen auf ca.100°C wird Toluol zugegeben und verrührt bis das Reaktionsgemisch abgekühlt ist. Man filtriert über Kieselgur und wäscht mehrfach mit Toluol nach. Dann wird solange mit verdünnter Ammoniaklösung gewaschen, bis die wässrige Phase nicht mehr blau gefärbt ist. Man wäscht mit gesättigter Natriumchloridlösung, trocknet und dampft ein. Der erhaltene Rückstand wird aus 500 ml Ethanol umkristallisiert, vollständige Kristallisation wird durch Zusatz von 100 ml Wasser erreicht. Man saugt ab und wäscht mehrfach mit Petrolether nach. Die bräunlichen Kristalle werden bei 45°C im Vakuum getrocknet.
Ausbeute 140.4 g (92.5 % der Theorie)
MS (DCI, NH₃): m/z (%): 271 (M+18) (100)
¹H-NMR (200 MHz, CDCl₃): δ = 1.45 (t, 3 H), 4.08 (q, 2 H); 5.01 (s, 2 H); 6.85-6.90 (1 H); 7.10-7.18 (2 H), 7.31-7.42 (m, 5 H).

### Beispiel 33A

### 3-Benzyloxy-6-ethoxybenzamidinhydrochlorid

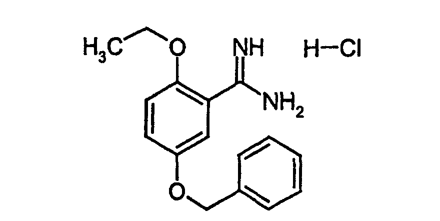

46.46 g Ammoniumchlorid (868.5 mol) werden in 650 ml Toluol suspendiert und auf 0-5°C abgekühlt. Man tropft Trimethylaluminium als 2M Lösung in Hexan (445 ml, 888.3 mmol) zu und rührt anschließend so lange bei Raumtemperatur nach bis die Gasentwicklung beendet ist. 5-Benzyloxy-2-ethoxybenzonitril (100 g, 394.8 mmol) (Beispiel 32A) wird zugegeben und über Nacht bei 80°C nachgerührt. Der abgekühlte Ansatz wird unter Eiskühlung zu einer Suspension aus 200 g Kieselgel und 2 1 Dichlormethan gegeben und 30 min verrührt. Man saugt ab und wäscht mit Methanol nach. Man vereinigt die organischen Phasen und dampft ein. Der erhaltene Rückstand wird mit einem Gemisch aus Dichlormethan/Methanol 9:1 verrührt, filtriert und einrotiert. Anschließend verrührt man mit Ether und saugt den farblosen Feststoff ab.
Ausbeute 59.4 g (49 % der Theorie)
¹H-NMR (300 MHz, D₆-DMSO): δ = 1.32 (t, 3 H); 4.09 (q, 2 H); 5.15 (s, 2 H); 7.10-7.49 (m, 8 H); 9.1-9.5 (m, 3).

### Beispiel 34A

### 2-[5-(benzyloxy)-2-ethoxyphenyl]-7-cyclopentyl-5-methyl-3H-imidazo[5,1-f][1,2,4]triazin-4-on

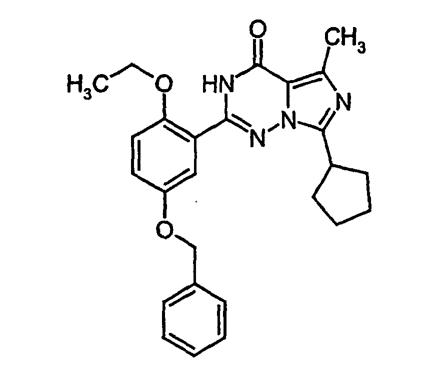

4.07 g 2-Cyclopentanoylamino-propionsäure (22 mmol) (Beispiel 9A) werden in 22 ml trockenem Tetrahydrofuran und 5.3 ml Pyridin (66 mmol) vorgelegt, 0.13 g 4-DMAP werden zugegeben und die Mischung wird wird auf Rückfluss erhitzt. Langsam wird Oxalsäureethylesterchlorid (6.7 ml, 44 mmol) zugetropft und die resultierende Suspension wird zwei Stunden unter Rückfluss erhitzt, bevor nach Abkühlung mit Ethylacetat verdünnt, filtriert und die wässrige Phase mit 1N Salzsäure (2x), gesättigter Natriumhydrogencarbonatlösung (2x) und gesättigter Natriumchloridlösung gewaschen, über Natriumsulfat getrocknet und konzentriert wird. Erhalten wird nach Trocknen im Hochvakuum ein gelbes Öl, das gelöst in 13 ml Ethanol zu einer folgendermaßen bereiteten Lösung gegeben wird:
3.37 g 3-Benzyloxy-6-ethoxybenzamidinhydrochlorid (11 mmol) (Beispiel 33A) werden in 13 ml Ethanol vorgelegt, auf 0°C abgekühlt und 1.13 g Hydrazinhydrat (16.5 mmol) werden zugetropft. Man erwärmt auf ∼ 40 °C und rührt 10 min nach.

Nach Ende der Zugabe ethanolischer Lösung wird die Mischung bei 70°C für 3.5 h gerührt. Es wird konzentriert und nach Trocknen im Hochvakuum wird der gelbe Schaum in 100 ml 1,2-Dichlorethan aufgelöst, mit 2 ml Phosphoroxychlorid versetzt und 1.5 h auf Rückfluss erhitzt. Nach Abkühlen wird mit Dichlormethan verdünnt und mit gesättigter Natriumhydrogencarbonatlösung und gesättigter Natriumchloridlösung gewaschen. Die organische Phase wird mit Magnesiumsulfat getrocknet und eingeengt. Der dunkle Rückstand wird in Ethylacetat aufgenommen und nach Zugabe von Petrolether tritt Fällung auf. Nach Abfiltrieren wird das Filtrat eingeengt und der Rückstand chromatographiert (Cyclohexan/Ethylacetat 3:2). Das Produkt wird aus Ethylacetat/Petrolether umkristallisiert.
Ausbeute 632 mg (12.9 % der Theorie)
MS (DCI, NH₃): m/z (%) = 445 (M+H) (100)
¹H-NMR (200 MHz, CDCl₃): δ = 1.55 (t, 3 H); 1.20-2.21 (m, 8 H); 2.55 (s, 3 H); 3.61 (qui, 1 H); 4.21 (q, 2 H); 5.12 (s, 2 H); 6.98 (d, 1 H); 7.11 (dd, 1 H); 7.32-7.50 (m, 5 H); 7.78 (d, 1 H); 10.10 (s, 1 H).

### Beispiel 35A

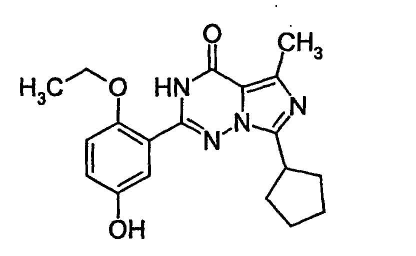

611 mg der Verbindung aus Beispiel 34A (1.37 mmol) werden in 13 ml Ethanol suspendiert. 13 ml Ether und einige Tropfen Essigsäure werden hinzugefügt (keine vollständige Auflösung). Zur Suspension unter Argon-Atmosphäre werden 200 mg 10 % Pd/C gegeben, die Suspension wird mehrmals mit Wasserstoff gespült, danach 2 h unter H₂-Atmosphäre (1 atm) kräftig gerührt, bevor über Celite abfiltriert wird. Das Filtrat wird eingeengt, am Hochvakuum getrocknet und der Rückstand mit Ether/Petrolether behandelt, filtriert und am Hochvakuum getrocknet.
Ausbeute 395 mg (81.5 % der Theorie)
MS (DCI, NH₃): m/z (%) = 355 (M+H) (100)

### Beispiel 36A

### Ethoxy-5-hydroxybenzonitril

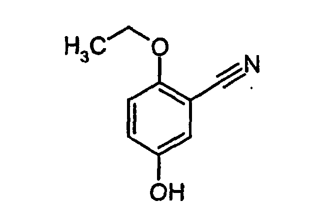

40.02 g 5-Benzyloxy-2-ethoxybenzonitril (158 mmol) (Beispiel 32A) werden mit 5 % Pd/C (4.0 g) in 1 l Methanol vorgelegt. Anschließend wird unter Wasserstoffatmosphäre (1 atm) ca. 4 h hydriert. Man filtriert über Kieselgur, dampft ein und trocknet den kristallinen Rückstand im Vakuum.
Ausbeute 25.5 g (99.6 % der Theorie)
¹H-NMR (200 MHz, CDCl₃): δ = 1.43 (t, 3 H); 4.05 (q, 2 H), 6.75-6.88 (m, 1 H); 7.0-7.07 (m, 2 H).

### Beispiel 37A

### 5-Allyloxy-2-ethoxybenzonitril

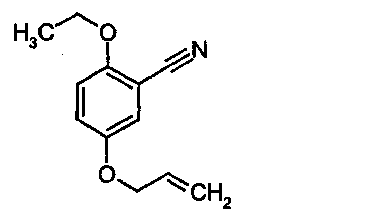

25 g 2-Ethoxy-5-hydroxybenzonitril (153.2 mmol) (Beispiel 36A) werden mit Kaliumcarbonat (63.52 g, 459.6 mmol) in 750 ml Aceton vorgelegt. Man gibt 19.9 ml Allylbromid (229.8 mmol) zu und rührt über Nacht unter Rückfluss nach. Es wird abfiltriert und eingedampft; erhalten wird ein oranges, dünnflüssiges Öl.
Ausbeute 31 g (99.6 % der Theorie)
¹H-NMR (200 MHz, D₆-DMSO): δ = 1.45 (t, 3 H); 4.10 (q, 2 H); 5.28-5.95 (m, 2 H); 5.92-6.11, m 1 H); 6.85-6.92 (m, 1 H); 7.06-7.13 (m, 2 H).

### Beispiel 38A

### 3-Allyloxy-6-ethoxybenzamidinhydrochlorid

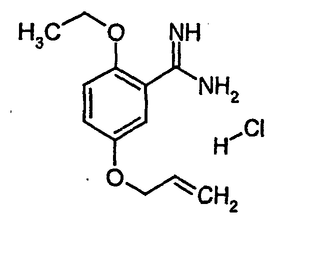

17.95 g Ammoniumchlorid (335.56 mmol) werden in Toluol suspendiert und auf 0-5°C abgekühlt. Man tropft Trimethylaluminium (2M Lösung in Hexan, 172 ml, 343.2 mmol) zu und rührt anschließend so lange bei Raumtemperatur nach bis die Gasentwicklung beendet ist. Dann wird 5-Allyloxy-2-ethoxybenzonitril (31 g, 152.5 mmol) (Beispiel 37A) zugegeben und über Nacht bei 80°C nachgerührt. Der abgekühlte Ansatz wird dann zu einer Mischung aus 100 g Kieselgel und 1 1 Dichlormethan gegeben und 30 min verrührt. Man saugt ab, wäscht zweimal mit Methanol nach und dampft ein. Der erhaltene Rückstand wird mit Dichlormethan/Methanol 9:1 verrührt, abfiltriert und einrotiert. Der Rückstand besteht aus einer rotbräunlichen halbkristallienen Masse. Mit Aceton erhält man nach Filtration 10 g farblosen Feststoff Die Mutterlauge ergibt nach Einrotieren 21 g zähes, rötliches Öl, das in wenig Dichlormethan gelöst wird. Es wird mit etwas Produkt angeimpft und über Nacht stehengelassen. Man erhält nach Absaugen und Waschen mit etwas Aceton noch weitere 6 g Feststoff.
Ausbeute 16 g (38.6 % der Theorie)
MS (DCI, NH₃): m/z (%) = 221 (M-Cl) (100)
¹H-NMR (200 MHz, D₆-DMSO): δ = 1.32 (t, 3 H); 4.08 (q, 2 H); 4.60 (d, 2 H); 5.35-5.97 (m, 2 H); 5.94-6.15 (m, 1 H); 7.13-7.22 (m, 3 H); 9.2/9.35 (2x s, zus. 4 H).

### Beispiel 39A

### 2-[5-(allyloxy)-2-ethoxyphenyl]-7-cyclopentyl-5-methyl-3H-imidazo[5,1-f][1,2,4]triazin-4-on

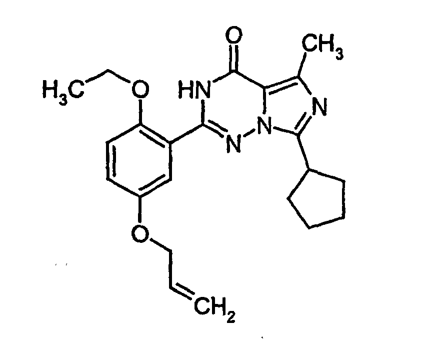

14 .82 g 2-Cycloentanoylamino-propionsäure (Beispiel 9A) (80 mmol) werden in 80 ml trockenem Tetrahydrofuran und 19.4 ml Pyridin (240 mmol) vorgelegt, 0.49 g 4-DMAP werden zugegeben und die Mischung wird wird auf Rückfluss erhitzt. Langsam wird Oxalsäureethylesterchlorid (17.9 ml, 160 mmol) zugetropft und die resultierende Suspension wird zwei Stunden unter Rückfluss erhitzt. Man gießt auf Eiswasser und extrahiert dreimal mit Ethylacetat. Es wird getrocknet und einrotiert. Der erhaltene ölige Rückstand wird in Methanol aufgenommen, mit Natriumhydrogencarbonat versetzt und 2,5 Std gekocht. Nach Abkühlen wird abfiltriert. Das Filtrat wird zu einer Lösung gegeben, die folgendermaßen bereitet wurde: 15 g 3-Allyloxy-6-ethoxybenzamidinhydrochlorid (58.4 mmol) (Beispiel 38A) werden in Ethanol unter Eiskühlung vorgelegt. Man tropft Hydrazinhydrat (3.07 g, 61.3 mmol) während 10 min zu und rührt dann noch 30 min bei Raumtemperatur nach.
Nach 4 h bei 70°C. wird eingeengt und der Rückstand in 80 ml 1,2-Dichlorethan aufgenommen, mit 10 ml Phosphoroxychlorid versetzt und 1 h unter Rückfluss nachgerührt. Man verdünnt mit Dichlormethan und stellt mit Natriumhydrogencarbonat neutral. Es wird nochmal mit Wasser gewaschen, dann getrocknet und einrotiert. Das Rohprodukt wird durch Flash-Chromatographie über Kieselgel mit Cyclohexan/Ethylacetat 1:1 vorgereinigt. Der erhaltene ölige Rückstand wird mit Ether kristallisiert. Nach erneuter Kristallisation aus Cyclohexan/Ethylacetat 1:1 werden 3.34 g Feststoff gewonnen. Die Mutterlauge wird einrotiert und mit Dichlormethan/Aceton 95:5 chromatographiert. Es werden weitere 2.9 g Produkt erhalten.
Ausbeute 6.24 g (27.1 % der Theorie)
MS (DCI, NH₃): m/z (%) = 395 (M+H) (100)
¹H-NMR (200 MHz, CDCl₃): δ = 1.56 (t, 3 H); 1.69-2.21 (m, 8 H); 2.65 (s, 3 H); 3.65 (qui, 1 H); 4.21 (q, 2 H); 4.59 (dd, 2 H); 5.30-5.51 (m, 2 H); 5.99-6.28 (m, 1 H); 6.95-7.09 (m, 2 H); 7.75 (d, 1 H); 10.10 (s, 1 H).

### Beispiel 40A

### 7-Cyclopentyl-2-[2-ethoxy-5-(2-oxiranylmethoxy)phenyl]-5-methyl-3H-imidazo[5,1-f][1,2,4]triazin-4-on

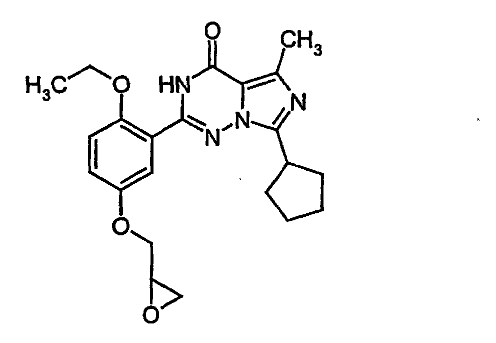

4.26 g (10.8 mmol) der Verbindung aus Beispiel 39A werden in Dichlormethan gelöst. Man gibt Metachlorperbenzoesäure (7.64 g, tech., circa 50 %, 22.1 mmol) zu und rührt 7 h bei Raumtemperatur nach. Man filtriert, wäscht mit Dichlormethan nach, wäscht anschließend mit Thiosulfitlösung und 3 mal mit Natriumhydrogecarbonatlösung, trocknet und dampft ein. Nach Flash-Chromatographie mit Ethylacetat/Cyclohexan 6:4 werden 1.6 g Edukt LMP 45-1 und 460 mg des Produkts erhalten.
Ausbeute 460 mg (9.3 % der Theorie)
MS (DCI, NH₃): m/z (%) = 411 (M+H) (100)
¹H-NMR (200 MHz, CDCl₃): δ = 1.55 (t, 3 H); 1.68-2.21 (m, 8 H); 2.67 (s, 3 H); 2.80 (dd, 1 H); 2.95 (t, 1 H); 3.38-3.41 (m, 1 H); 3.67 (qui, 1 H); 3.98 (dd, 1 H); 4.12-4.32 (m, 3 H); 6.92-7.13 (m, 2 H); 7.78 (d, 1 H).

### Beispiel 41A

### 2-(2-Ethoxy-5-nitrophenyl)-5-methyl-7-cyclopentyl-3H-imidazo[5,1-f][1,2,4]-triazin-4-on

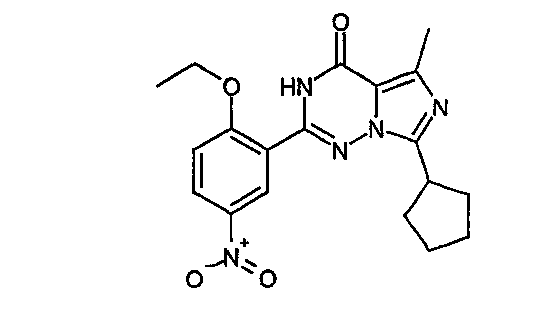

48.6 ml Trifluoressigsäure (TFE) und 12.1 ml 70 %ige Salpetersäure werden mittels Eis-Aceton-Bad auf -10°C gekühlt und 3.0 g (8.86 mmol) der Verbindung aus Beispiel 25A gelöst in 7 ml TFE hinzugetropft und 20 Stunden bei 0°C gerührt. Die Reaktionslösung wird in 400 ml Eiswasser und 200 ml Dichlormethan eingerührt und mit ca. 200 ml gesättigter Natriumhydrogencarbonat-Lösung neutralisiert. Die wässrige Schicht wird abgetrennt, 3 mal mit Dichlormethan extrahiert und die vereinigten organischen Phasen werden getrocknet und eingeengt. Der Rückstand wird über Kieselgel mit Toluol unter Zusatz von Essigsäureethylester mit einem Gradienten von 11 bis 60% chromatographiert.
Ausbeute: 2.56 g (75.5% d. Th.)
¹H-NMR (200 MHz, DMSO): 1.37 (t, 3H), 1.58-2.00 (m, 8H), 2.49 (s, 3H), 3.50 (quin., 1H), 4.26 (quar, 2H), 7.39 (d, 1H), 8.39-8.47 (m, 2H), 11.77 (s, 1H).

### Beispiel 42A

### 2-(5-Amino-2-ethoxyphenyl)-5-methyl-7-cyclopentyl-3H-imidazo[5,1-f][1,2,4]-triazin-4-on

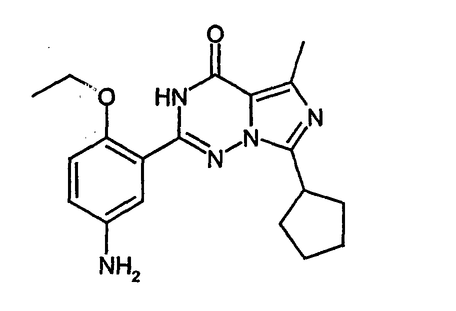

2.56 g (6.68 mmol) der Verbindung aus Beispiel 41A werden in 86 ml Ethanol und 86 ml Tetrahydrofuran in Gegenwart von 288 mg Pd/C (10%) 20 Stunden unter H₂-Atmosphäre gerührt. Die Reaktionslösung wird über 30 ml Kieselgel abgesaugt, mit Ethanol/Tetrahydrofuran nachgewaschen, eingengt und über Nacht im Hochvakuum getrocknet. Das Rohprodukt wird über 500 ml Kieselgel mit Toluol unter Verwendung von Essisäureethylester im Gradientensystem chromatographiert.
Ausbeute: 2.1 g (92.8% d. Th.)
¹H-NMR (200 MHz, DMSO): 1.25 (t, 3H), 1.58-2.0 (m, 8H), 2.48 (s, 3H), 3.41-3.58 (quin., 1H), 3.97 (quar, 2H), 4.92 (s, 2H), 6.69-6.90 (dd und d, 3H), 11.34 (s, 1H).

### Beispiel 43A

### 2-(5-Nitro-2-propoxyphenyl)-5-methyl-7-cyclopentyl-3H-imidazo[5,1-f][1,2,4]-triazin-4-on

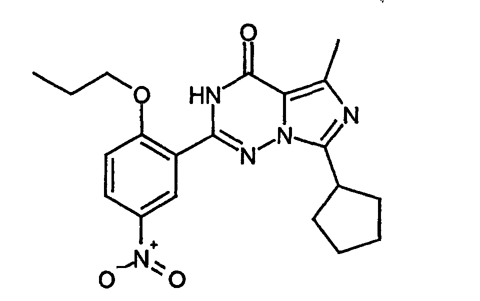

In Analogie zur Vorschrift des Beispiels 41A werden 10.0 g (28.4 mmol) der Verbindung aus Beispiel 26A in 160 ml Trifluoressigsäure und 40 ml 70 %iger Salpetersäure nitriert. Die Reinigung des Produktes erfolgt durch Chromatographie über Kieselgel mit Toluol unter Zusatz von Essigsäureethylester im Gradientensystem.
Ausbeute: 5.15 g (45.7% d. Th.).
¹H-NMR (200MHz, DMSO): 0.95 (t, 3H), 1.60-1.93 (m, 8H), 1.93-2.10 (m, 2H), 2.50 (s, 3H), 3.50 (quin., 1H), 4.17 (t, 2H), 7.40 (dd, 1H), 8.38-8.46 (m, 2H), 11.62 (s, 1H).

### Beispiel 44A

### 2-(5-Amino-2-propoxyphenyl)-5-methyl-7-cyclopentyl-3H-imidazo[5,1-f][1,2,4]-triazin-4-on

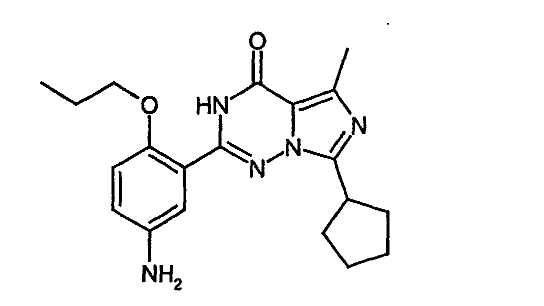

In Analogie zur Vorschrift des Beispiels 42A werden 5.13 g (12.96 mmol) der Verbindung aus Beispiel 43A in Tetrahydrofuran/Ethanol (1:1) mit 1.11 g 10% Pd/C hydriert. Die Reinigung des Produktes erfolgt durch Chromatographie über Kieselgel mit Toluol unter Verwendung von Essigsäureethylester als Lösungsmittelgradient.
Ausbeute: 4.39 g (92.1% d. Th.).
¹H-NMR (200MHz, DMSO): 0.91 (t, 3H), 1.57-2.00 (mm, 10H), 2.45 (s, 3H), 3.41-3.58 (quin., 1H), 3.88 (t, 2H), 4.93 (s, 2H), 6.72 (dd, 1H), 6.80 (d, 1H), 6.90 (d, 1H), 11.30 (s, 1H).

### Beispiel 45A

### 2-(2-Ethoxy-3-nitrophenyl)-5-methyl-7-propyl-3H-imidazo[5,1-f][1,2,4]-triazin-4-on

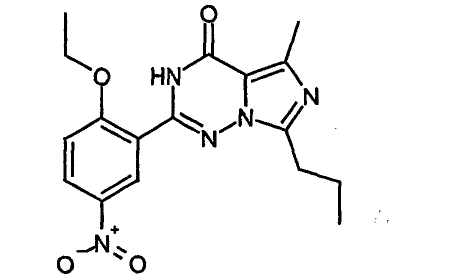

In Analogie zur Vorschrift des Beispiels 41A werden 1.5 g (4.80 mmol) der Verbindung aus Beispiel 16A in 27 ml Trifluoressigsäure und 6.6 ml 70%iger Salpetersäure nitriert.
Ausbeute: 1.73 g (83.7% d. Th.).
MS (ESI): 358 (M+H),
HPLC (analytisch): 83.0% RT: 5.86 min, Säule: Nucleosil C18 (125X4mm), Laufmittel: 0.01m H₃PO₄/ Acetoniril (Gradient), Fluss:2ml/min, 200-400nm,
DC: R_{f}=0.43 (Toluol:Essigester = 2:8).

### Beispiel 46A

### 2-(5-Amino-2-ethoxyphenyl)-5-methyl-7-propyl-3H-imidazo[5,1-f][1,2,4]-triazin-4-on

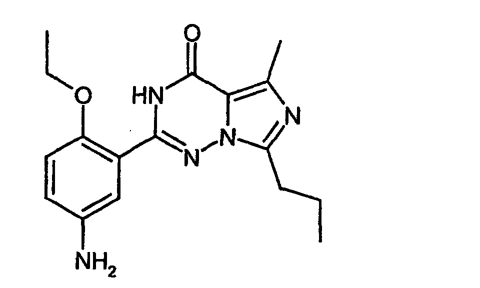

In Analogie zur Vorschrift des Beispiels 42A werden 1.72 g (4.63 mmol) der Verbindung aus Beispiel 45A in 150 ml Ethanol mit 200 mg 10 % Pd/C hydriert.
Ausbeute: 862 mg (56.9% d. Th.).
¹H-NMR (200MHz, DMSO): 0.92 (t, 3H), 1.25 (t, 3H), 1.64-1.82 (hex, 2H), 2.50 (s, 3H), 2.82 (t, 2H), 3.90-4.01 (quar, 2H), 4.93 (s, 2H),6.72 (dd, 1H), 6.80 (d, 1H), 6.90 (d, 1H), 11.35 (s, 1H);
MS (DCI): 354 (M+H).
DC: R_{f}=0.33 (Toluol:Essigester = 1:9).

### Beispiel 47A

### 2-(2-Ethoxy-5-nitrophenyl)-5-methyl-7-(1-ethylpropyl)-3H-imidazo[5,1-f][1,2,4]-triazin-4-on

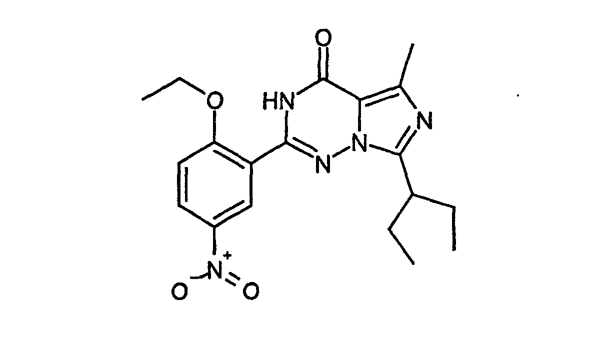

In Analogie zur Vorschrift des Beispiels 41A werden 2.0 g (5.88 mmol) der Verbindung aus Beispiel 18A in 33 ml Trifluoressigsäure und 8.3 ml 70 %iger Salpetersäure nitriert. Die Reinigung des Produktes erfolgt durch Chromatographie über 1000 ml Kieselgel mit Toluol unter Zusatz von Essigsäureethylester im Gradientensystem.
Ausbeute: 1.84 g (81.3 % d. Th.).
¹H-NMR (200MHz, DMSO): 0.73 (t, 6H), 1.16 (t, 3H), 1.61-1.82 (m, 4H), 2.50 (s, 3H), 3.01-3.18 (m, 1H), 4.00 (quar, 2H), 7.49 (t, 1H), 7.91 (dd, 1H), 8.12 (dd, 1H), 12.92 (s, 1H).

### Beispiel 48A

### 2-(5-Amino-2-ethoxyphenyl)-5-methyl-7-(1-ethylpropyl)-3H-imidazo[5,1-f][1,2,4]-triazin-4-on

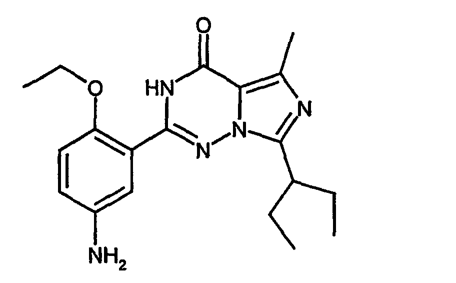

In Analogie zur Vorschrift des Beispiels 42A werden 1.84 g (4.77 mmol) der Verbindung aus Beispiel 47A in 150 g Ethanol mit 200 mg 10% Pd/C hydriert.
Ausbeute: 1.57 g (92.4 % d. Th.).
¹H-NMR (200MHz, DMSO): 0.75 (t, 6H), 1.24 (t, 3H), 1.66-1.84 (m, 4H), 2.50 (s, 3H), 3.11 (quin., 1H), 3.98 (quar, 2H), 4.93 (s, 2H), 6.71 (dd, 1H), 6.78 (d, 1H), 6.90 (d, 1H), 11.33 (s, 1H).

### Beispiel 49A

### 2-(2-Ethoxy-5-nitrophenyl)-5-methyl-7-(2-ethylheptyl)-3H-imidazo[5,1-f][1,2,4]-triazin-4-on

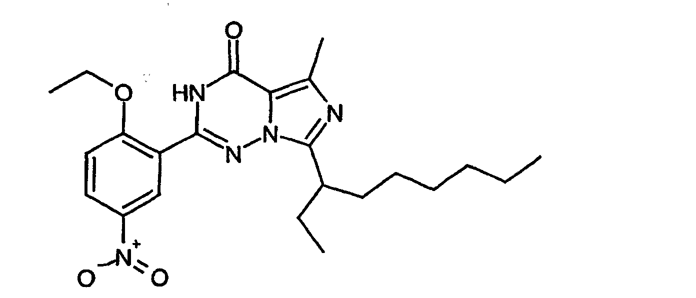

In Analogie zur Vorschrift des Beispiels 41A werden 3.0 g (7.57 mmol) der Verbindung aus Beispiel 23A in 42.5 ml Trifluoressigsäure und 10.7 ml 70 %iger Salpetersäure nitriert. Die Reinigung des Produktes erfolgt durch Chromatographie über 500 ml Kieselgel mit Cyclohexan unter Zusatz von Essigsäureethylester im Gradientensystem von 95:5 bis 40:60.
Ausbeute: 1.95 g (58.4 % d. Th.).
DC: R_{f}=0.65 (Cyclohexan:Essigester = 2:8).

### Beispiel 50A

### 2-(5-Amino-2-ethoxyphenyl)-5-methyl-7-(2-ethylheptyl)-3H-imidazo[5,1-f][1,2,4]-triazin-4-on

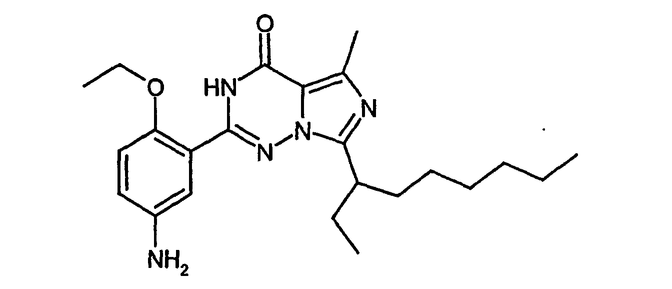

In Analogie zur Vorschrift des Beispiels 42A werden 1.95 g (4.42 mmol) der Verbindung aus Beispiel 49A in 120 g Ethanol mit 200 mg 10 % Pd/C hydriert. Nach der Chromatographie über 400 ml Kieselgel mit Cyclohexan unter Zusatz von Essigsäureethylester im Gradientensystem von 90:10 bis 40:60 erhält man 1.26 g (69.4 % d. Th.).
¹H-NMR (200MHz, DMSO): 0.70-0.83 (m, 6H), 1.11-1.80 (m, 12H), 1.62-1.81 (m, 3H), 2.50 (s, 3H), 3.11-3.25 (quin., 1H), 3.97 (quar, 2H), 4.95 (s, 2H), 6.70-6.80 (m, 2H), 6.90 (d, 1H), 11.35 (s, 1H).

### Beispiel 51A

### 2-(2-Ethoxy-5-chlormethylphenyl)-5-methyl-7-n-propyl-3H-imidazo[5,1-f][1,2,4]-triazin-4-on

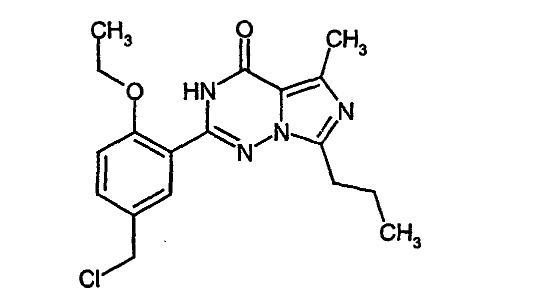

Eine Suspension von 1,50 g (4,8 mmol) der Verbindung aus Beispiel 16A und 0,43 g (4,8 mmol) Paraformaldehyd in 25 ml konz. HCl wurde 2 h auf 120°C erhitzt. Das Reaktionsgemisch wurde auf Eiswasser gegeben, zweimal mit Essigester und anschließend zweimal mit CH₂Cl₂ extrahiert. Die CH₂Cl₂-Phase wurde über MgSO₄ getrocknet und im Vakuum eingeengt. Man erhielt 1,22 g (70,4 %) des gewünschen Produktes.
MS (DCI, NH₃): m/z (%) = 361 [M+H] (100)
¹H-NMR (200 MHz, D₆-DMSO): δ = 0,94 (t, 3H, CH₃); 1,32 (t, 3H, CH₃); 1,82 (g, 2H, CH₂); 2,61 (s, 3H, CH₃); 3,02 (t, 2H, CH₂); 4,12 (g, 2H, CH₂); 4,81 (s, 2H, CH₂); 7,21 (d, 1H); 7,57 - 7,65 (m, 2H); 12,22 (bs, 1H, NH).

### Beispiel 52A

### 2-(2-Ethoxy-5-chlormethylphenyl)-5-ethyl-7-n-propyl-3H-imidazo[5,1-f][1,2,4]-triazin-4-on

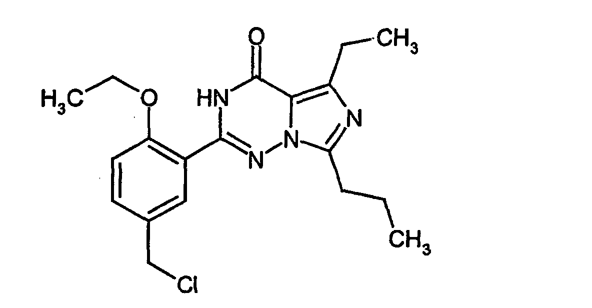

Die Herstellung erfolgt in Analogie zur Vorschrift des Beispiels 51A aus 1 g (3,06 mmol) der Verbindung aus Beispiel 17A und 276 mg (3,06 mmol) Paraformaldehyd.
Ausbeute: 732 mg (59,6 %)

### Beispiel 53A

### 2-(2-Ethoxy-5-chlormethylphenyl)-5-methyl-7-(1,1-dimethylbutyl)-3H-imidazo[5,1-f][1,2,4]-triazin-4-on

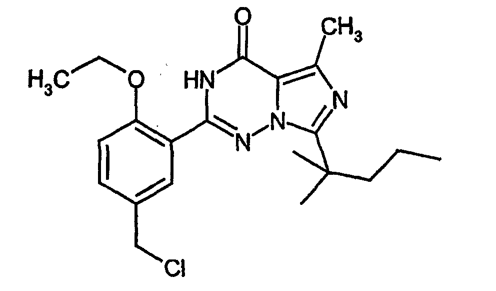

Die Herstellung erfolgt in Analogie zur Vorschrift des Beispiels 51A aus 1,5 g (4,2 mmol) der Verbindung aus Beispiel 19A und 380 mg (4,2 mmol) Paraformaldehyd.
Ausbeute: 850 mg (49,8%)
¹H-NMR (200 MHz, CDCl₃): 0,83 (t, 3H), 1,05 - 1,2 (m, 2H); 1,55 (s, 6H); 1,6 (t, 3H); 1,95 - 2,1 (m, 2H); 2,65 (s, 3H); 4,3 (quar., 2H); 4,62 (s, 2H); 7,05 (d, 1H); 7,53 (dd, 1H); 8,12 (d, 1H); 9,9 (s, 1H).

### Beispiel 54A

### 2-(2-Ethoxy-5-chlormethylphenyl)-5-ethyl-7-cyclopentyl-3H-imidazo[5,1-f][1,2,4]-triazin-4-on

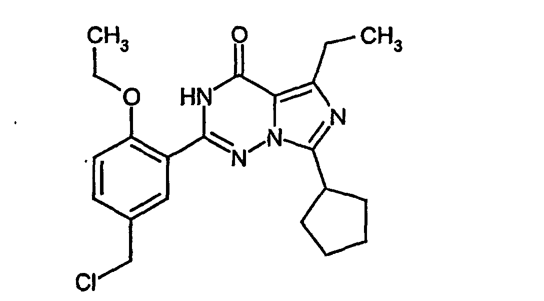

Eine Suspension aus 1.0 g (2.8 mmol) der Verbindung aus Beispiel 27A und 256 mg (2.8 mmol) Paraformaldehyd in 20 ml konz. HCl wurde 2 h auf 120°C erhitzt, wobei eine homogene Lösung entstand. Man goss auf Eiswasser, extrahierte zweimal mit CH₂Cl₂, trocknete die organische Phase über MgSO₄ und engte im Vakuum ein. Durch Umkristallisation aus CH₂Cl₂ / Ether erhielt man 314 mg (27.6 %) des gewünschten Produktes. Durch Einengen der Mutterlauge fielen weitere 806 mg (70.9 %) Produkt an.
MS (EI): m/z (%) = 400 [M⁺] (28)
¹H-NMR (200 MHz, CDCl₃): δ = 1.50 (t, 3H, CH₃), 1.61 (t, 3H, CH₃), 1.62 - 2.45 (m, 8H, 4 x CH₂), 3.33 (t, 2H, CH₂), 3.91 (m, 1H, CH), 4.28 (g, 2H, CH₂O), 4.61 (s, 2H, CH₂), 7.10 (d, 1H), 7.63 (dd, 1H), 8.15 (d, 1H), 10.51 (bs, 1H, NH).

### Beispiel 55A

### 2-(2-Ethoxy-5-chlormethylphenyl)-5-methyl-7-cycloheptyl-3H-imidazo[5,1-f][1,2,4]-triazin-4-on

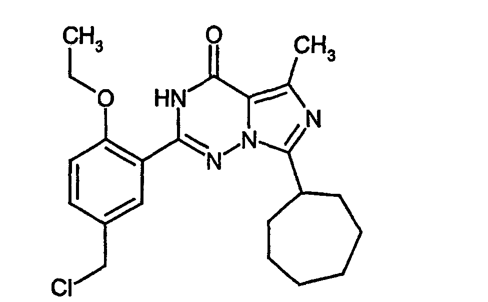

Eine Suspension aus 600 mg (1.6 mmol) der Verbindung aus Beispiel 28A und 147 mg (1.6 mmol) Paraformaldehyd in 10 ml konz. HCl wurde insgesamt 4 h auf 120°C erhitzt, wobei zwischenzeitlich aufschäumendes Reaktionsprodukt vom Kühler gespült wurde. Das Gemisch wurde auf Eiswasser gegossen, die wässrige Phase zweimal mit Essigester extrahiert und die organische Phase über MgSO₄ getrocknet. Nach dem Einengen wurde der Rückstand mit Ether verrieben und vom ausfallenden Produkt abfiltriert. Man erhielt 558 mg eines 9:1 - Gemisches aus Produkt und Edukt und nochmals 189 mg (26.5 %) Produkt durch Einengen der Mutterlauge.
MS (DCI, NH₃): m/z (%) = 415 [ M + H ] (100)
¹H-NMR (200 MHz, D₆-DMSO): δ = 1.30 (t, 3H, CH₃), 1.45 - 2.15 (m, 12H, 6 x CH₂), 2.60 (s, 3H, CH₃), 3.45 (m, 1H, CH), 4.13 (g, 2H, CH₂), 4.82 (s, 2H, CH₂), 7.19 (dd, 1H), 7.62 (m, 2H), 12.18 (bs, 1H, NH).

### Beispiel 56A

### 2-(2-Ethoxy-5-chlormethylphenyl)-5-ethyl-7-cycloheptyl-3H-imidazo[5,1-f][1,2,4]-triazin-4-on

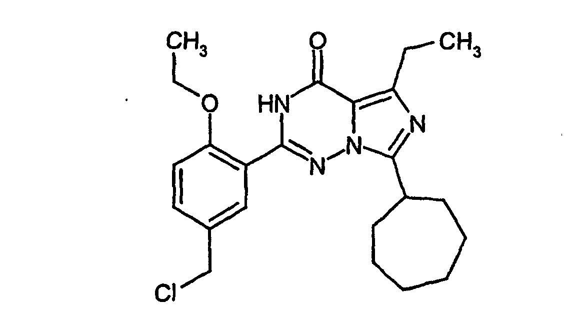

Analog Beispiel 51A wurden 100 mg (0.26 mmol) der Verbindung aus Beispiel 29A in 2 ml konz. HCl mit 23.7 mg (0.26 mmol) Paraformaldehyd 2 h bei 120°C erhitzt. Chromatographische Reinigung (Gradient: CH₂Cl₂ : MeOH = 1 *---->* 50 : 1) lieferte 60.8 mg (53.9 %) des gewünschten Produktes.
MS (DCI, NH₃): m/z (%) = 429 [ M + H ] (100)
¹H-NMR (200 MHz, CDCl₃): δ = 1.32 (t, 3H, CH₃), 1.58 (t, 3H, CH₃), 1.60 - 2.08 (m, 12H, 6 x CH₂), 3.02 (g, 2H, CH₂), 3.44 (m, 1H, CH), 4.26 (g, 2H, CH₂O), 4.63 (s, 2H, CH₂), 7.06 (d, 1H), 7.54 (dd, 1H), 8.16 (d, 1H), 9.84 (bs, 1H, NH).

### Beispiel 57A

### 2-[5-(2-bromacetyl)-2-ethoxyphenyl]-5-methyl-7-propyl.3H-imidazo[5,1-f][1,2,4]-triazin-4-o

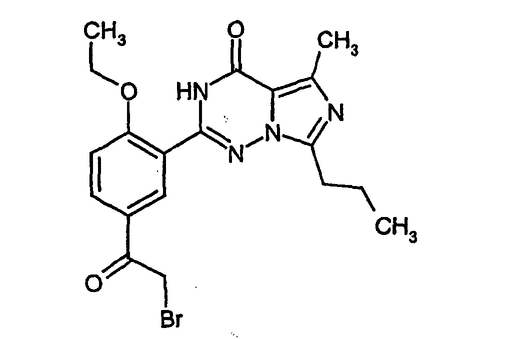

Zu einer auf 0°C gekühlten Lösung aus 4g (12.8 mmol) der Verbindung aus Beispiel 16A in 80 ml CH₂Cl₂ wurden zunächst 5.17g (25.6 mmol) Bromacetylbromid zugetropft und anschließend 5.12g (38.4 mmol) AlCl₃ portionsweise zugegeben. Nach dem Erwärmen auf Raumtemperatur wurde 30 Min. nachgerührt und 2 h unter Rückfluss erhitzt. Das Reaktionsgemisch wurde auf Eiswasser gegeben und einmal mit CH₂Cl₂ extrahiert, die organische Phase mit ges. NaCl-Lsg. gewaschen und über MgSO₄ getrocknet. Nach dem Einengen im Vakuum wurde mit Ether verrührt und das Produkt abgesaugt. Man erhielt 6.2g (> 95 %) des gewünschten Produktes als Gemisch aus Phenacylbromid und Phenacylchlorid.
MS (ESI): m/z = 435 [M (Br) + H] (100), 389 [ M (Cl) + H] (85)
¹H-NMR (200 MHz, D₆-DMSO) : δ = 0.94 (t, 3H, CH₃), 1.32 (t, 3H, CH₃), 1.78 (m, 2H, CH₂), 2.61 (s, 3H, CH₃), 3.03 (t, 2H, CH₂), 4.25 (g, 2H, CH₂), 4.89 (s, 2H, CH₂-Br), 5.18 (s, 2H, CH₂-Cl), 7.34 (d, 1H), 8.07 - 8.25 (m, 2H), 12.40 (bs, 1H, NH)

### Beispiel 58A

### 2-[5-(2-bromacetyl)-2-ethoxyphenyl]-5-ethyl-7-propyl-3H-imidazo[5,1-f][1,2,4]triazin-4-on

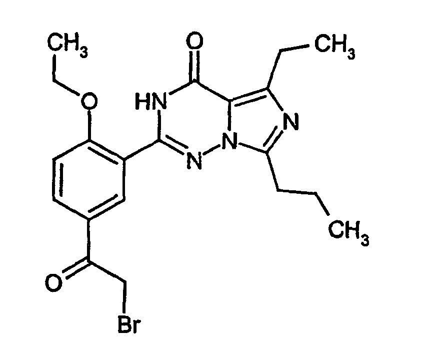

Zu einer auf 0°C gekühlten Lösung aus 1g (3.1 mmol) der Verbindung aus Beispiel 17A in 80 ml CH₂Cl₂ wurden 1.2 g (6.1 mmol) Bromacetylbromid zugetropft und portionsweise 1.2 g (9.1 mmol) AlCl₃ zugegeben. Man erwärmte auf Raumtemperatur (30 Min.), erhitzte dann für 2 h auf Rückfluss und goss das Reaktionsgemisch vorsichtig auf Eiswasser. Nach der Extraktion mit CH₂Cl₂, dem Trocknen über MgSO₄ und Einengen im Vakuum, wurde der Rückstand mit Ether verrieben. Man erhielt 1.33 g (nach LC-MS = 33 % ig) des gewünschten Produktes, welches ohne weitere Reinigung weiter umgesetzt wurde.
MS (ESI): m/z (%) = 447 [M + H] (100)

### Beispiel 59A

### 2-[5-(2-bromacetyl)-2-ethoxyphenyl]-5-methyl-7-cyclopentyl-3H-imidazo[5,1-f][1,2,4]triazin-4-on

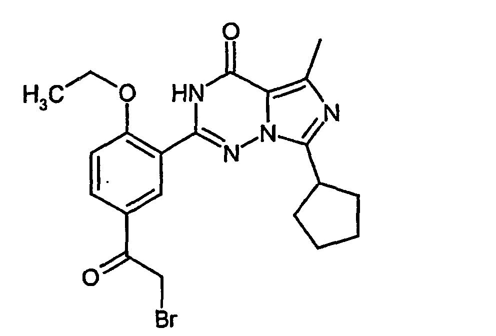

Die Verbindung wurde analog der Verbindung aus Beispiel 57A aus 1 g (2,95 mmol) der Verbindung aus Beispiel 25A und 1,19 g (5,9 mmol) Bromacetylbromid in Gegenwart von 1,18 g (8,86 mmol) Aluminiumtrichlorid erhalten.
Fp.: 186°C (Essigsäureethylester/Ether)
Ausbeute: 770 mg (57%)

### Beispiel 60A

### 2-[5-(2-bromacetyl)-2-ethoxyphenyl]-5-methyl-7-cycloheptyl-3H-imidazo[5,1-f][1,2,4]triazin-4-on

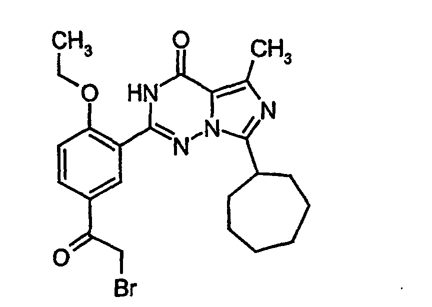

Analog Beispiel 57A wurden 1.36g (3.7 mmol) der Verbindung aus Beispiel 28A mit 1.5g (7.4 mmol) Bromacetylbromid und 1.48g (1.1 mmol) Aluminiumtrichlorid umgesetzt. Nach dem Verrühren mit Ether erhielt man 1.2 g (66.3 %) des gewünschten Produktes.
MS (DCI / NH₃): m/z (%) = 487 [ M + H ] (27 %)
¹H-NMR (200 MHz, CDCl₃): δ = 1.63 (t, 3H, CH₃), 1.75 (bs, 6H,), 1.89 - 2.42 (m, 6H), 2.94 (s, 3H, CH₃), 3.75 (m, 1H, CH), 4.37 (s, 2H, CH₂-Br), 4.43 (g, 2H, CH₂), 4.63 (s, 2H, CH₂Cl), 7.23 (d, 1H), 8.25 (dd, 1H), 8.35 (d, 1H), 10.38 (bs, 1H, NH)

### Beispiel 46

### 2-[2-Ethoxy-5-(4-morpholino-carbonyl-amino)-phenyl]-5-methyl-7-cyclopentyl-3Himidazo[5,1-f][1,2,4]-triazin-4-on

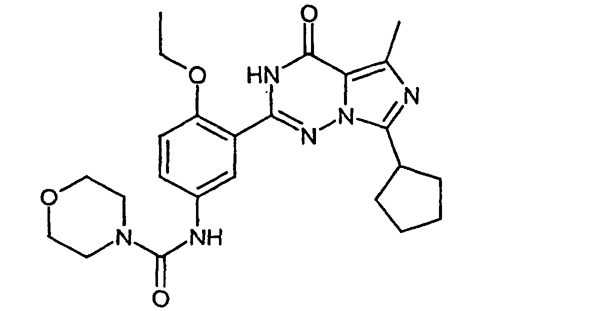

213 mg (0.601 mmol) der Aminoverbindung aus Beispiel 42A und 0.2 ml (1.43 mmol) Triethylamin werden in 6 ml Tetrahydrofuran gelöst; man kühlt auf 5°C und spritzt 0.489 ml (4.22 mmol) 4-Morpholino-carbonylchlorid gelöst in 4 ml Tetrahydrofuran hinzu. Nach Rühren über Nacht bei Raumtemperatur wird die Reaktionslösung mit Essigsäureethylester, NH₄Cl- und NaHCO₃-Lösung versetzt, die organische Phase abgetrennt, die wässrige Phase noch 3 mal mit Essigsäureethylester extrahiert, die organischen Phasen vereinigt, getrocknet und eingeengt. Man chromatografiert das Produkt an 60 ml Kieselgel mit Essigsäureethylester/Methanol als Eluent.
Ausbeute: 275 mg (98% d. Th.).
¹H NMR (200 MHz, DMSO): 1.30 (t, 3H), 1.58-2.07 (m, 8H), 2.48 (s, 3H), 3.12 (t, 4H), 3.41 (quin., 1H), 3.55 (t, 4H), 4.08 (quar., 2H), 7.08 (d, 1H), 7.55-7.62 (m, 2H), 8.56 (s, 1H).

### Beispiel 51

### 2-[5-(4-Morpholino-carbonylamino)-2-propoxy-phenyl]-5-methyl-7-cyclopentyl-3Himidazo[5,1-f][1,2,4]-triazin-4-on

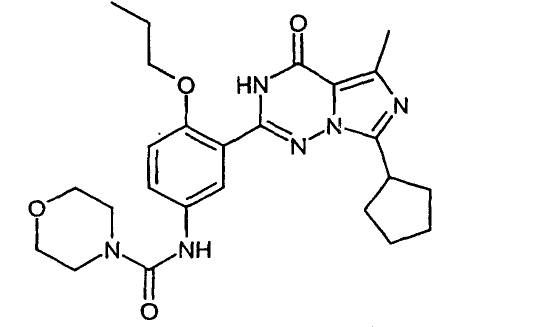

150 mg (0.408 mmol) der Aminoverbindung aus Beispiel 44A in 6 ml Tetrahydrofuran werden unter Argon mit 0.32 g (4.082 mmol) Pyridin und durch portionsweise Zugabe von 0.449 g (3.0 mmol) 4-Morpholino-N-carbonsäurechlorid versetzt und über Nacht bei Raumtemperatur gerührt. Die Reaktionslösung wird mit Essigsäureethylester und Wasser verdünnt, die wässrige Phase nochmals mit Essigsäure-ethylester extrahiert, die vereinigten organischen Phasen getrocknet und im Vakuum eingeengt. Der Rückstand wird an 50 ml Kieselgel mit Toluol unter Zusatz von Essigsäureethylester im Gradientenverfahren chromatografiert.
Ausbeute: 139 mg (70.7% d. Th.).
¹H NMR (300 MHz, DMSO): 0.93 (t, 3H), 1.57-2.08 (m, 10H), 2.48 (s, 3H), 3.42 (t, 4H), 3.50 (quin., 1H), 3.61 (t, 4H), 3.97 (t, 2H), 7.08 (d, 1H), 7.60 (m, 2H), 8.58 (s, 1H), 11.47 (s, 1H);
MS (ESI): 481 (M+H).

### Beispiel 54

### 1-{3-(7-Cyclopentyl-5-methyl-4-oxo-3,4-dihydro-imidazo[5,1-f][1,2,4]-triazin-2-yl)-4-propoxy-phenyl}-3-(4-trifluoromethyl-thio-phenyl)-harnstoff

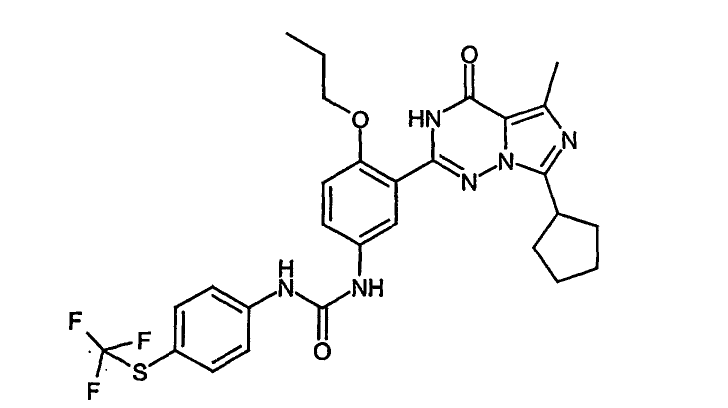

Zu einer Lösung von 150 mg (0.408 mmol) der Aminoverbindung aus Beispiel 44A in 8 ml Tetrahydrofuran gibt man unter Argon 179 mg (0.816 mmol) 4-(Trifluormethylthio)phenylisocyanat gelöst in 4 ml Tetrahydrofuran hinzu und rührt 1 Stunde bei Raumtemperatur nach. Die Reaktionslösung wird mit Essigsäureethylester, Wasser und 6 ml NH₄Cl-Lösung versetzt, die organische Phase abgetrennt, die wässrige Schicht nochmals mit Essigsäureethylester extrahiert, die vereinigten organischen Phasen getrocknet und im Vakuum eingeengt. Man chromatografiert an 40 ml Kieselgel mit Toluol und Essigsäureethylester im Gradientenverfahren von 9:1 bis 6:4.
Ausbeute: 77.7 mg (32.4% d. Th.).
¹H NMR (200 MHz, DMSO): 0.95 (t, 3H), 1.52-2.10 (m, 10H), 2.47 (s, 3H), 3.50 (quin., 1H), 3.98 (t, 2H), 7.12 (d, 1H), 7.60 (s, 6H), 8.82 (s, 1H), 9.08 (s, 1H), 11.47 (s, 1H);
MS (DCI/NH₃): 587 (M+H).
Man erhält zusätzlich eine 2. Fraktion, die mit etwas Edukt verunreinigt ist (185 mg = 77.3% d. Th.).

### Beispiel 55

### 1-{3-(7-(1-Ethylpropyl)-5-methyl-4-oxo-3,4-dihydro-imidazo[5,1-f][1,2,4]-triazin-2-yl)-4-ethoxy-phenyl}-3-(4-trifluoromethylthio-phenyl)-harnstoff

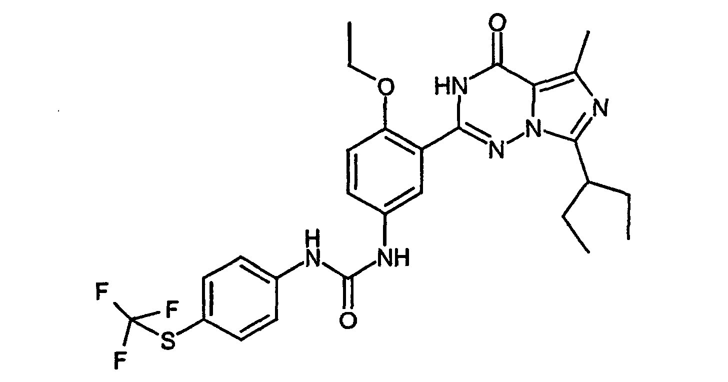

In Analogie zur Vorschrift des Beispiels 54 werden 230 mg (0.647 mmol) der Aminoverbindung aus Beispiel 48A mit 284 mg (1.29 mmol) 4-(Trifluormethylthio)phenylisocyanat in 20 ml Tetrahydrofuran innerhalb von 4 Stunden umgesetzt. Die Reinigung des Rohproduktes erfolgt durch Cromatografie über Kieselgel mit Toluol und Essigsäureethylester als Eluens.
Ausbeute: 138 mg (37.2% d. Th.).
¹H NMR (200 MHz, DMSO): 0.77 (t, 6H), 1.30 (t, 3H), 1.63-1.81 (m, 4H), 2.50 (s, 3H), 3.11 (quin., 1H), 4.09 (quar, 2H), 7.12 (d, 1H), 7.56 (d, 1H), 7.62 (s, 4H), 7.68 (d, 1H), 8.34 (s, 1H), 13.52 (s, 1H);
MS (ESI): 575 (M+H).

### Beispiel 56

### 1-{3-(7-(1-Ethylpropyl)-5-methyl-4-oxo-3,4-dihydro-imidazo[5,1-f][1,2,4]-triazin-2-yl)-4-ethoxy-phenyl}-3-(4-fluorphenyl-sulfonyl)-harnstoff

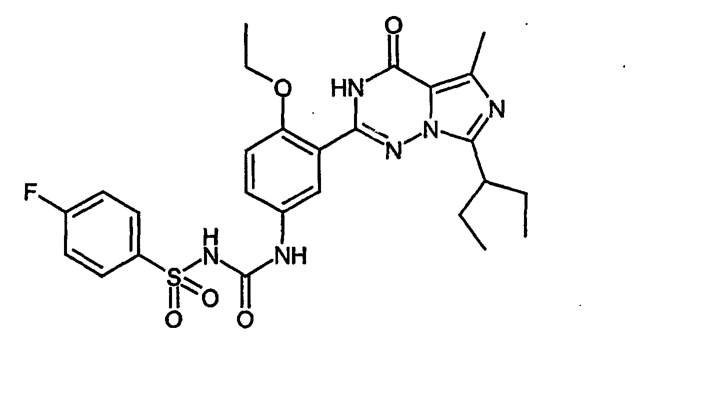

Die Herstellung erfolgt in Analogie zur Vorschrift des Beispiels 54 aus 230 mg (0.647 mmol) der Aminoverbindung des Beispiels 48A und 260 mg (1.294 mmol) 4-Fluorbenzolsulfonyl-isocyanat in 15 ml Tetrahydrofuran. Nach 4.Stunden Rühren bei Raumtemperatur erfolgt die Isolierung des Rohproduktes an Kieselgel mittels Toluol und Essigsäureethylester.
Ausbeute: 54.6 mg (15.2% d. Th.).
MS (ESI): 557 (M+H),
HPLC (analytisch): 70.0% RT: 6.68 min, Säule: Nucleosil C18 (125X4mm), Laufmittel: 0.01m H₃PO₄/ Acetonitril (Gradient), Fluss:2ml/min, 200-400nm.

### Beispiel 57

### 1-{3-(7-Cyclopentyl-5-methyl-4-oxo-3,4-dihydro-imidazo[5,1-f][1,2,4]-triazin-2-yl)-4-propoxy-phenyl}-3-(4-fluor-phenyl-sulfonyl)-harnstoff

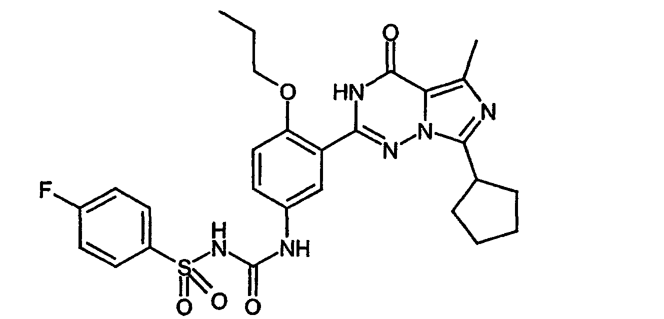

Die Herstellung erfolgt in Analogie zur Vorschrift des Beispiels 54 aus 150 mg (0.408 mmol) der Aminoverbindung des Beispiels 44A und 411 mg (2.04 mmol) 4-Fluorbenzol-sulfonylisocyanat in 12 ml Tetrahydrofuran durch Reaktion über Nacht.
Ausbeute: 163 mg (70.1 % d. Th.).
¹H NMR (400 MHz, DMSO): 0.90 (t, 3H), 1.54-2.08 (m, 10H), 2.45 (s, 3H), 3.45 (quin., 1H), 3.88 (t, 2H), 6.92 (d, 1H), 7.17 (t, 2H), 7.53 (d, 1H), 7.63 (s, 1H), 7.82 (m, 2H), 8.47 (s, 1H), 11.37 (s, 1H);
MS (ESI): 569 (M+H).

### Beispiel 58

### 1-{3-[7-(2-Ethylheptyl)-5-methyl-4-oxo-3,4-dihydro-imidazo[5,1-f][1,2,4]-triazin-2-yl]-4-ethoxy-phenyl}-3-(4-fluorphenyl-sulfonyl)-harnstoff

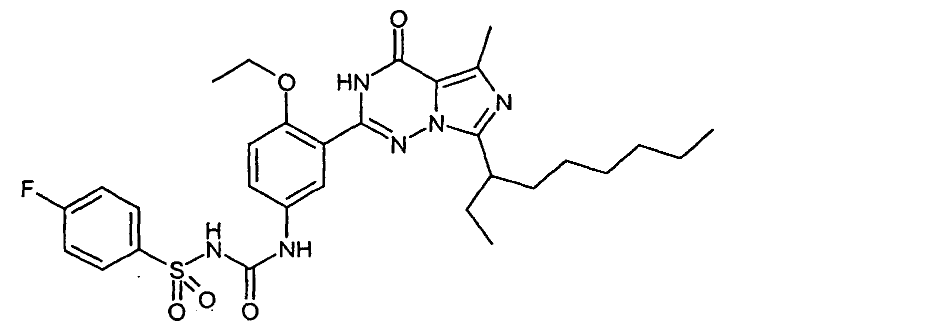

Analog zur Vorschrift des Beispiels 54 werden 50 mg (0.121 mmol) der Aminoverbindung aus Beispiel 50A mit 548 mg (2.72 mmol) 4-Fluorbenzolsulfonylisocyanat in Tetrahydrofuran über 2 Tage umgesetzt. Nach Reinigung des Rohproduktes über Kieselgel mit Toluol und Essigsäuretehylester als Eluens von 4:6 bis 3:7 erhält man 36.2 mg (48.6% d. Th.).
MS (ESI): 613 (M+H),
HPLC (analytisch): 81.0% RT: 4.92 min, Säule: Nucleosil C18 (125X4mm), Laufmittel: 0.01m H₃PO₄ / Acetonitril (Gradient), Fluss:2ml/min, 200-400nm.

### Beispiel 60

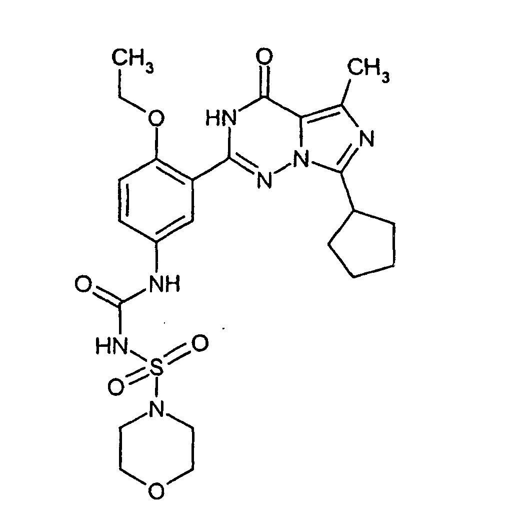

Zu einer auf 0°C gekühlten Lösung aus 50 mg (0.014 mmol) des Amins Beispiel 42A in 1,2-Dichlorethan wurden langsam 12.3 µl (0.014 mmol) Chlorsulfonylisocyanat zugetropft. Nach dem Erwärmen auf Raumtemperatur rührte man 1 h nach und fügte 12.3 mg (0.014 mmol) Morpholin und 1 Äquivalent Morpholinomethyl-polystyrol (3.47 mmol/g) zu. Es wurde über Nacht nachgerührt und anschließend über 1g Kieselgel filtriert (Essigester) und das Rohprodukt durch präparative Dünnschichtchromatographie (CH₂Cl₂ : MeOH = 20:1) gereinigt. Man erhielt 2.3 mg (3.0 %) des Sulfonylharnstoffderivates (91 % nach HPLC)
¹H-NMR (200 MHz, CDCl₃): δ = 1.55 (t, 3H, CH₃), 1.64 - 2.15 (m, 8H), 2.63 (s, 3H, CH₃), 3.51 (t, 4H, CH₂), 3.64 (m, 1H), 3.78 (t, 4H, CH₂), 4.23 (g, 2H, CH₂), 6.38 (bs, 1H, NH), 7.02 (d, 1H), 7.73 (dd, 1H), 7.86 (d, 1H), 9.95 (bs, 1H, NH).

### Beispiel 61

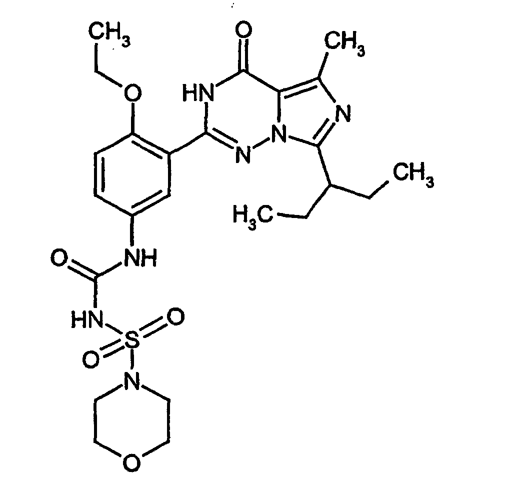

Zu einer auf 0°C gekühlten Lösung aus 30 mg (0.08 mmol) des Amins Beispiel 48A in 1,2-Dichlorethan wurden zunächst 7.4 µl (0.08 mmol) Chlorsulfonylisocyanat zugetropft. Nach dem Erwärmen auf Raumtemperatur rührte man 1 h nach und fügte 7.3 mg (0.08 mmol) Morpholin und eine Suspension aus 30 mg Morpholinomethyl-polystyrol (3.47 mmol/g) in 1,2-Dichlorethan zu. Nach 3 h wurde das Reaktionsgemisch filtriert und das Rohprodukt chromatographisch gereinigt (Gradient: CH₂Cl₂ → CH₂Cl₂ : MeOH = 100 → 40:1). Man erhielt 4.8 mg (10.9 %) des Sulfonylharnstoffes (91 % nach HPLC).
¹H-NMR (200 MHz, D₆-DMSO): δ = 0.75 (t, 6H, CH₃), 1.29 (t, 3H, CH₃), 1.72 (m, 4H, CH₂), 2.53 (s, CH₃, shoulder o / D₆-DMSO), 3.12 (m, 1H, CH), 3.42 (bt, 4H, CH₂), 3.59 (bt, 4H, CH₂), 4.06 (g, 2H, CH₂), 7.07 (d, 1H), 7.57 (m, 2H), 8.55 (bs, 1H, NH), 11.45 (bs, 1H, NH).

### Beispiel 62

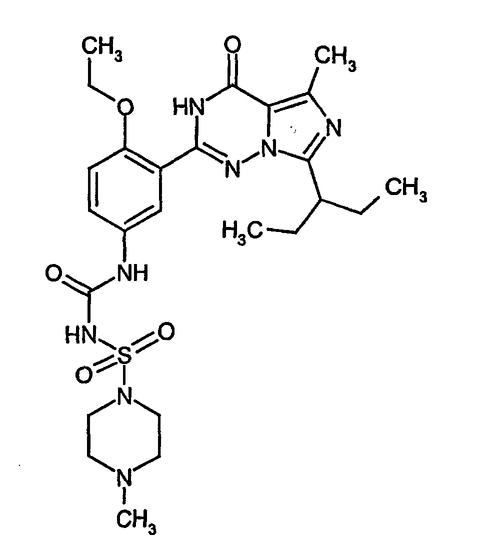

Analog Beispiel 61 wurden 30 mg (0.08 mmol) des Amins Beispiel 48A zu 9.5 mg (20.1 %) des Sulfonylharnstoffes (86 % nach HPLC) umgesetzt.
MS (ESI): m/z (%) = 561 [M + H ] (30)
¹H-NMR (300 MHz, D₃COD): δ = 0.80 (t, 6H, CH₃), 1.42 (t, 3H, CH₃), 1.83 (m, 4H, CH₂, CH), 2.34 (s, 3H CH₃), 2.52 (t, 4H, CH₂), 2.54 (s, 3H, CH₃), 3.57 (t, 4H, CH₂), 4.17 (g, 2H, CH₂), 7.09 (d, 1H), 7.49 (dd, 1H), 7.69 (d, 1H).

### Beispiel 66

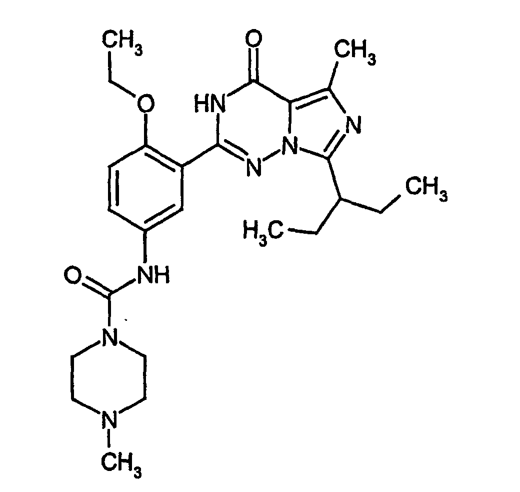

Zu einer Lösung aus 80 mg (0.23 mmol) des Amins Beispiel 48A in 1,4-Dioxan wurden 19 µl (0.16 mmol) Diphosgen zugetropft und 20 h nachgerührt. Nach dem Einengen im Vakuum wurde der Rückstand zweimal in Benzol aufgenommen und nochmals eingeengt. 30 mg (0.07 mmol) des erhaltenen Carbamoylchlorids wurden als Rohprodukt in 1 ml 1,2-Dichlorethan gelöst, mit 8.6 mg (0.09 mmol) N-Methylpiperazin versetzt und 20 h bei Raumtemperatur nachgerührt. Nach dem Quenchen mit 0.5 ml H₂O und der Filtration über Extrelut / Kieselgel (CH₂Cl₂ : MeOH = 95:5), erhielt man 30 mg (87 %) des Harnstoffderivates (90 % nach HPLC).
MS (DCI, NH₃): m/z (%) 482 [ M + H ] (10)
¹H-NMR (200 MHz, D₆-DMSO): δ = 0.75 (t, 6H, CH₃), 1.28 (t, 3H, CH₃), 1.72 (m, 4H, CH₂), 2.22 (bt, 4H, CH₂), 3.12 (m, 1H, CH), 3.43 (bt, 4H, CH₂), 3.91 (s, 3H, CH₃), 4.05 (g, 2H, CH₂), 7.05 (d, 1H), 7.58 (m, 2H), 8.53 (bs, 1H, NH), 11.48 (bs, 1H, NH)

### Beispiel 67

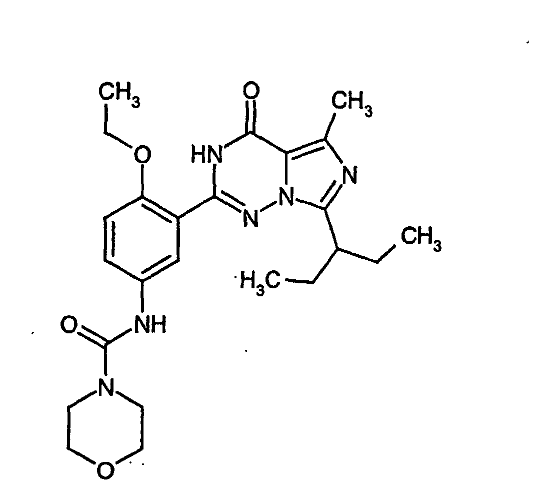

Analog Beispiel 66 wurden 30 mg (0.08 mmol) des Carbamoylchlorids mit 7.51 (0.09 mmol) Morpholin umgesetzt. Zur Aufarbeitung wurde mit 0.5 ml 2 N HCl versetzt und über 500 mg Extrelut und 500 mg SiO₂ filtriert (CH₂Cl₂). Man erhielt 26.2 mg (77.9 %) des Harnstoffes (95.2 % nach HPLC).
MS (DCI, NH₃): m/z (%) 469 [M + H] (10 %); 382 [M - 87 ] (100)
¹H-NMR (200 MHz, D₆-DMSO): δ = 0.75 (t, 6H, CH₃), 1.28 (t, 3H, CH₃), 1.72 (m, 4H, CH₂), 3.10 (m, 1H, CH), 3.42 (t, 4H, CH₂), 3.59 (t, 4H, CH₂), 4.06 (g, 2H, CH₂), 7.05 (d, 1H) 7.58 (m, 2H), 8.57 (bs, 1H, NH), 11.49 (bs, 1H, NH)

### Beispiel 68

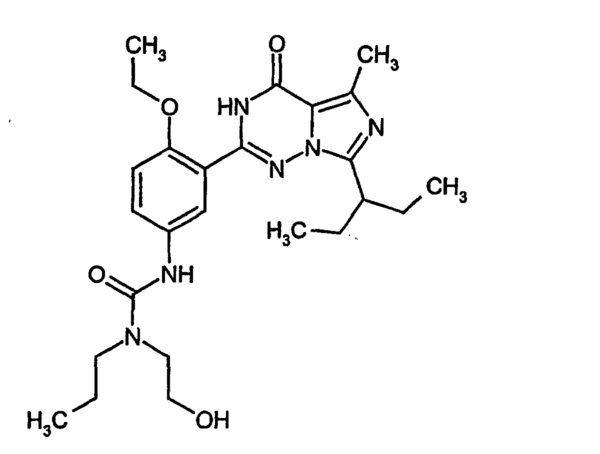

Analog Beispiel 66 wurden 30 mg (0.08 mmol) des Carbamoylchlorids zu 33 mg (95%) des Harnstoffes (87% nach HPLC) umgesetzt.
MS (DCI, NH₃): m/z (%) 485 [ M + H] (10); 382 [ M - 87] (100)
¹H-NMR (200 MHz, D₆-DMSO): δ = 0.74 (t, 6H, CH₃), 0.83 (t, 3H, CH₃), 1.28 (t, 3H, CH₃), 1.50 (m, 2H, CH₂), 1.72 (m, 4H, CH₂), 3.05 - 3.61 (m, 6H, CH₂), 4.07 (g, 2H, CH₂), 7.07 (d, 1H), 7.51 (m, 2H), 8.42 (bs, 1H, NH), 11.49 (bs, 1H, NH).

Die Verbindungen der folgenden Tabelle 1 wurden parallelsynthetisch analog Beispiel 66 aus dem Amin Beispiel 48A, Diphosgen und dem entsprechenden Amin hergestellt. Neutrale Endprodukte wurden mit 0,5 ml 1 molarer Schwefelsäurelösung, basische Endprodukte mit 0,5 ml gesättigter Natriumhydrogencarbonatlösung gequencht.

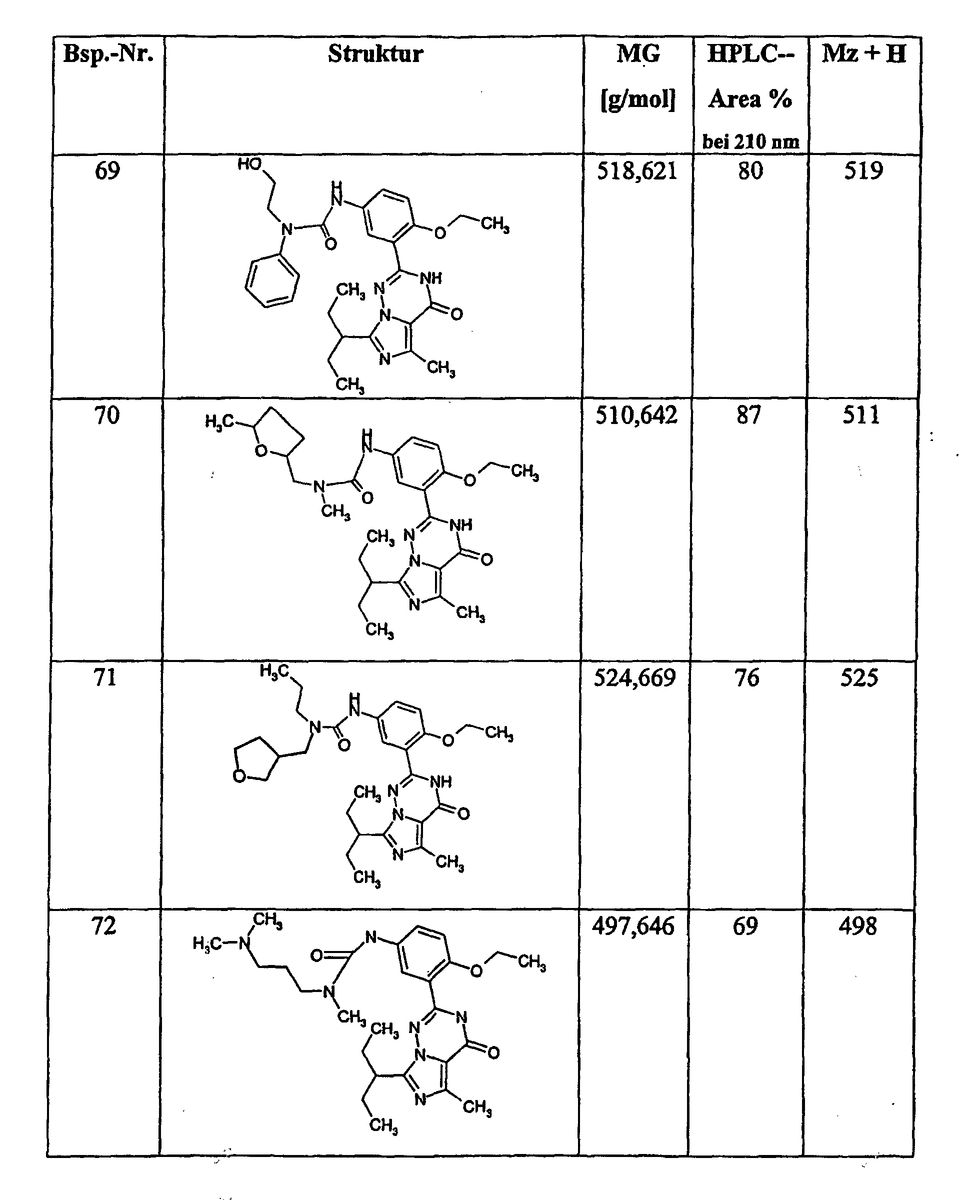

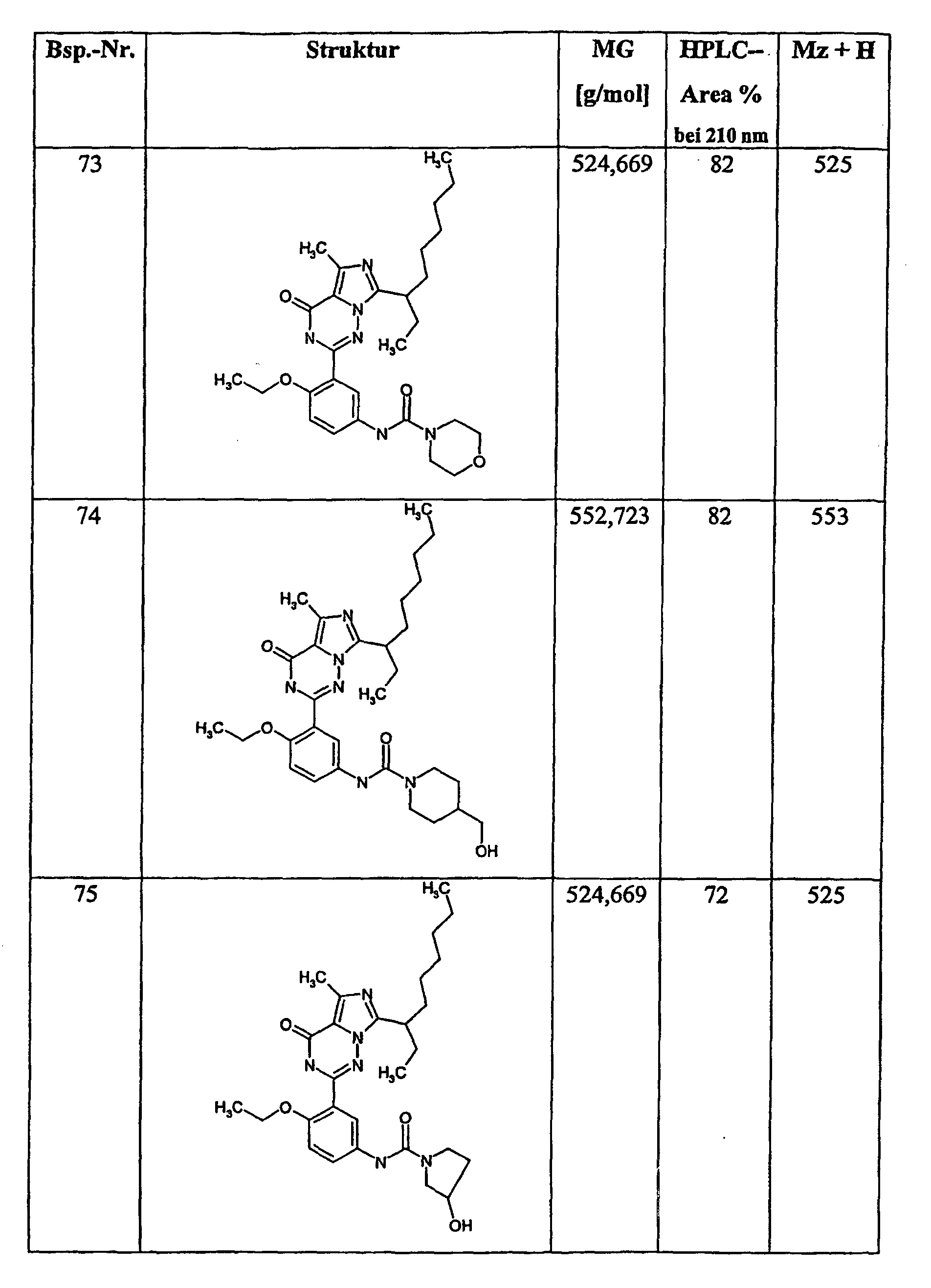

## Patentansprüche

1. Imidazotriazinone der folgenden allgemeinen Formel (I) 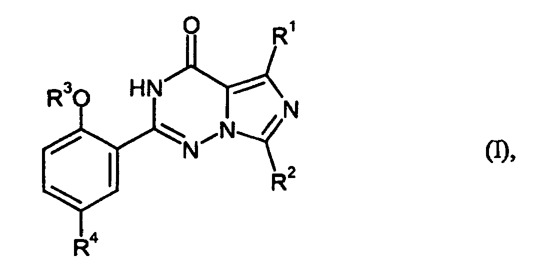 in welcher
R¹ für (C₁-C₆)-Alkyl steht,
R² für (C₃-C₈)-Cycloalkyl oder (C₁-C₁₂)-Alkyl steht,
R³ für (C₁-C₆)-Alkyl steht,
R⁴ für einen Rest der Formel -NH-CO-NR¹⁷R¹⁸ steht,
worin
R¹⁷ und R¹⁸ gleich oder verschieden sind und Wasserstoff oder (C₁-C₆)-Alkyl bedeuten, das gegebenenfalls durch Hydroxy oder durch einen Rest der Formeln 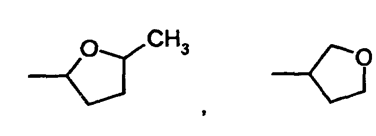 oder -NR¹⁹R²⁰ substituiert ist,
worin
R¹⁹ und R²⁰ gleich oder verschiedene sind und Wasserstoff, Phenyl oder (C₁-C₆)-Alkyl bedeuten
oder
R¹⁷ und R¹⁸ gemeinsam mit dem Stickstoffatom, an das sie gebunden sind, einen heterocyclischen Ring der Formeln 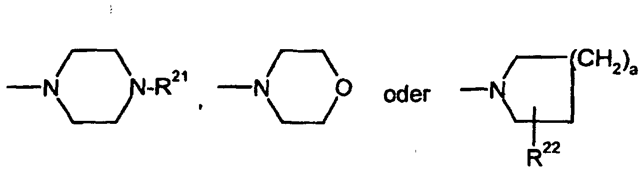 bilden,
worin
R²¹ Wasserstoff oder (C₁-C₆)-Alkyl bedeutet,
a entweder 1 oder 2 bedeutet,
R²² Hydroxy oder (C₁-C₆)-Alkyl bedeutet, das gegebenenfalls durch Hydroxy substituiert ist,
oder
R¹⁷ und/oder R¹⁸ (C₆-C₁₂)-Aryl bedeuten, das gegebenenfalls durch Halogen, Trifluorethyl oder durch -SCF₃ substituiert ist
oder
R¹⁷ Wasserstoff bedeutet und
R¹⁸ einen Rest der Formel -SO₂-R²³ bedeutet,
worin
R²³ (C₁-C₆)-Alkyl oder (C₆-C₁₂)-Aryl bedeutet, das gegebenenfalls durch Halogen substituiert ist,
oder für einen Rest der Formeln 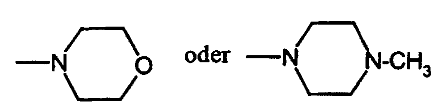 steht,
und ihre Tautomeren sowie deren pharmazeutisch verträgliche Salze, Hydrate und Prodrugs.

2. Imidazotriazinone der allgemeinen Formel (I) nach Anspruch 1,
wobei
R¹ für (C₁-C₄)-Alkyl steht,
R² für Cyclopentyl, Cycloheptyl oder (C₁-C₁₀)-Alkyl steht,
R³ für (C₁-C₄)-Alkyl steht,
R⁴ für einen Rest der Formel -NH-CO-NR¹⁷R¹⁸ steht,
worin
oder
R¹⁷ und R¹⁸ gleich oder verschieden sind und Wasserstoff oder (C₁-C₄)-Alkyl bedeuten, das gegebenenfalls durch Hydroxy oder durch einen Rest der Formeln 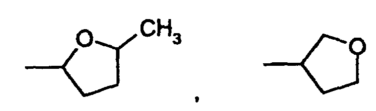 oder -NR¹⁹R²⁰ substituiert ist,
worin
R¹⁹ und R²⁰ gleich oder verschiedene sind und Wasserstoff, Phenyl oder (C₁-C₄)-Alkyl bedeuten
oder
R¹⁷ und R¹⁸ gemeinsam mit dem Stickstoffatom, an das sie gebunden sind, einen heterocyclischen Ring der Formeln 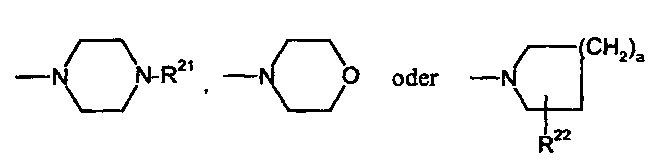 bilden, worin
R²¹ Wasserstoff oder (C₁-C₄)-Alkyl bedeutet,
a entweder 1 oder 2 bedeutet,
R²² Hydroxy oder (C₁-C₄)-Alkyl bedeutet, das gegebenenfalls durch Hydroxy substituiert ist,
oder
R¹⁷ und/oder R¹⁸ Phenyl bedeuten, das gegebenenfalls durch Chlor, Trifluorethyl oder durch -SCF₃ substituiert ist
oder
R¹⁷ Wasserstoff bedeutet und
R¹⁸ einen Rest der Formel -SO₂-R²³ bedeutet, worin
R²³ (C₁-C₄)-Alkyl oder Phenyl bedeutet, das gegebenenfalls durch Halogen substituiert ist,
oder für einen Rest der Formeln 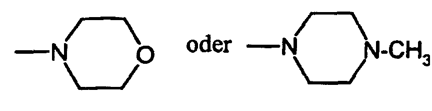 steht,
und ihre Tautomeren sowie deren pharmazeutisch verträgliche Salze, Hydrate und Prodrugs.

3. Imidazotriazinone der allgemeinen Formel (I) nach Anspruch 1 oder 2,
wobei
R¹ für (C₁-C₄)-Alkyl steht,
R² für Cyclopentyl, Cyclohexyl, Cycloheptyl oder (C₁-C₁₀)-Alkyl steht,
R³ für (C₁-C₄)-Alkyl steht,
R⁴ für einen Rest der Formel -NH-CO-NR¹⁷R¹⁸ steht,
worin
R¹⁷ und R¹⁸ gleich oder verschieden sind und Wasserstoff oder (C₁-C₄)-Alkyl bedeuten, das gegebenenfalls durch Hydroxy substituiert ist,
oder
R¹⁷ und R¹⁸ gemeinsam mit dem Stickstoffatom, an das sie gebunden sind, einen heterocyclischen Ring der Formeln 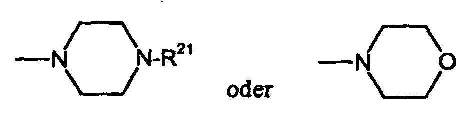 bilden,
worin
R²¹ Wasserstoff oder (C₁-C₄)-Alkyl bedeutet,
oder
R¹⁷ und/oder R¹⁸ Phenyl bedeuten, das gegebenenfalls durch Chlor, Trifluorethyl oder durch -SCF₃ substituiert ist
oder
R¹⁷ Wasserstoff bedeutet und
R¹⁸ einen Rest der Formel -SO₂-R²³ bedeutet, worin
R²³ (C₁-C₄)-Alkyl oder Phenyl bedeutet, das gegebenenfalls durch Halogen substituiert ist,
oder für einen Rest der Formeln 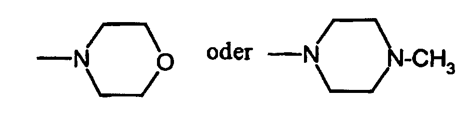 steht,
und ihre Tautomeren sowie deren pharmazeutisch verträgliche Salze, Hydrate und Prodrugs.

4. Imidazotriazinone nach Anspruch 1 mit den folgenden Strukturen: 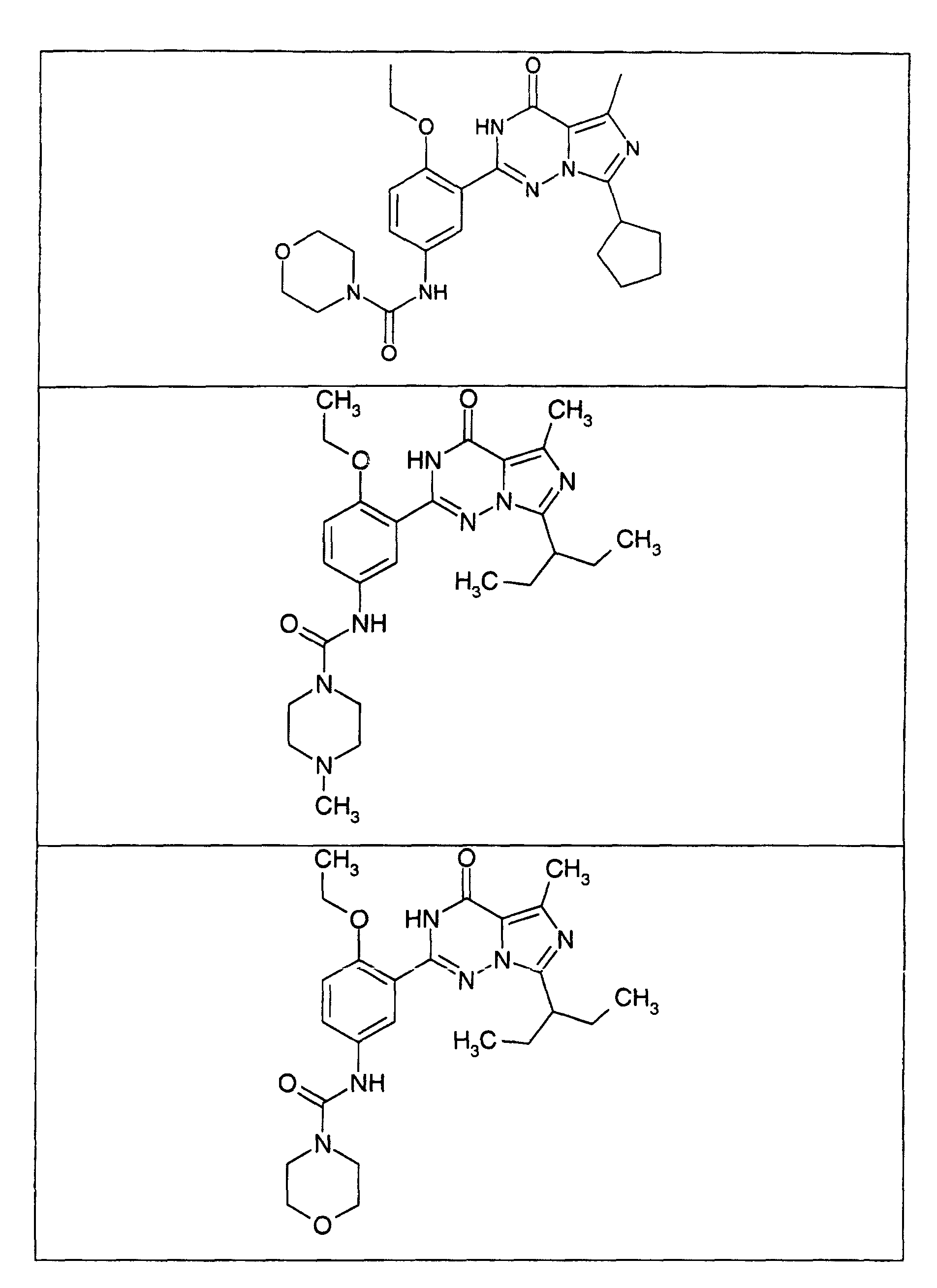 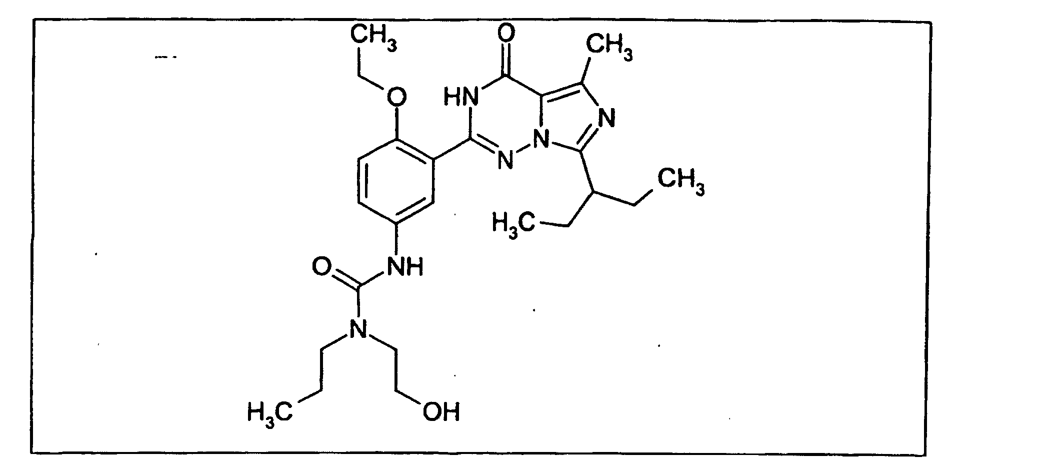 und ihre Tautomeren sowie deren pharmazeutisch verträgliche Salze, Hydrate und Prodrugs.

5. Verbindungen der allgemeinen Formel (I) gemäß Ansprüchen 1 bis 4 zur Prophylaxe und/oder Behandlung von Erkrankungen.

6. Verfahren zur Herstellung von Imidazotriazinonen gemäß Ansprüchen 1 bis 4, **dadurch gekennzeichnet, daß** man
[A] im Fall, dass R⁴ für einen wie zuvor definierten, über ein Stickstoffatom gebundenen Rest steht, Verbindungen der allgemeinen Formel (II) 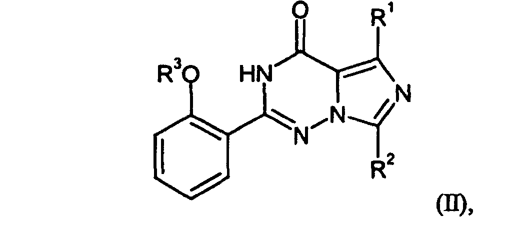 in welcher
R¹, R² und R³ die oben angegebene Bedeutung haben,
zunächst durch Umsetzung mit HNO₃/CF₃CO₂H in die Verbindungen der allgemeinen Formel (III) 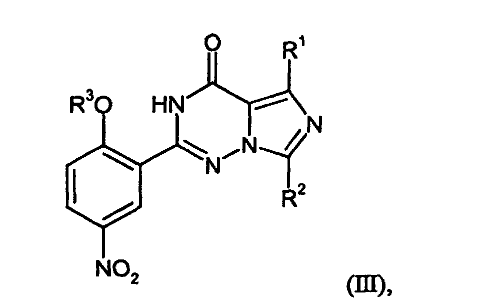 in welcher
R¹, R² und R³ die oben angegebene Bedeutung haben,
überführt,
in einem nächsten Schritt mit H₂/Pd-C zu den Aminen der allgemeinen Formel (IV) 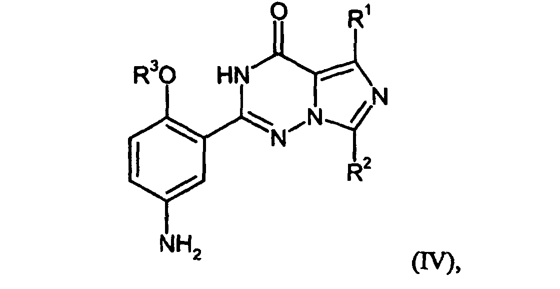 in welcher
R¹, R² und R³ die oben angegebene Bedeutung haben,
reduziert
und
abschließend mit Verbindungen der allgemeinen Formel (V)
A - D (V),
worin
A für die oben unter R⁴ aufgeführten Reste R¹⁷ oder R¹⁸ steht,
D für die Reste -NH-CO-Cl, -N=C=O oder -SO₂-N=C=O steht,
in inerten Lösemitteln, gegebenenfalls in Anwesenheit einer Base und/oder eines Hilfsmittels umsetzt.

7. Arzneimittel oder pharmazeutische Zusammensetzung, enthaltend mindestens eine Verbindung der allgemeinen Formel (I) gemäß einem der Ansprüche 1 bis 4 sowie einen oder mehrere pharmakologisch unbedenkliche Hilfs- und Trägerstoffe.

8. Arzneimittel oder pharmazeutische Zusammensetzung gemäß Anspruch 7 zur Prophylaxe und/oder Behandlung von Erkrankungen, die im Zusammenhang mit cGMP-regulierten Vorgängen stehen ('cGMP-related diseases').

9. Arzneimittel oder pharmazeutische Zusammensetzung gemäß Anspruch 7 oder 8 zur Prophylaxe und/oder Behandlung von kardiovaskulären Erkrankungen, Erkrankungen des Urogenitalsystems sowie cerebrovaskulären Erkrankungen.

10. Arzneimittel oder pharmazeutische Zusammensetzung gemäß einem der Ansprüche 7 bis 9 zur Prophylaxe und/oder Behandlung von kardiovaskulären Erkrankungen wie Bluthochdruck, neuronale Hypertonie, stabile und instabile Angina, periphere und kardiale Gefäßerkrankungen, Arrhythmien, thromboembolische Erkrankungen und Ischämien wie Myokardinfarkt, Himschlag, transistorische und ischämische Attacken, Angina pectoris, periphere Durchblutungsstörungen, Verhinderung von Restenosen nach Thrombolysetherapie, percutaner transluminaler Angioplastie (PTA), percutan transluminaler Koronarangioplastien (PTCA) und Bypass.

11. Arzneimittel oder pharmazeutische Zusammensetzung gemäß einem der Ansprüche 7 bis 9 zur Prophylaxe und/oder Behandlung von cerebrovaskulären Erkrankungen wie cerebrale Ischämie, Himschlag, Reperfusionsschäden, Hirntrauma, Ödeme, cerebrale Thrombose, Demenz und Alzheimer'sche Erkrankung.

12. Arzneimittel oder pharmazeutische Zusammensetzung gemäß einem der Ansprüche 7 bis 9 zur Prophylaxe und/oder Behandlung von Erkrankungen des Urogenitalsystems wie Prostatahypertrophie, Inkontinenz sowie insbesondere erektile Dysfunktion und weibliche sexuelle Dysfunktion.

13. Arzneimittel oder pharmazeutische Zusammensetzung gemäß einem der Ansprüche 7 bis 12, **dadurch gekennzeichnet, dass** das Arzneimittel oder die pharmazeutische Zusammensetzung intravenös oder oral appliziert wird.

14. Verwendung der Verbindungen der allgemeinen Formel (I) gemäß einem der Ansprüche 1 bis 4 zur Herstellung von Arzneimitteln oder pharmazeutischen Zusammensetzungen zur Prophylaxe und/oder Behandlung von Krankheiten.

15. Verwendung gemäß Anspruch 14 zur Herstellung eines Arzneimittels oder einer pharmazeutischen Zusammensetzung zur Prophylaxe und/oder Behandlung von Erkrankungen, die im Zusammenhang mit cGMP-regulierten Vorgängen stehen ('cGMP-related diseases').

16. Verwendung gemäß Anspruch 14 oder 15 zur Herstellung eines Arzneimittels oder einer pharmazeutischen Zusammensetzung zur Prophylaxe und/oder Behandlung von kardiovaskulären Erkrankungen, Erkrankungen des Urogenitalsystems sowie cerebrovaskulären Erkrankungen.

17. Verwendung gemäß einem der Ansprüche 14 bis 16 zur Herstellung eines Arzneimittels oder einer pharmazeutischen Zusammensetzung zur Prophylaxe und/oder Behandlung von kardiovaskulären Erkrankungen wie Bluthochdruck, neuronale Hypertonie, stabile und instabile Angina, periphere und kardiale Gefäßerkrankungen, Arrhythmien, thromboembolische Erkrankungen und Ischämien wie Myokardinfarkt, Himschlag, transistorische und ischämische Attacken, Angina pectoris, periphere Durchblutungsstörungen, Verhinderung von Restenosen nach Thrombolysetherapie, percutaner transluminaler Angioplastie (PTA), percutan transluminaler Koronarangioplastien (PTCA) und Bypass.

18. Verwendung gemäß einem der Ansprüche 14 bis 16 zur Herstellung eines Arzneimittels oder einer pharmazeutischen Zusammensetzung zur Prophylaxe und/oder Behandlung von cerebrovaskulären Erkrankungen wie cerebrale Ischämie, Hirnschlag, Reperfusionsschäden, Hirntrauma, Ödeme, cerebrale Thrombose, Demenz und Alzheimer'sche Erkrankung.

19. Verwendung gemäß einem der Ansprüche 14 bis 16 zur Herstellung eines Arzneimittels oder einer pharmazeutischen Zusammensetzung zur Prophylaxe und/oder Behandlung von Erkrankungen des Urogenitalsystems wie Prostatahypertrophie, Inkontinenz sowie insbesondere erektile Dysfunktion und weibliche sexuelle Dysfunktion.

20. Verwendung gemäß einem der Ansprüche 14 bis 19, **dadurch gekennzeichnet, dass** die Arzneimittel oder Zusammensetzungen intravenös oder oral appliziert werden.

## Claims

1. Imidazotriazinones of the following general formula (I) 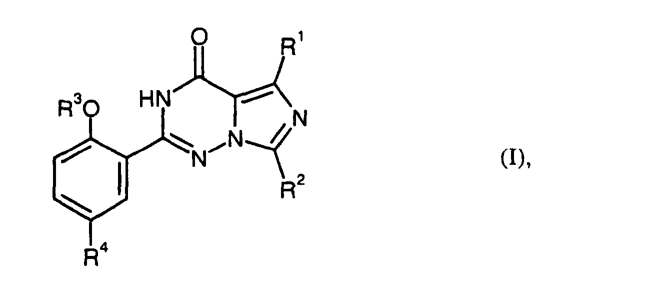 in which
R¹ represents (C₁-C₆)-alkyl,
R² represents (C₃-C₈)-cycloalkyl or (C₁-C₁₂)-alkyl,
R³ represents (C₁-C₆)-alkyl,
R⁴ represents a radical of the formula -NH-CO-NR¹⁷R¹⁸,
in which
R¹⁷ and R¹⁸ are identical or different and represent hydrogen or (C₁-C₆)-alkyl which is optionally substituted by hydroxyl or by a radical of the formulae 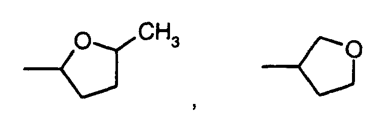 or -NR₁₉R₂₀,
in which
R¹⁹ and R²⁰ are identical or different and represent hydrogen, phenyl or (C₁-C₆)-alkyl
or
R¹⁷ and R¹⁸ together with the nitrogen atom to which they are attached form a heterocyclic ring of the formulae 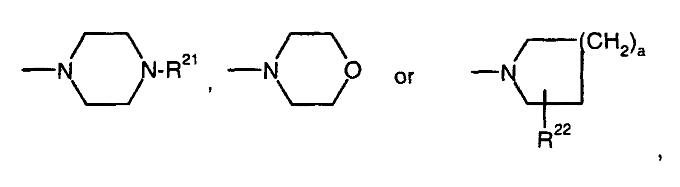
in which
R²¹ represents hydrogen or (C₁-C₆)-alkyl,
a represents either 1 or 2,
R²² represents hydroxyl or (C₁-C₆)-alkyl which is optionally substituted by hydroxyl,
or
R¹⁷ and/or R¹⁸ represent (C₆-C₁₂)-aryl which is optionally substituted by halogen, trifluoroethyl or by -SCF₃
or
R¹⁷ represents hydrogen and
R¹⁸ represents a radical of the formula -SO₂-R²³,
in which
R²³ represents (C₁-C₆)-alkyl or (C₆-C₁₂)-aryl which is optionally substituted by halogen,
or represents a radical of the formulae 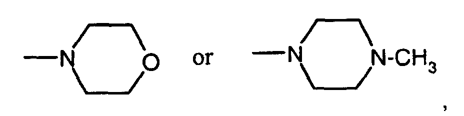
and its tautomers and its pharmaceutically acceptable salts, hydrates and prodrugs.

2. Imidazotriazinones of the general formula (I) according to Claim 1,
where
R¹ represents (C₁-C₄)-alkyl,
R² represents cyclopentyl, cycloheptyl or (C₁-C₁₀)-alkyl,
R³ represents (C₁-C₄)-alkyl,
R⁴ represents a radical of the formula -NH-CO-NR¹⁷R¹⁸,
in which
R¹⁷ and R¹⁸ are identical or different and represent hydrogen or (C₁-C₄)-alkyl which is optionally substituted by hydroxyl or by a radical of the formulae 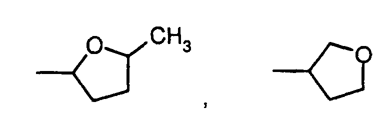 or -NR₁₉R₂₀,
in which
R¹⁹ and R²⁰ are identical or different and represent hydrogen, phenyl or (C₁-C₄)-alkyl
or
R¹⁷ and R¹⁸ together with the nitrogen atom to which they are attached form a heterocyclic ring of the formulae 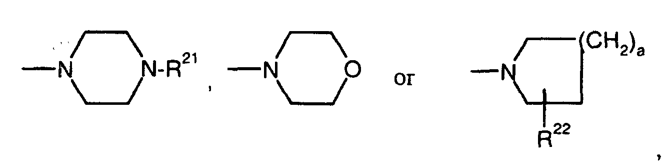 in which
R²¹ represents hydrogen or (C₁-C₄)-alkyl,
a represents either 1 or 2,
R²² represents hydroxyl or (C₁-C₄)-alkyl which is optionally substituted by hydroxyl,
or
R¹⁷ and/or R¹⁸ represent phenyl which is optionally substituted by chlorine, trifluoroethyl or by -SCF₃
or
R¹⁷ represents hydrogen and
R¹⁸ represents a radical of the formula -SO₂-R²³,
in which
R²³ represents (C₁-C₄)-alkyl or phenyl which is optionally substituted by halogen,
or represents its a radical of the formulae 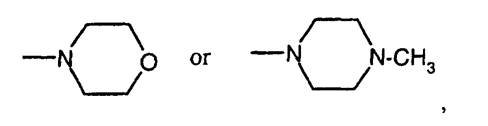
and its tautomers and its pharmaceutically acceptable salts, hydrates and prodrugs.

3. Imidazotriazinones of the general formula (I) according to Claim 1 or 2,
where
R¹ represents (C₁-C₄)-alkyl,
R² represents cyclopentyl, cyclohexyl, cycloheptyl or (C₁-C₁₀)-alkyl,
R³ represents (C₁-C₄)-alkyl,
R⁴ represents a radical of the formula -NH-CO-NR¹⁷R¹⁸,
in which
R¹⁷ and R¹⁸ are identical or different and represent hydrogen or (C₁-C₄)-alkyl which is optionally substituted by hydroxyl,
or
R¹⁷ and R¹⁸ together with the nitrogen atom to which they are attached form a heterocyclic ring of the formulae 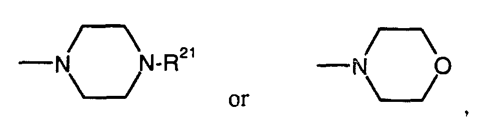
in which
R²¹ represents hydrogen or (C₁-C₄)-alkyl,
or
R¹⁷ and/or R¹⁸ represent phenyl which is optionally substituted by chlorine, trifluoroethyl or by -SCF₃
or
R¹⁷ represents hydrogen and
R¹⁸ represents a radical of the formula -SO₂-R²³,
in which
R²³ represents (C₁-C₄)-alkyl or phenyl which is optionally substituted by halogen,
or represents a radical of the formulae 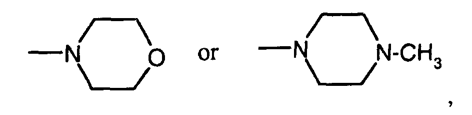
and its tautomers and its pharmaceutically acceptable salts, hydrates and prodrugs.

4. Imidazotriazinones according to Claim 1 having the following structures: 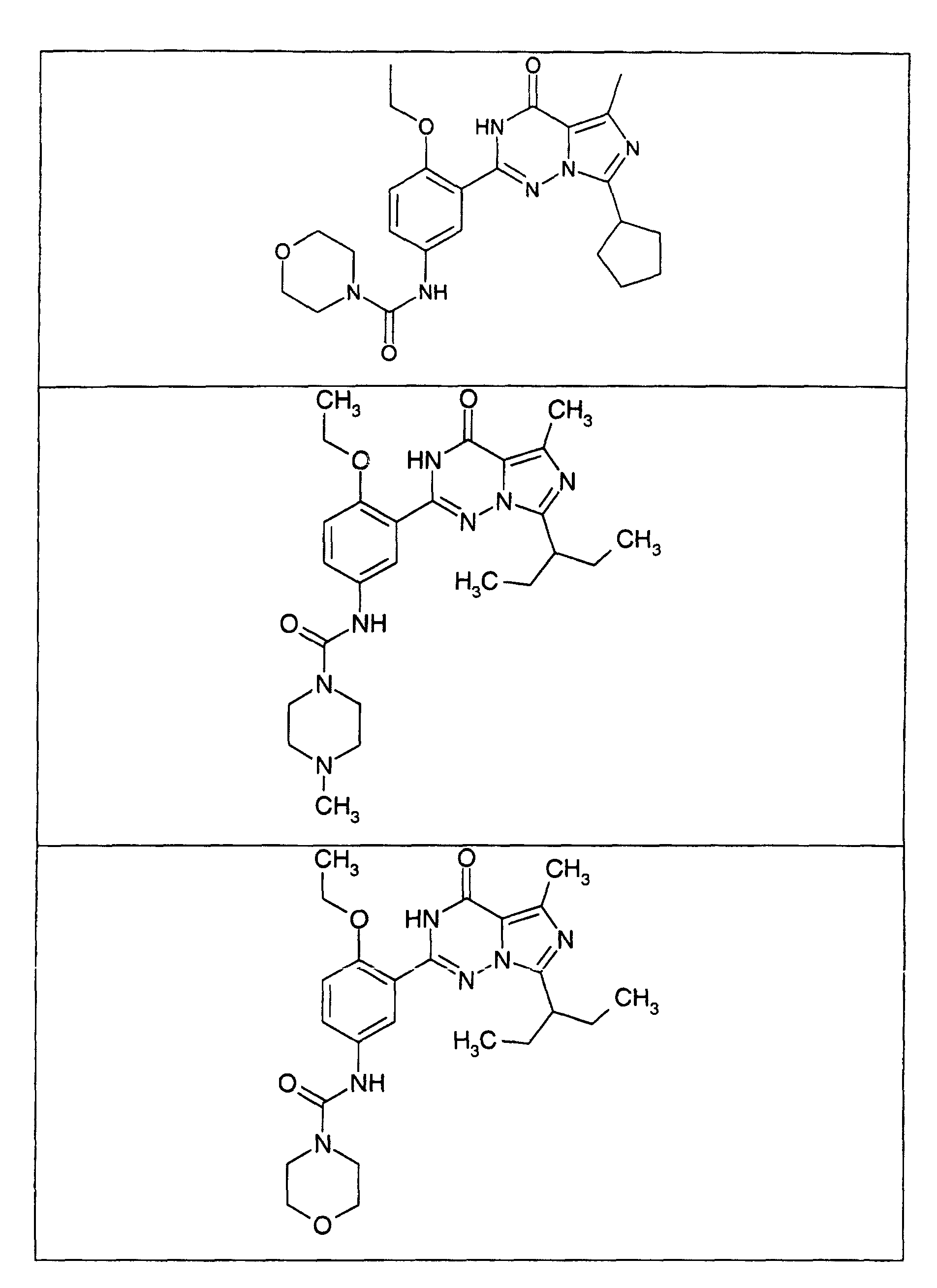 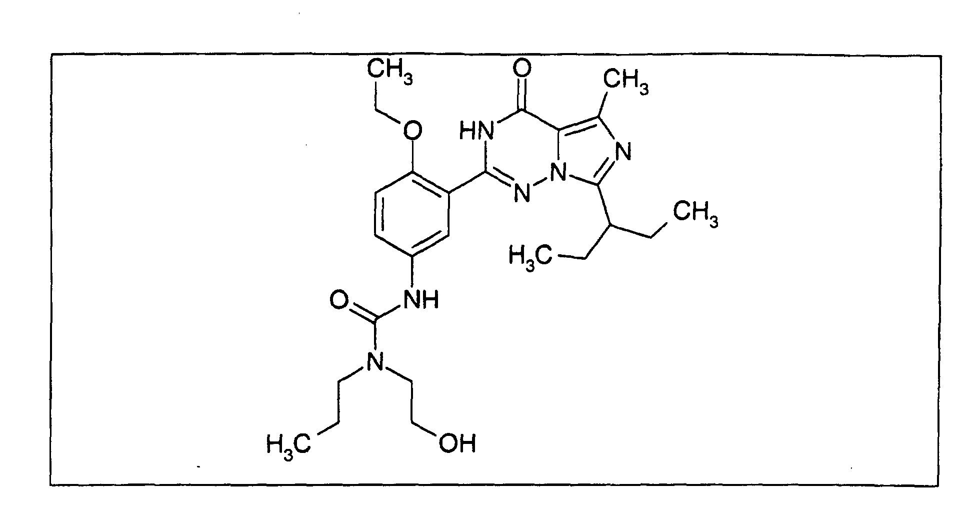 and its tautomers and its pharmaceutically acceptable salts. hydrates and prodrugs.

5. Compounds of the general formula (I) according to any of Claims 1 to 4 for the prophylaxis and/or treatment of disorders.

6. Process for preparing imidazotriazinones according to any of Claims 1 to 4, **characterized in that**
[A] in the case that R⁴ is a radical as defined above which is attached via a nitrogen atom. compounds of the general formula (II) 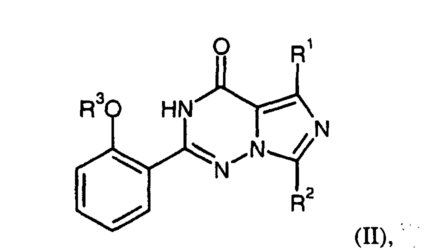 in which
R¹, R² and R³ are as defined above
are initially converted, by reaction with HNO₃/CF₃CO₂H, into the compounds of the general formula (III) 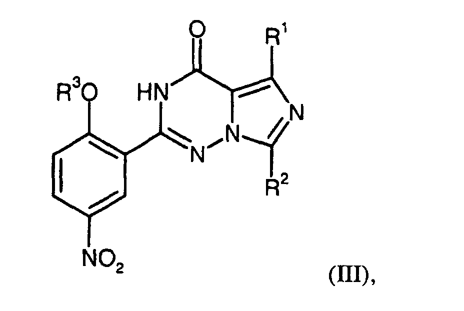 in which
R¹, R² and R³ are as defined above,
in a next step reduced with H₂/Pd-C to give amines of the general formula (IV) 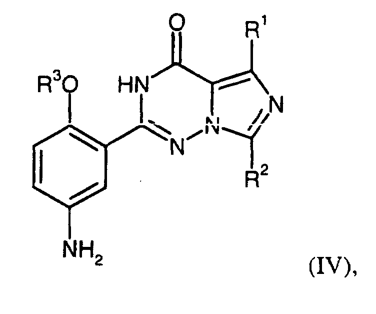 in which
R¹, R² and R³ are as defined above,
and
finally reacted with compounds of the general formula (V)
A - D (V),
in which
A represents the radicals R¹⁷ or R¹⁸ listed above under R⁴,
D represents the radicals -NH-CO-Cl, -N=C=O or -SO₂-N=C=O,
in inert solvents, if appropriate in the presence of a base and/or an auxiliary.

7. Medicament or pharmaceutical composition which comprises at least one compound of the general formula (I) according to any of Claims 1 to 4 and one or more pharmacologically acceptable auxiliaries and carriers.

8. Medicament or pharmaceutical composition according to Claim 7 for the prophylaxis and/or treatment of cGMP-related diseases.

9. Medicament or pharmaceutical composition according to Claim 7 or 8 for the prophylaxis and/or treatment of cardiovascular disorders, disorders of the urogenital system and cerebrovascular disorders.

10. Medicament or pharmaceutical composition according to any of Claims 7 to 9 for the prophylaxis and/or treatment of cardiovascular disorders such as hypertension, neuronal hypertonia, stable and unstable angina, peripheral and cardial vasculopathies, arrhythmias, thromboembolic disorders and ischaemias such as myocardial infarction, stroke, transitory and ischaemic attacks, angina pectoris, obstruction of peripheral circulation, prevention of restenoses after thrombolysis therapy, percutaneous transluminal angioplasty (PTA), percutaneous transluminal coronary angioplasties (PTCA) and bypass.

11. Medicament or pharmaceutical composition according to any of Claims 7 to 9 for the prophylaxis and/or treatment of cerebrovascular disorders such as cerebral ischaemia, stroke, reperfusion damage, cerebral trauma, oedema, cerebral thrombosis, dementia and Alzheimer's disease.

12. Medicament or pharmaceutical composition according to any of Claims 7 to 9 for the prophylaxis and/or treatment of disorders of the urogenital system such as hypertrophy of the prostate, incontinence and in particular erectile dysfunction and female sexual dysfunction.

13. Medicament or pharmaceutical composition according to any of Claims 7 to 12, **characterized in that** the medicament or the pharmaceutical composition is administered intravenously or orally.

14. Use of the compounds of the general formula (I) according to any of Claims 1 to 4 for preparing medicaments or pharmaceutical compositions for the prophylaxis and/or treatment of diseases.

15. Use according to Claim 14 for preparing a medicament or a pharmaceutical composition for the prophylaxis and/or treatment of cGMP-related diseases.

16. Use according to Claim 14 or 15 for preparing a medicament or a pharmaceutical composition for the prophylaxis and/or treatment of cardiovascular disorders, disorders of the urogenital system and cerebrovascular disorders.

17. Use according to any of Claims 14 to 16 for preparing a medicament or a pharmaceutical composition for the prophylaxis and/or treatment of cardiovascular disorders such as hypertension, neuronal hypertonia, stable and unstable angina, peripheral and cardial vasculopathies, arrhythmias, thromboembolic disorders and ischaemias such as myocardial infarction, stroke, transistory and ischaemic attacks, angina pectoris, obstruction of peripheral circulation, prevention of restenoses after thrombolysis therapy, percutaneous transluminal angioplasty (PTA), percutaneous transluminal coronary angioplasties (PTCA) and bypass.

18. Use according to any of Claims 14 to 16 for preparing a medicament or a pharmaceutical composition for the prophylaxis and/or treatment of cerebrovascular disorders such as cerebral ischaemia, stroke, reperfusion damage, cerebral trauma, oedema, cerebral thrombosis, dementia and Alzheimer's disease.

19. Use according to any of Claims 14 to 16 for preparing a medicament or a pharmaceutical composition for the prophylaxis and/or treatment of disorders of the urogenital system such as hypertrophy of the prostate, incontinence and in particular erectile dysfunction and female sexual dysfunction.

20. Use according to any of Claims 14 to 19, **characterized in that** the medicaments or compositions are administered intravenously or orally.

## Revendications

1. Imidazotriazinones de la formule générale (I) suivante : 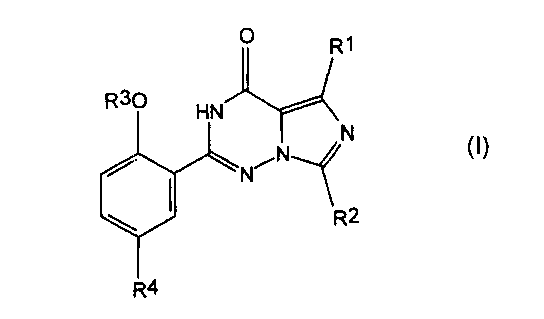 dans laquelle :
R¹ représente un radical alcoyle (C₁-C₆) ;
R² représente un radical cycloalcoyle (C₃-C₈) ou un radical alcoyle (C₁-C₁₂) ;
R³ représente un radical alcoyle (C₁-C₆) ;
R⁴ représente un reste de la formule -NH-CO-NR¹⁷R¹⁸, où
R¹⁷ et R¹⁸ sont identiques ou différents et représentent l'atome d'hydrogène ou un radical alcoyle (C₁-C₆), qui est le cas échéant substitué par le radical hydroxyle ou par un reste de la formule : 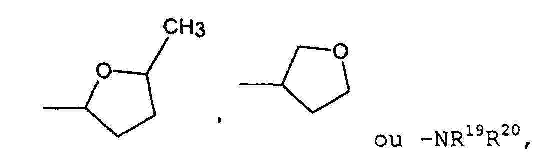 où
R¹⁹ et R²⁰ sont identiques ou différents et représentent l'atome d'hydrogène, le radical phényle ou un radical alcoyle (C₁-C₆),
ou
R¹⁷ et R¹⁸, avec l'atome d'azote, sur lequel ils sont liés, forment un hétérocycle des formules : 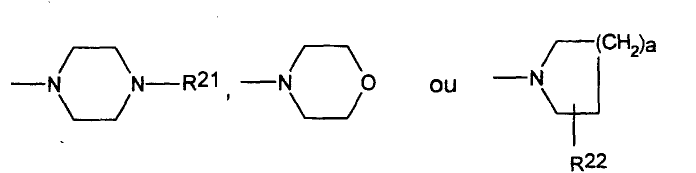 où
R²¹ représente l'atome d'hydrogène ou un radical alcoyle (C₁-C₆),
a signifie 1 ou 2,
R²² représente le radical hydroxyle ou un radical alcoyle (C₁-C₆), qui est le cas échéant substitué par le radical hydroxyle,
ou
R¹⁷ et/ou R¹⁸ représentent un radical aryle (C₆-C₁₂), qui est le cas échéant substitué par un atome d'halogène, le radical trifluoroéthyle ou par le radical -SCF₃,
ou
R¹⁷ représente l'atome d'hydrogène et
R¹⁸ représente un reste de la formule -SO₂-R²³,
où
R²³ représente un radical alcoyle (C₁-C₆) ou aryle (C₆-C₁₂), qui est le cas échéant substitué par un atome d'halogène,
ou représente un reste des formules : 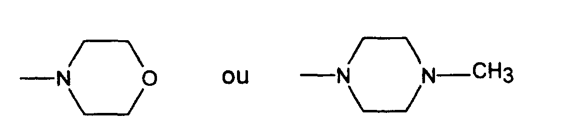 et leurs tautomères ainsi que leurs sels, hydrates et promédicaments pharmaceutiquement compatibles.

2. Imidazotriazinones de la formule générale (I) suivant la revendication 1,
où
R¹ représente un radical alcoyle (C₁-C₄) ;
R² représente le radical cyclopentyle, cycloheptyle ou un radical alcoyle (C₁-C₁₀) ;
R³ représente un radical alcoyle (C₁-C₄) ;
R⁴ représente un reste de la formule -NH-CO-NR¹⁷R¹⁸, où
R¹⁷ et R¹⁸ sont identiques ou différents et représentent l'atome d'hydrogène ou un radical alcoyle (C₁-C₄), qui est le cas échéant substitué par le radical hydroxyle ou par un reste de la formule : 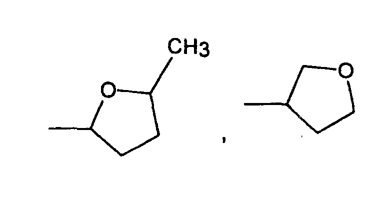 ou -NR¹⁹R²⁰,
où
R¹⁹ et R²⁰ sont identiques ou différents et représentent l'atome d'hydrogène, le radical phényle ou un radical alcoyle (C₁-C₄),
ou
R¹⁷ et R¹⁸, avec l'atome d' azote, sur lequel ils sont liés, forment un hétérocycle des formules : 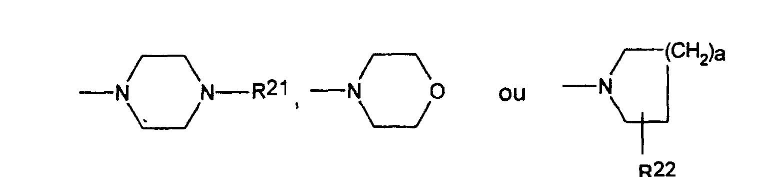 où
R²¹ représente l'atome d'hydrogène ou un radical alcoyle (C₁-C₄),
a signifie 1 ou 2,
R²² représente le radical hydroxyle ou un radical alcoyle (C₁-C₄), qui est le cas échéant substitué par le radical hydroxyle,
ou
R¹⁷ et/ou R¹⁸ représentent le radical phényle, qui est le cas échéant substitué par l'atome de chlore, le radical trifluoroéthyle ou par le radical -SCF₃,
ou
R¹⁷ représente l'atome d'hydrogène et
R¹⁸ représente un reste de la formule -SO₂-R²³,
où
R²³ représente un radical alcoyle (C₁-C₄) ou le radical phényle, qui est le cas échéant substitué par un atome d'halogène,
ou représente un reste des formules : 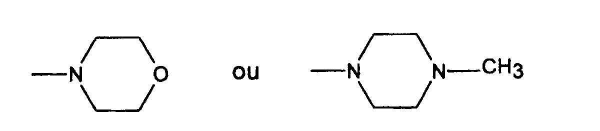 et leurs tautomères ainsi que leurs sels, hydrates et promédicaments pharmaceutiquement compatibles.

3. Imidazotriazinones de la formule générale (I) suivant la revendication 1 ou 2,
où
R¹ représente un radical alcoyle (C₁-C₄) ;
R² représente le radical cyclopentyle, cyclohexyle, cycloheptyle ou un radical alcoyle (C₁-C₁₀) ;
R³ représente un radical alcoyle (C₁-C₄) ;
R⁴ représente un reste de la formule -NH-CO-NR¹⁷R¹⁸, où
R¹⁷ et R¹⁸ sont identiques ou différents et représentent l'atome d'hydrogène ou un radical alcoyle (C₁-C₄), qui est le cas échéant substitué par le radical hydroxyle,
ou
R¹⁷ et R¹⁸, avec l'atome d'azote, sur lequel ils sont liés, forment un hétérocycle des formules : 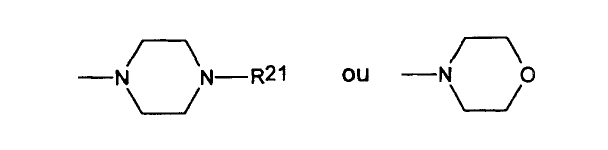 où
R²¹ représente l'atome d'hydrogène ou un radical alcoyle (C₁-C₄),
ou
R¹⁷ et/ou R¹⁸ représentent le radical phényle, qui est le cas échéant substitué par l'atome de chlore, le radical trifluoroéthyle ou par le radical -SCF₃,
ou
R¹⁷ représente l'atome d'hydrogène et
R¹⁸ représente un reste de la formule -SO₂-R²³,
où
R²³ représente un radical alcoyle (C₁-C₄) ou phényle, qui est le cas échéant substitué par un atome d'halogène,
ou représente un reste des formules : 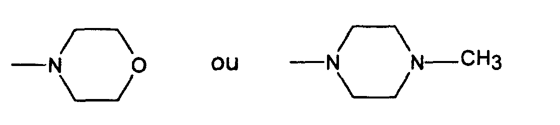 et leurs tautomères ainsi que leurs sels, hydrates et promédicaments pharmaceutiquement compatibles.

4. Imidazotriazinones suivant la revendication 1, avec les structures suivantes : 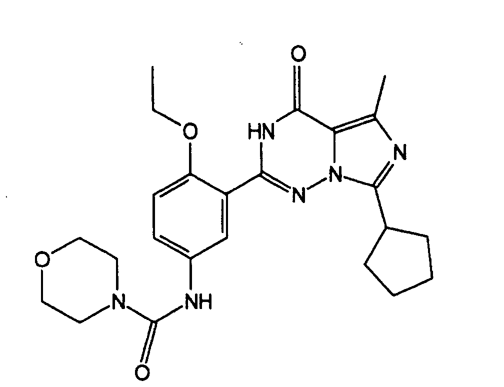 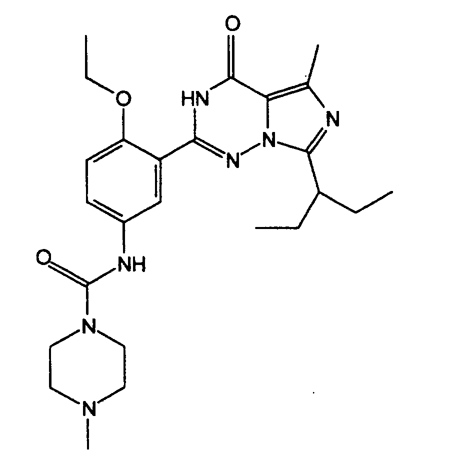 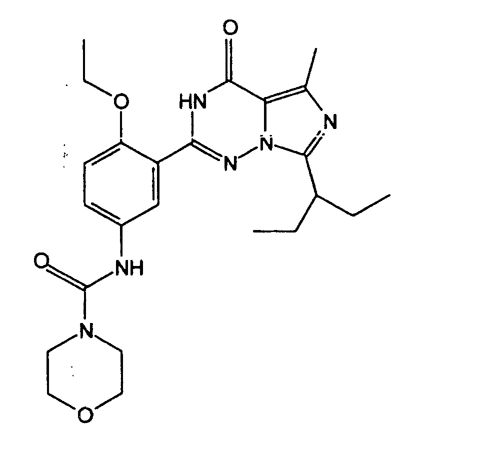 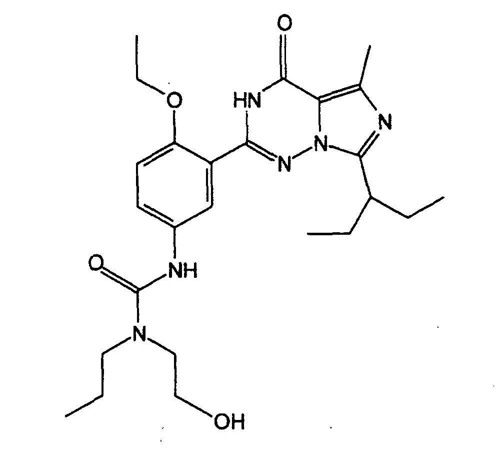 et leurs tautomères ainsi que leurs sels, hydrates et promédicaments pharmaceutiquement compatibles.

5. Composés de la formule générale (I) selon les revendications 1 à 4, pour la prophylaxie et/ou le traitement de maladies.

6. Procédé de préparation des imidazotriazinones selon les revendications 1 à 4, **caractérisé en ce que**
[A] dans le cas où R⁴ représente un reste lié par un atome d'azote, comme défini ci-dessus, on convertit des composés de la formule générale (II) : 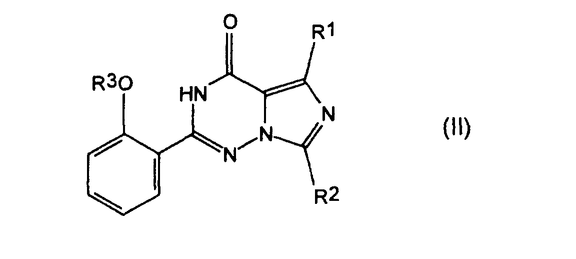 dans laquelle
R¹, R² et R³ ont la signification donnée ci-dessus,
d'abord par réaction avec HNO₃/CF₃CO₂H, en les composés de la formule générale (III) : 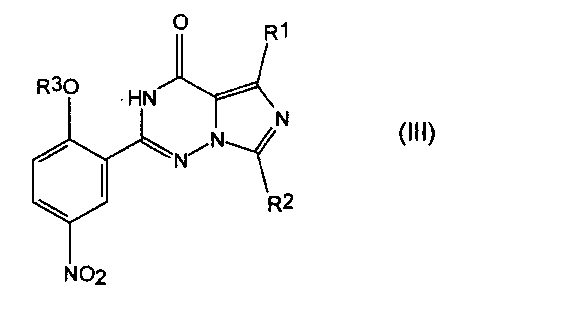 dans laquelle
R¹, R² et R³ ont la signification donnée ci-dessus,
dans une étape suivante, on réduit avec H₂/Pd-C en les amines de la formule générale (IV) : 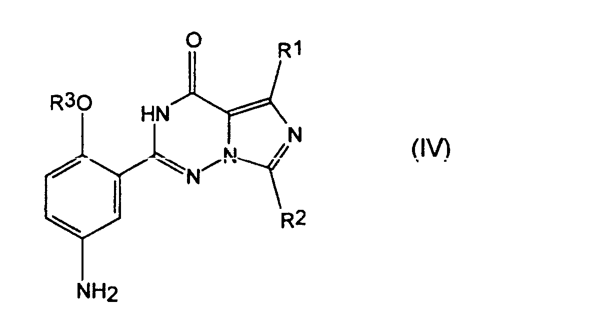 dans laquelle
R¹, R² et R³ ont la signification donnée ci-dessus,
et ensuite, on fait réagir avec des composés de la formule générale (V) :
A - D (V)
dans laquelle:
A représente les restes R¹⁷ ou R¹⁸ indiqués ci-dessus pour R⁴,
D représente le reste -NH-CO-Cl, -N=C=O ou -SO₂-N=C=O,
dans un solvant inerte, le cas échéant en présence d'une base et/ou d'un auxiliaire.

7. Agent pharmaceutique ou composition pharmaceutique, contenant au moins un composé de la formule générale (I) selon l'une quelconque des revendications 1 à 4, ainsi qu'un ou plusieurs auxiliaires et véhicules pharmacologiquement compatibles.

8. Agent pharmaceutique ou composition pharmaceutique suivant la revendication 7, pour la prophylaxie et/ou le traitement de maladies, qui se produisent dans le contexte des processus régulés par le cGMP (« cGMP-related diseases »).

9. Agent pharmaceutique ou composition pharmaceutique suivant la revendication 7 ou 8, pour la prophylaxie et/ou le traitement de maladies cardiovasculaires, de maladies du système urogénital ainsi que des maladies cérébrovasculaires.

10. Agent pharmaceutique ou composition pharmaceutique suivant l'une quelconque des revendications 7 à 9, pour la prophylaxie et/ou le traitement de maladies cardiovasculaires comme l'hypertension artérielle, l'hypertonie neuronale, les angines stable et instable, les maladies des vaisseaux périphériques et cardiaques, les arythmies, les maladies thromboemboliques et les ischémies comme l'infarctus du myocarde, l'attaque, les attaques transitoires et ischémiques, l'angine de poitrine, les troubles de la circulation périphérique, la prévention des resténoses après thérapie de thrombolyse, l'angioplastie transluminale percutanée (PTA), les coronaroangioplasties transluminales percutanés (PTCA) et les pontages.

11. Agent pharmaceutique ou composition pharmaceutique suivant l'une quelconque des revendications 7 à 9, pour la prophylaxie et/ou le traitement de maladies cérébrovasculaires comme l'ischémie cérébrale, l'attaque, les troubles de reperfusion, les traumatismes cérébraux, les oedèmes, la thrombose cérébrale, la démence et la maladie d'Alzheimer.

12. Agent pharmaceutique ou composition pharmaceutique suivant l'une quelconque des revendications 7 à 9, pour la prophylaxie et/ou le traitement de maladies du système urogénital comme l'hypertrophie prostatique, l'incontinence et en particulier, le dysfonctionnement érectile et le dysfonctionnement sexuel féminin.

13. Agent pharmaceutique ou composition pharmaceutique suivant l'une quelconque des revendications 7 à 12, **caractérisé en ce que** l'agent pharmaceutique ou la composition pharmaceutique est administrée de manière intraveineuse ou orale.

14. Utilisation de composés de la formule générale (I), suivant l'une quelconque des revendications 1 à 4, pour la préparation d'agents pharmaceutiques ou de compositions pharmaceutiques pour la prophylaxie et/ou le traitement de maladies.

15. Utilisation suivant la revendication 14, pour la préparation d'un agent pharmaceutique ou d'une composition pharmaceutique pour la prophylaxie et/ou le traitement de maladies qui se produisent dans le contexte des processus régulés par le cGMP (« cGMP-related diseases »).

16. Utilisation suivant la revendication 14 ou 15, pour la préparation d'un agent pharmaceutique ou d'une composition pharmaceutique pour la prophylaxie et/ou le traitement de maladies cardiovasculaires, de maladies du système urogénital ainsi que des maladies cérébrovasculaires.

17. Utilisation suivant l'une quelconque des revendications 14 à 16, pour la préparation d'un agent pharmaceutique ou d'une composition pharmaceutique pour la prophylaxie et/ou le traitement de maladies cardiovasculaires comme l'hypertension artérielle, l'hypertonie neuronale, les angines stable et instable, les maladies des vaisseaux périphériques et cardiaques, les arythmies, les maladies thromboemboliques et les ischémies comme l'infarctus du myocarde, l'attaque, les attaques transitoires et ischémiques, l'angine de poitrine, les troubles de la circulation périphérique, la prévention des resténoses après thérapie de thrombolyse, l'angioplastie transluminale percutanée (PTA), les coronaroangioplasties transluminales percutanés (PTCA) et les pontages.

18. Utilisation suivant l'une quelconque des revendications 14 à 16, pour la préparation d'un agent pharmaceutique ou d'une composition pharmaceutique pour la prophylaxie et/ou le traitement de maladies cérébrovasculaires comme l'ischémie cérébrale, l'attaque, les troubles de reperfusion, les traumatismes cérébraux, les oedèmes, la thrombose cérébrale, la démence et la maladie d'Alzheimer.

19. Utilisation suivant l'une quelconque des revendications 14 à 16, pour la préparation d'un agent pharmaceutique ou d'une composition pharmaceutique pour la prophylaxie et/ou le traitement de maladies du système urogénital comme l'hypertrophie prostatique, l'incontinence et en particulier, le dysfonctionnement érectile et le dysfonctionnement sexuel féminin.

20. Utilisation suivant l'une quelconque des revendications 14 à 19, **caractérisé en ce que** l'agent pharmaceutique ou la composition pharmaceutique est administrée de manière intraveineuse ou orale.
